# EUROPEAN PATENT APPLICATION

(11) **EP 4 730 977 A1**
(43) Date of publication of application: **22.04.2026**
(21) Application number: 25209143.4
(22) Date of filing: 16.10.2025
(51) Int. Cl.: H10K 85/60, H10K 50/19

(54) **NOVEL COMPOUND AND ORGANIC LIGHT-EMITTING DEVICE COMPRISING THE SAME**

(30) Priority: 18.10.2024 KR 20240143213; 24.03.2025 KR 20250037553
(71) Applicant: TOP RUN MATERIAL SOLUTION Co., Ltd., Yangju-si, Gyeonggi-do 11410 (KR)
(72) Inventor: LEE, A Reum, Yangju-si, Gyeonggi-do (KR); MOON, So Jeong, Yangju-si, Gyeonggi-do (KR); JANG, Joo Hee, Yangju-si, Gyeonggi-do (KR); YOO, Jae Duk, Yangju-si, Gyeonggi-do (KR); KWON, Young Jae, Yangju-si, Gyeonggi-do (KR); PARK, Da Young, Yangju-si, Gyeonggi-do (KR); HYUN, A Reum, Yangju-si, Gyeonggi-do (KR)
(74) Representative: Isarpatent

(57) **Abstract**

The present invention provides a novel compound and an organic light-emitting device comprising the same, in which a rigid fluorene-based substituent is introduced into 1,10-phenanthroline having a non-shared electron pair. When the compound is applied to one or more organic layers of an electron transport layer (ETL), a hole blocking layer (HBL), and an N-type charge generation layer (N-CGL), the compound enables the implementation of low voltage, high efficiency, and long life of an organic light-emitting device.

## Description

### BACKGROUND

### 1. Technical Field

The present invention relates to a novel compound and an organic light-emitting device comprising the same.

### 2. Description of the Related Art

The technology of organic light emitting devices (OLEDs), one of the currently widely used flat panel display devices, is developing rapidly.

In general, the organic light-emitting device has an organic thin film layer including a light-emitting layer formed between an anode (hole injection electrode) and a cathode (electron injection electrode), and has a principle of emitting light when holes injected from the anode and electrons injected from the cathode pair up in the light-emitting layer and then disappear.

More specifically, the organic light-emitting device is configured to include an organic thin film layer formed between an anode and a cathode, and the organic thin film layer may be configured to include a hole injection layer, a hole transport layer, a light-emitting layer, an electron transport layer, and an electron injection layer sequentially laminated on the anode, and holes injected from the anode and electrons injected from the cathode combine in the light-emitting layer to form excitons, which enter an unstable energy state (excited state) and then return to a stable ground state to emit light.

In the development of organic light-emitting devices, high efficiency and long lifespan, as well as color purity, color stability under current and voltage changes, and ease of device manufacturing, are crucial. Therefore, research and development are underway for each approach. Organic light-emitting device structures can be broadly categorized into single-emitting structures with a single light-emitting member and stacked structures with two or more light-emitting members. Among these, the tandem structure, which stacks two or more light-emitting elements, is the most commonly adopted for long-life organic light-emitting devices.

Meanwhile, organic light-emitting devices with this tandem structure are equipped with a charge generation layer (CGL) between the emission layers, which doubles the current efficiency generated in the emission layer and facilitates charge distribution. This charge generation layer, which generates charges (electrons and holes), doubles the current efficiency generated by the emission layer and facilitates charge distribution, thereby preventing an increase in operating voltage. This charge generation layer is composed of a P-type charge generation layer and an N-type charge generation layer.

Currently, compounds applied to charge generation layers, especially N-type charge generation layers, are increasingly required to develop high-performance devices, and thus, there is a need to develop new materials that can exhibit excellent performance.

### SUMMARY

The present invention is to provide a novel compound in which a rigid fluorene-based substituent is introduced into 1,10-phenanthroline having a non-shared electron pair, and to provide an organic light-emitting device capable of realizing low voltage, high efficiency, and long life when the novel compound is applied to at least one organic layer among an electron transport layer (ETL), a hole blocking layer (HBL), and an N-type charge generation layer (N-CGL).

The above-mentioned objectives and other objectives will be understood from the description provided below.

To address the above challenges, in one aspect, the present invention provides a novel compound represented by the following Chemical Formula 1.

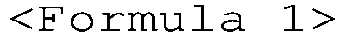

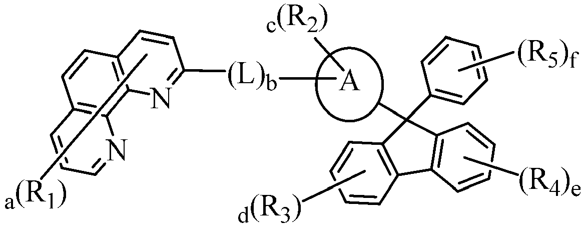

In the Chemical Formula 1,
ring A is phenyl or naphthyl,
R1 is each independently hydrogen, deuterium, a halogen group, a cyano group, a nitro group, a nitrile group, a hydroxyl group, a thiol group, a substituted or unsubstituted C0~C50 amine group, a substituted or unsubstituted C1~C50 alkyl group, a substituted or unsubstituted C1~C50 alkenyl group, a substituted or unsubstituted C1~C50 alkynyl group, a substituted or unsubstituted C1~C50 alkoxy group, a substituted or unsubstituted C1~C50 sulfide group, a substituted or unsubstituted C0~C50 silyl group, a substituted or unsubstituted C3~C50 cycloalkyl group, a substituted or unsubstituted C2~C50 heterocycloalkyl group, a substituted or unsubstituted C3~C50 cycloalkenyl group, a substituted or unsubstituted C1~C30 phosphine oxide group, a substituted or unsubstituted C2~C50 heterocycloalkenyl group, a substituted or unsubstituted C6~C50 aryl group, a substituted or unsubstituted C6~C50 aryloxy group, a substituted or unsubstituted C2~C50 heteroaryloxy group, or a substituted or unsubstituted C2~C50 heteroaryl group, wherein adjacent groups may or may not combine with each other to form a substituted or unsubstituted ring,
L is each independently a direct bond, a substituted or unsubstituted C6~C50 arylene group, or a substituted or unsubstituted C2~C50 heteroarylene group,
R2 to R5 are each independently hydrogen or deuterium, or may or may not be combined with each other to form a ring substituted or unsubstituted with one or more deuteriums,
a is an integer from 0 to 7,
b is an integer from 0 to 3,
c is an integer from 0 to 6,
d and e are each independently integers from 0 to 4, and
f is an integer from 0 to 5.

In addition, the present invention provides, in one aspect, an organic light-emitting device comprising the novel compound described above.

The novel compound according to one embodiment of the present invention is particularly preferable for application to an N-type charge generation layer because it can easily combine with dopants such as lithium and ytterbium by introducing 1,10-phenanthroline having a non-shared electron pair, and also has excellent thermal stability by introducing a rigid fluorene-based substituent.

In addition, the novel compound according to one embodiment of the present invention has a reverse structure in which 1,10-phenanthroline is bonded to phenyl or naphthyl located above diphenylfluorene or naphthylphenylfluorene, and thus has a lower RE (Reorganization Energy) value compared to a structure in which it is bonded to the lower fluorene, thereby increasing the efficiency of the device.

In addition, the novel compound according to one embodiment of the present invention has a structure in which the position (position 2 or 9) immediately adjacent to N of 1,10-phenanthroline is bonded, thereby expanding the conjugation and lowering the RE value compared to other positions, thereby increasing the efficiency of the device.

Furthermore, by eliminating the introduction of additional functional groups to fluorene, the packing density and refractive index can be increased, resulting in an increase in the lifespan of the device.

The novel compound of the present invention can be usefully applied to one or more organic layers of an electron transport layer (ETL), a hole blocking layer (HBL), and an N-type charge generation layer (N-CGL) among organic layers of an organic light-emitting device, and in particular, when applied to a host of an N-type charge generation layer of a tandem organic light-emitting device, it can enable the implementation of low voltage, high efficiency, and long life of the device.

The above effects and other effects will be described in detail below.

### DETAILED DESCRIPTION

In describing the present invention, if it is determined that a detailed description of a related known configuration or function may obscure the gist of the present invention, the detailed description will be omitted.

In this specification, when it is described that a part "includes" a certain component, this does not mean that other components are excluded, but rather that other components may be included, unless specifically stated otherwise.

In this specification, when it is described that a member is located 'on' another member, this includes not only cases where a member is in contact with another member, but also cases where another member exists between the two members.

When describing components of the present invention, terms such as first, second, A, B, (a), (b), etc. may be used. These terms are only intended to distinguish the components from other components, and the nature, order, or sequence of the components are not limited by the terms. When it is described that a component is "bonded," "coupled," or "connected" to another component, it should be understood that the component may be directly bonded or connected to the other component, but another component may also be "bonded," "coupled," or "connected" between each component.

Examples of substituents in this specification are described below, but are not limited thereto.

As used herein, the term "substitution" refers to the replacement of a hydrogen atom bonded to a carbon atom of a compound with another substituent. Substitution can occur at any position where substitution is possible, without particular limitations. If two or more substituents are substituted, the substituents may be the same or different.

In this specification, the term 'substituted or unsubstituted' means that it is substituted with one or more substituents selected from a group consisting of deuterium, halogen, cyano, nitro, nitrile, alkyl, alkenyl, alkynyl, cycloalkyl, heterocycloalkyl, alkoxy, sulfide, aryloxy, heteroaryloxy, thio, amine, silyl, phosphine oxide, aryl, and heteroaryl groups, or is substituted with a substituent in which two or more substituents selected from the above group are bonded, or has no substituents, and the selected substituents may or may not form a ring by combining with each other. An example of a substituent in which two or more substituents are bonded is a biphenyl group. That is, a biphenyl group may correspond to an aryl group and at the same time correspond to a substituent having two phenyl groups bonded, and at the same time correspond to an aryl group having one phenyl group substituted.

In the present specification, an alkyl group may be a linear or branched chain having 1 to 60 carbon atoms, and specific examples thereof include, but are not limited to, methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethylpropyl, isohexyl, 2-methylpentyl, 4-methylhexyl, and 5-methylhexyl. Specifically, the alkyl group may have 1 to 30 carbon atoms, and more specifically, 1 to 20 carbon atoms.

In the present specification, an alkenyl group may be a linear or branched chain having 2 to 60 carbon atoms, and specific examples thereof include, but are not limited to, a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, and a styrenyl group. Specifically, the number of carbon atoms in the alkenyl group may be 2 to 30, and more specifically, 2 to 20.

In the present specification, an alkynyl group may be a linear or branched chain having 2 to 60 carbon atoms. Specifically, the alkynyl group may have 2 to 30 carbon atoms, and more specifically, 2 to 20 carbon atoms.

In the present specification, an alkoxy group may be a linear, branched or cyclic chain having 1 to 60 carbon atoms, and specific examples thereof include, but are not limited to, methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, etc. Specifically, the alkoxy group may have 1 to 30 carbon atoms, and more specifically, 1 to 20 carbon atoms.

In the present specification, a cycloalkyl group may be monocyclic or polycyclic having 3 to 60 carbon atoms, and specific examples thereof include, but are not limited to, cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl. Specifically, the cycloalkyl group may have 3 to 30 carbon atoms, and more specifically, 3 to 20 carbon atoms.

In the present specification, a heterocycloalkyl group is a group that contains at least one non-carbon atom, i.e., a heteroatom, and specifically, may be a cycloalkyl group that contains at least one heteroatom selected from the group consisting of O, N, S, Se, etc., and may be a monocyclic or polycyclic group having 2 to 60 carbon atoms. Specifically, the heterocycloalkyl group may have 2 to 30 carbon atoms, and more specifically, may have 2 to 20 carbon atoms.

In the present specification, a sulfide group includes S and may have 1 to 60 carbon atoms. Specific examples include, but are not limited to, an alkyl sulfide group such as a dimethyl sulfide group, an aryl sulfide group such as diphenyl sulfide, and a heteroaryl sulfide group substituted with a heteroaryl group. Specifically, the sulfide group may have 1 to 30 carbon atoms, and more specifically, 1 to 20 carbon atoms.

In the present specification, an aryloxy group may be a substituent that contains O, and the O atom may be directly connected as a radical, and may have 6 to 60 carbon atoms. Specific examples include phenoxy groups, naphthoxy groups, biphenoxy groups, etc., which are oxy groups substituted with aryl groups, but are not limited thereto. The heteroaryloxy group is an oxy group substituted with heteroaryl groups, and may have 2 to 60 carbon atoms. Specifically, the aryloxy group may have 6 to 30 carbon atoms, and more specifically, may have 6 to 20 carbon atoms.

In the present specification, a thio group includes S and may have 1 to 60 carbon atoms. Specific examples include, but are not limited to, alkylthio groups such as methylthio, ethylthio, butylthio, pentylthio, and hexylthio; arylthio groups such as phenylthio, and naphthylthio; and heteroarylthio groups substituted with heteroaryl groups. Specifically, the thio group may have 1 to 30 carbon atoms, and more specifically, 1 to 20 carbon atoms.

In the present specification, a silyl group may be a substituent containing Si, and the Si atom may be directly bonded as a radical, and may have 1 to 60 carbon atoms. Specific examples thereof include, but are not limited to, alkylsilyl groups such as trimethylsilyl, triethylsilyl, t-butyldimethylsilyl, vinyldimethylsilyl, and propyldimethylsilyl groups; arylsilyl groups such as triphenylsilyl, diphenylsilyl, and phenylsilyl groups; and heteroarylsilyl groups substituted with heteroaryl groups. Specifically, the silyl group may have 1 to 30 carbon atoms, and more specifically, 1 to 20 carbon atoms.

In the present specification, a phosphine oxide group includes P=O and may have 1 to 60 carbon atoms. Specific examples include, but are not limited to, an alkylphosphine oxide group such as a dimethylphosphine oxide group, an arylphosphine oxide group such as a diphenylphosphine oxide group and a dinaphthylphosphine oxide group, and a heteroarylphosphine oxide group substituted with a heteroaryl group. Specifically, the phosphine oxide group may have 1 to 30 carbon atoms, and more specifically, 1 to 20 carbon atoms.

In the present specification, an aryl group may be monocyclic or polycyclic and have 6 to 60 carbon atoms. In the case of a monocyclic aryl group, specific examples thereof include a phenyl group, a biphenyl group, a terphenyl group, etc., but are not limited thereto. In the case of a polycyclic aryl group, specific examples thereof include a naphthyl group, anthracenyl group, a phenanthrenyl group, a triphenylenyl group, a pyrenyl group, a fluorenyl group, etc., but are not limited thereto. Specifically, the aryl group may have 6 to 50 carbon atoms, and more specifically, 6 to 30 carbon atoms.

In the present specification, a heteroaryl group includes at least one non-carbon atom, i.e., a heteroatom, and may specifically include at least one heteroatom selected from the group consisting of O, N, S, and Se, and may be a monocyclic or polycyclic group having 2 to 60 carbon atoms. Specific examples include, but are not limited to, a thiophenyl group, a furanyl group, a pyrrolyl group, an imidazolyl group, a thiazolyl group, an oxazolyl group, an oxadiazolyl group, a pyridinyl group, a bipyridinyl group, a pyrimidinyl group, a pyrimidinyl group, a pyrazinyl group, a quinolinyl group, a quinazolyl group, a quinoxalinyl group, a phthalazinyl group, a pyridopyrimidinyl group, a pyridopyrazinyl group, a pyrazinopyrazinyl group, a pyridoindolyl group, a benzothienopyrimidyl group, an indenocarbazolyl group, an isoquinolinyl group, an indolyl group, a carbazolyl group, a benzoxazolyl group, a benzimidazolyl group, a benzothiazolyl group, a benzocarbazolyl group, a benzothiophenyl group, a dibenzothiophenyl group, a benzofuranyl group, a phenanthridinyl group, a phenanthrolinyl group, an isoxazolyl group, a thiadiazolyl group, a phenothiazinyl group, and a dibenzofuranyl group. Specifically, the heteroaryl group may have 2 to 50 carbon atoms, and more specifically, 2 to 30 carbon atoms.

In the present specification, an amine group may be selected from the group consisting of -NH2, an alkylamine group, an N-alkylarylamine group, an arylamine group, an N-arylheteroarylamine group, an N-alkylheteroarylamine group, and a heteroarylamine group.

In this specification, an arylene group refers to a divalent aryl group having two bonding positions within an aryl group, and a heteroarylene group also refers to a divalent heteroaryl group having two bonding positions within a heteroaryl group. The descriptions of the aryl group and heteroaryl group described above can be applied to these groups, except that they are each divalent groups.

In the present specification, the fused ring group includes a fused ring group in which an aryl group and a cycloalkyl group are fused, a fused ring group in which a heteroaryl group and a cycloalkyl group are fused, a fused ring group in which an aryl group and a heterocycloalkyl group are fused, or a fused ring group in which a heteroaryl group and a heterocycloalkyl group are fused. According to one embodiment, the tetralin group represented by the structural formula below may correspond to one of the fused ring groups in which an aryl group and a cycloalkyl group are fused, and benzo-1,4-dioxane may correspond to one of the fused ring groups in which an aryl group and a heterocycloalkyl group are fused. 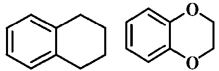 In the chemical formula or structural formula in this specification, "*" or 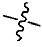 indicates a bonding position.

In the chemical formulas or structural formulas in this specification, identical symbols may be the same or different.

In this specification, when a range such as "C2~C50", "0 to 7" is described, even if there is no special description, it can be reduced to various ranges within the described range and is considered to be described in this specification. For example, C2~C50 is considered to describe C2~C50 and various reduction ranges such as C5~C50, C6~C30, C6~C20, C6~C15, C6~C10, C12~C30, etc. together. Therefore, the description of numerical ranges in this specification may be reduced or corrected in the future.

Throughout this specification, the term "interaction with a dopant" may mean coordination of an organic compound with a dopant, such as an alkali metal, an alkaline earth metal, a rare earth metal, or a lanthanum metal within an N-type charge generation layer, and in one embodiment, may include coordination of an organic compound with a dopant to form a gap state.

Meanwhile, the various embodiments of the present invention may be combined with any other embodiments unless explicitly stated otherwise. Hereinafter, embodiments of the present invention and the resulting effects will be described.

Throughout this specification, an organic light-emitting device may mean an organic light-emitting diode and a panel including the organic light-emitting diode, or may mean an electronic device including the panel and a circuit. Here, for example, an electronic device may include a display device, a lighting device, a solar cell, a portable or mobile terminal (e.g., a smart phone, a tablet, a PDA, an electronic dictionary, a PMP, etc.), a navigation terminal, a game machine, various TVs, various computer monitors, etc., and is not limited thereto, and may be any type of device as long as it includes the above-described component(s).

The present invention will now be described in detail.

The present invention relates to a novel compound and an organic light-emitting device comprising the same.

The novel compound according to one embodiment of the present invention may be represented by the following Chemical Formula 1.

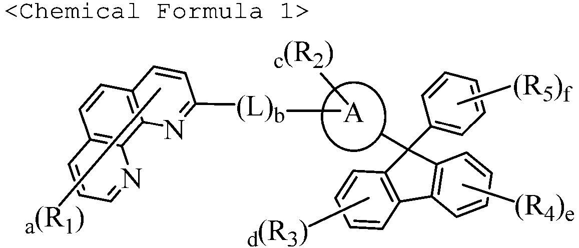

In the Chemical Formula 1,
ring A is phenyl or naphthyl,
R1 is each independently hydrogen, deuterium, a halogen group, a cyano group, a nitro group, a nitrile group, a hydroxyl group, a thiol group, a substituted or unsubstituted C0~C50 amine group, a substituted or unsubstituted C1~C50 alkyl group, a substituted or unsubstituted C1~C50 alkenyl group, a substituted or unsubstituted C1~C50 alkynyl group, a substituted or unsubstituted C1~C50 alkoxy group, a substituted or unsubstituted C1~C50 sulfide group, a substituted or unsubstituted C0~C50 silyl group, a substituted or unsubstituted C3~C50 cycloalkyl group, a substituted or unsubstituted C2~C50 heterocycloalkyl group, a substituted or unsubstituted C3~C50 cycloalkenyl group, a substituted or unsubstituted C1~C30 phosphine oxide group, a substituted or unsubstituted C2~C50 heterocycloalkenyl group, a substituted or unsubstituted C6~C50 aryl group, a substituted or unsubstituted C6~C50 aryloxy group, a substituted or unsubstituted C2~C50 heteroaryloxy group, or a substituted or unsubstituted C2~C50 heteroaryl group, wherein adjacent groups may or may not combine with each other to form a substituted or unsubstituted ring,
L is each independently a direct bond, a substituted or unsubstituted C6~C50 arylene group, or a substituted or unsubstituted C2~C50 heteroarylene group,
R2 to R5 are each independently hydrogen or deuterium, or may or may not be combined with each other to form a ring substituted or unsubstituted with one or more deuteriums,
a is an integer from 0 to 7,
b is an integer from 0 to 3,
c is an integer from 0 to 6,
d and e are each independently integers from 0 to 4, and
f is an integer from 0 to 5.

The compound according to one embodiment of the present invention is a compound in which 1,10-phenanthroline and fluorene are bonded, and more specifically, 1,10-phenanthroline is bonded with the upper phenyl or naphthyl of diphenylfluorene or naphthylphenylfluorene, and is characterized by being bonded at a position(position 2 or 9) immediately adjacent to N of 1,10-phenanthroline. At the same time, the compound is structurally characterized by the absence of any additional substituents other than hydrogen or deuterium on the fluorene.

Meanwhile, in the Chemical Formula 1 and the following Chemical Frmulas, unless otherwise specifically defined, the substituent in the case of substitution may include one or more of a deuterium, halogen group, amino group, cyano group, hydroxy group, thiol group, cyano group, nitro group, nitroso group, sulfamoyl group, isothiocyanate group, thiocyanate group, carboxyl group, carbonyl group, a substituted or unsubstituted C1~C30 alkyl group, a substituted or unsubstituted C1~C30 alkylsulfinyl group, a substituted or unsubstituted C1~C30 alkylsulfonyl group, a substituted or unsubstituted C1~C30 alkylsulfanyl group, a substituted or unsubstituted C1~C30 fluoroalkyl group, a substituted or unsubstituted C2~C30 alkenyl group, a substituted or unsubstituted C1~C30 alkoxy group, a substituted or unsubstituted C0~C30 amino group, a substituted or unsubstituted C1~C12 N-alkylamino group, a substituted or unsubstituted C2~C20 N,N-dialkylamino group, a substituted or unsubstituted C1~C30 sulfide group, a substituted or unsubstituted C1~C6 N-alkylsulfamoyl group, a substituted or unsubstituted C2~C12 N,N-dialkylsulfamoyl group, a substituted or unsubstituted C0~C30 silyl group, a substituted or unsubstituted C3~C30 cycloalkyl group, a substituted or unsubstituted C1~C30 heterocycloalkyl group, a substituted or unsubstituted C6~C50 aryl group, and a substituted or unsubstituted C3~C50 heteroaryl group. More specifically, the substituent may be at least one of deuterium, halogen group, cyano group, a C1-C10 alkyl group, a C1-C10 alkoxy group, a C1-C10 alkylthio group, a C1-C10 fluoroalkyl group in which hydrogen is substituted with fluorine, a substituted or unsubstituted C6-C20 aryl group, and a substituted or unsubstituted C3-C20 heteroaryl group, and in one embodiment, may be exemplified by a deuterium, halogen group, cyano group, methyl group, ethyl group, t-butyl group, -CD3, phenyl group, naphthyl group, biphenyl group, pyridine group, and the like.

Specifically, according to one embodiment, the Chemical Formula 1 may be one of the following Chemical Formula 2 to Chemical Formula 4. Chemical Formula 2 below represents the case where ring A is phenyl, and Chemical Formula 3 and Chemical Formula 4 represent the case where ring A is naphthyl.

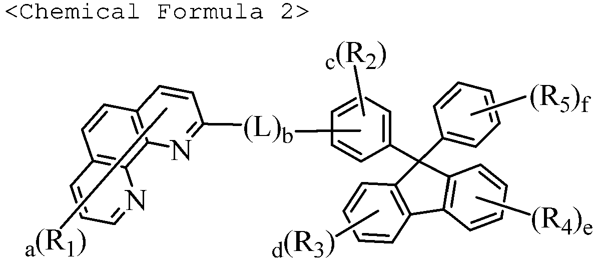

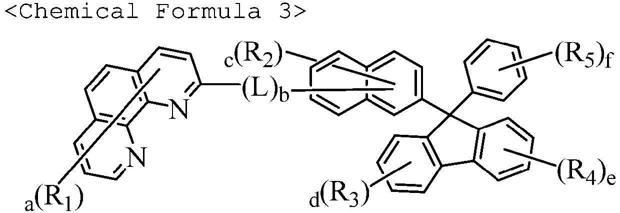

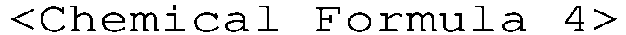

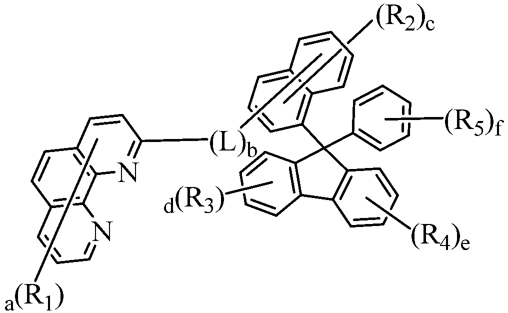

In the Chemical Formulas 2 to 4, R1, L, R2 to R5, and a to f have the same definitions as in the Chemical Formula 1.

Specifically, the Chemical Formula 2 may be one of the following Chemical Formulas 2-1 to 2-3. The Chemical Formula 2-1 below represents a case where it is bonded as ortho or 1,2-phenylene, the Chemical Formula 2-2 represents a case where it is bonded as meta or 1,3-phenylene, and the Chemical Formula 2-3 represents a case where it is bonded as para or 1,4-phenylene.

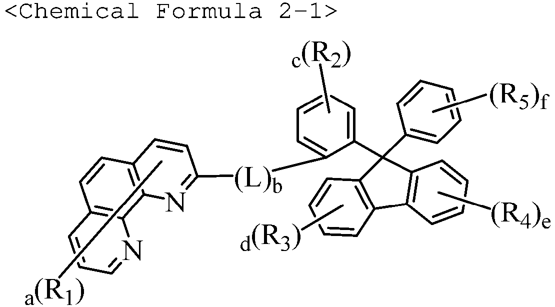

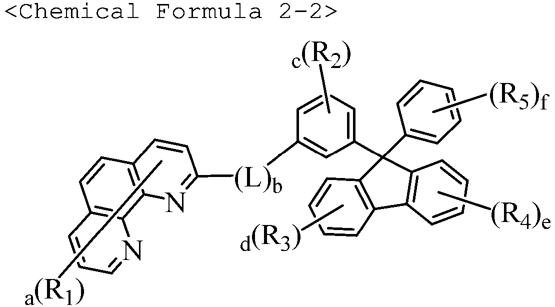

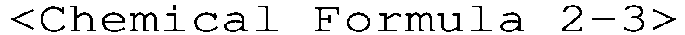

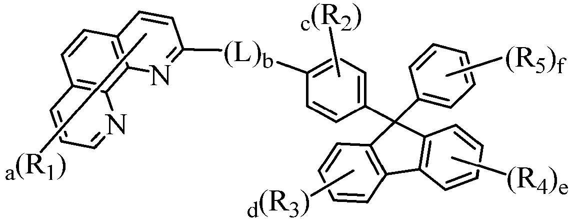

In the Chemical Formulas 2-1 to 2-3, R1, L, R2 to R5 and a to f are the same as defined in the Chemical Formula 1.

Specifically, the Chemical Formula 3 may be one of Chemical Formulas 3-1 to 3-3 below. The Chemical Formula 3-1 below represents a case where it is bonded as 2,6-naphthyl, the Chemical Formula 3-2 represents a case where it is bonded as 2,7-naphthyl, and the Chemical Formula 3-3 below represents a case where it is bonded as 1,3-naphthyl.

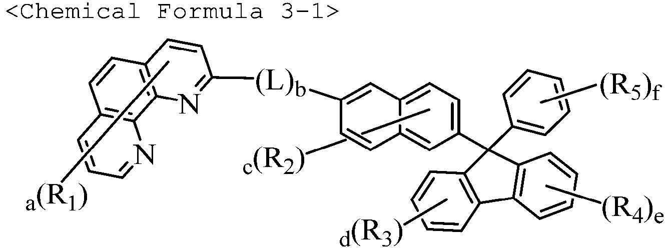

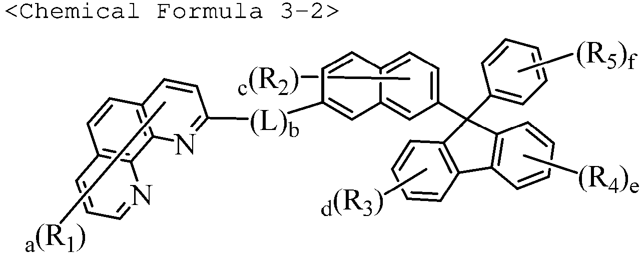

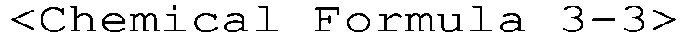

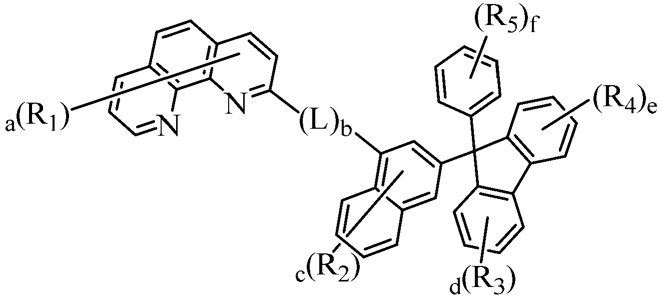

In the Chemical Formulas 3-1 to 3-3, R1, L, R2 to R5, and a to f have the same definitions as in the Chemical Formula 1.

Specifically, the Chemical Formula 4 may be Chemical Formula 4-1 or 4-2 below. the Chemical Formula 4-1 below represents a case where it is bonded as 1,4-naphthyl, and the Chemical Formula 4-2 below represents a case where it is bonded as 1,3-naphthyl.

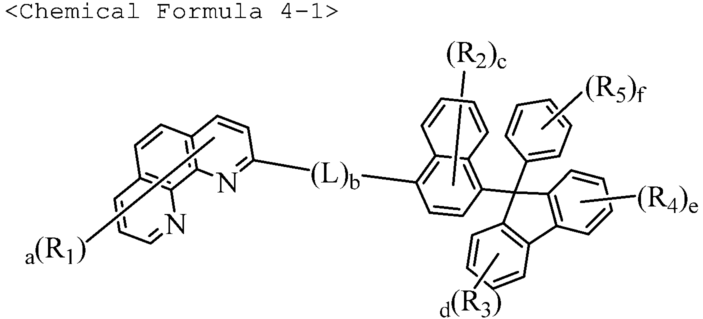

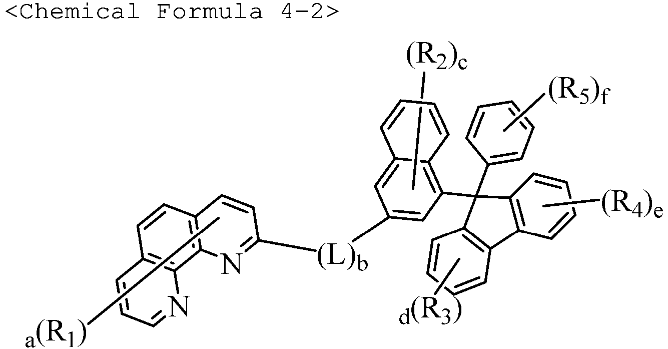

In the Chemical Formulas 4-1 and 4-2, R1, L, R2 to R5 and a to f are the same as defined in the Chemical Formula 1.

Meanwhile, in the above Chemical Formulas,
According to one embodiment of the present invention, R1 may independently be one of hydrogen, deuterium, a methyl group, -CD3, a phenyl group, a biphenyl group, a naphthyl group, a pyridine group, a pyrimidine group, a quinoline group, an isoquinoline group, a quinazoline group, and a naphthyridine group, and specifically may be hydrogen, a phenyl group, or a pyridine group, and even more specifically may be hydrogen or a phenyl group.

According to one embodiment of the present invention, a may be an integer from 0 to 3, specifically an integer from 0 to 2, and more specifically 0 or 1.

Meanwhile, according to one embodiment of the present invention, adjacent ones of R1 may be bonded to form a ring substituted or unsubstituted with one or more substituents, specifically, a substituted or unsubstituted benzene. In this case, the 1,10-phenanthroline moiety in the Chemical Formula 1 may be specifically represented by one of the following Structural Formula P-1 to Structural Formula P-4.

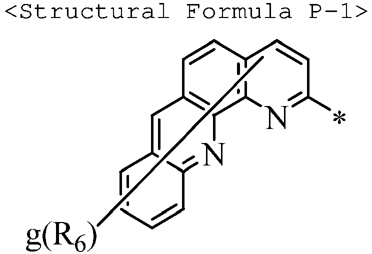

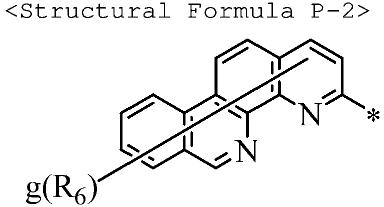

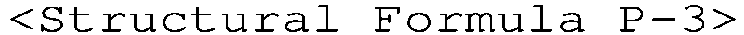

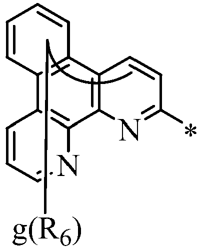

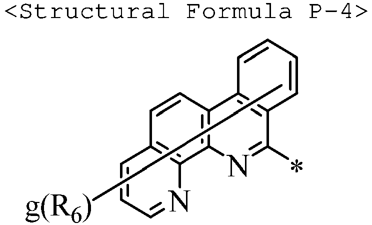

In the Structural Formula P-1 to Structural Formula P-4, R6 has the same definition as R1 in the above Chemical Formula 1,
g may each independently be an integer from 0 to 9, and
"*" indicates a position bonded to L in the above chemical formula 1.

Specifically, the above R6, like the aforementioned R1, can each independently be one of hydrogen, deuterium, a methyl group, -CD3, a phenyl group, a biphenyl group, a naphthyl group, a pyridine group, a pyrimidine group, a quinoline group, an isoquinoline group, a quinazoline group, and a naphthyridine group, and specifically can be hydrogen, a phenyl group, or a pyridine group, and even more specifically can be hydrogen or a phenyl group.

The above g may be an integer from 0 to 3, specifically an integer from 0 to 2, and more specifically 0 or 1.

Meanwhile, the bonding position of R1 may be preferably a position adjacent to N of 1,10-phenanthroline as shown in the following Chemical Formula 5. In other words, when fluorene is bonded adjacent to N located at position 1 based on 1,10-phenanthroline, R1 may be bonded at position 9, which is adjacent to N located at position 10. Based on this, according to one embodiment, Chemical Formula 1 may be expressed as Chemical Formula 5 below. The same applies to R6, and R6 in the structural formulas P-1 to P-4 may also be bonded immediately adjacent to N.

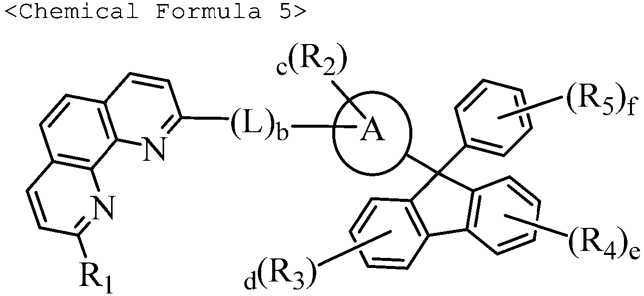

In the above Chemical Formula 5, L, R2 to R5 and b to f are the same as defined in the above Chemical Formula 1, and R1 is the same as described above. Specifically, R1 may be hydrogen, a phenyl group, or a pyridine group, and specifically, R1 may be hydrogen or a phenyl group. When R1 is a phenyl group, it may be preferable in terms of structural stability and thermal stability, and it may be preferable in terms of increasing π-conjugation and increasing electron mobility. Through this, it may be effective in lowering the operating voltage of the device and improving the lifespan.

Meanwhile, according to one embodiment of the present invention, L may each independently be a direct bond, a substituted or unsubstituted C6~C20 arylene group, or a substituted or unsubstituted C2~C20 heteroarylene group. More specifically, L may each independently a direct bond, a substituted or unsubstituted phenylene group, a substituted or unsubstituted naphthylene group, a substituted or unsubstituted biphenylene group, a substituted or unsubstituted terphenylene group, a substituted or unsubstituted phenanthrenylene group, a substituted or unsubstituted anthracenylene group, a substituted or unsubstituted chrysenylene group, a substituted or unsubstituted benzophenanthrenylene group, a substituted or unsubstituted benzoanthracenylene group, a substituted or unsubstituted pyrenylene group, a substituted or unsubstituted diphenylfluorenylene group, a substituted or unsubstituted dimethylfluorenylene group, a substituted or unsubstituted spirobifluorenylene group, a substituted or unsubstituted pyridinylene group, a substituted or unsubstituted pyrimidinylene group, a substituted or unsubstituted pyrazinylene group, a substituted or unsubstituted pyridazinylene group, a substituted or unsubstituted trizinylene group, a substituted or unsubstituted quinolinylene group, a substituted or unsubstituted isoquinolinylene group, a substituted or unsubstituted quinazolilene group, a substituted or unsubstituted quinoxalylene group, a substituted or unsubstituted phthalazinylene group, a substituted or unsubstituted cinnolinylene group, a substituted or unsubstituted 1,5-naphthyridinylene group, a substituted or unsubstituted 1,6-naphthyridinylene group, a substituted or unsubstituted 1,7-naphthyridinylene group, a substituted or unsubstituted 1,8-naphthyridinylene group, a substituted or unsubstituted 2,5-naphthyridinylene group, a substituted or unsubstituted 2,6-naphthyridinylene group, a substituted or unsubstituted 2,7-naphthyridinylene group, a substituted or unsubstituted dibenzofuranylene group, a substituted or unsubstituted dibenzothiophenylene group, a substituted or unsubstituted benzoxazolilene group, a substituted or unsubstituted benzothiazolylene group or a substituted or unsubstituted benzoimidazolilene group.

According to an embodiment of the present invention, when b is 2 or more, multiple Ls may be the same or different. Specifically, b may be 0, 1, or 2. According to an embodiment of the present invention, L may each independently be one of the structural formulas below, but is not particularly limited thereto. In the case of a structure in which the left and right sides are asymmetrical among the structural formulas below, the left and right sides are not limited to the figure below and may be changed to all possible cases.

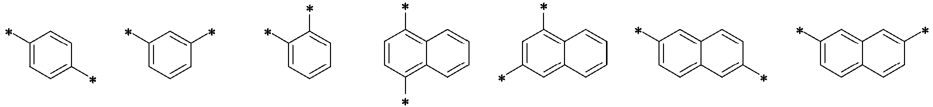

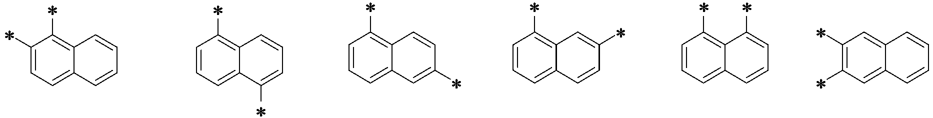

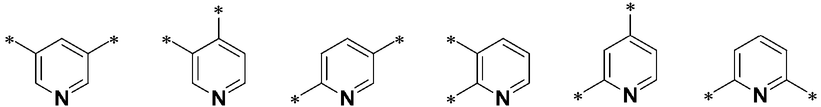

Meanwhile, according to one embodiment of the present invention, R2 to R5 may each independently be hydrogen or deuterium, and specifically, all may be hydrogen. In this way, by excluding the introduction of additional functional groups to fluorene and positioning fluorene at the terminal, the packing density and refractive index can be increased, resulting in an increase in the lifespan of the device.

Meanwhile, according to one embodiment of the present invention, at least one group among R3, R4, and R5 is bonded to adjacent one to form a ring unsubstituted or substituted with one or more deuteriums, and specifically, may form a benzene unsubstituted or substituted with one or more deuteriums. In this case, the fluorene moiety in the above Chemical Formula 1 may be specifically represented by one of the following Structural Formula F-1 to Structural Formula F-5.

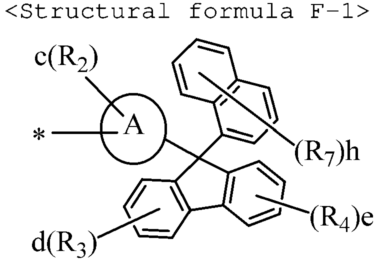

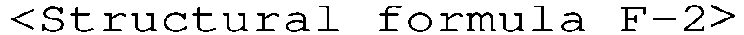

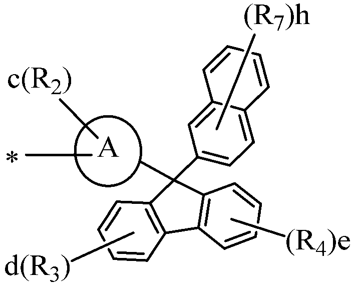

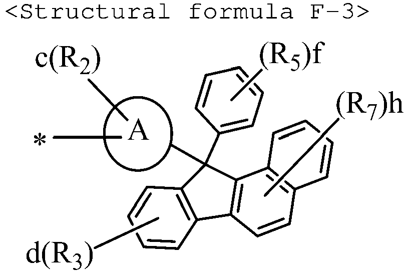

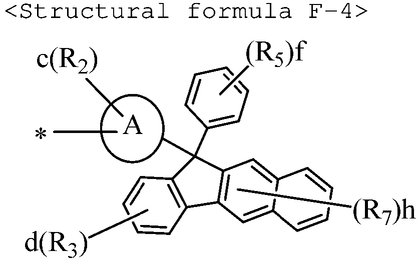

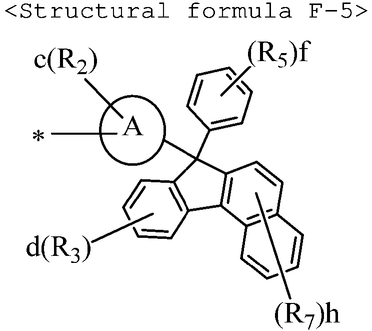

In the Structural formula F-1 to F-5, ring A is the same as defined in the Chemical Formula 1,
R2 to R5 and R7 are each independently hydrogen or deuterium,
c to f are the same as defined in the Chemical Formula 1,
h is each independently an integer from 0 to 6, and
"*" indicates a position bonded to L, a linker in Chemical Formula 1.

Specifically, R2 to R5 and R7 may all be hydrogen.

Based on the above, according to one embodiment of the present invention, the Chemical Formula 1 may be one of the following Chemical Formulas A to H.

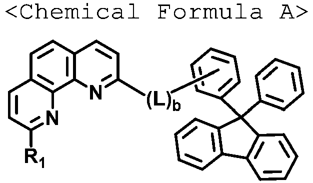

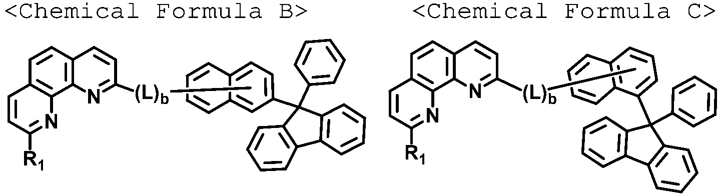

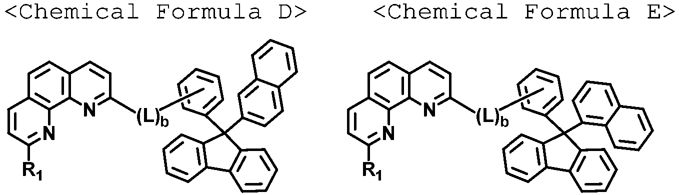

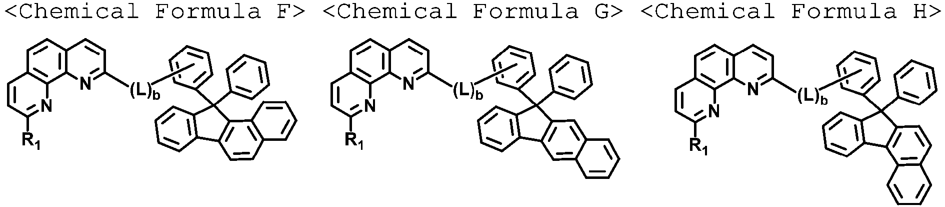

In the above Chemical Formulas A to H, R1, L and b are the same as defined in the Chemical Formula 1,
According to one embodiment of the present invention, it may be preferable that ring A is naphthyl or that one or more adjacent ones of R3 to R5 are combined with each other to form a ring. In other words, it may be preferable that the fluorene substituent includes naphthyl in the form of two benzenes fused together, as in the Chemical Formula B to Chemical Formula H. Through this structure, conjugation is increased, thereby increasing the stability of the molecule, improving electron mobility, and ensuring a low RE value below a certain level, thereby ultimately improving device performance such as high efficiency and long life. More specifically, the position of the naphthyl may be as in Chemical Formula B to Chemical Formula E, which are above the fluorene, and even more specifically, as in Chemical Formula B and Chemical Formula C.

According to one embodiment of the present invention, the compound represented by the Chemical Formula 1 may be selected from compound 1 to compound 543 below. The compounds below are merely examples for explaining the present invention, and the present invention is not limited thereto.

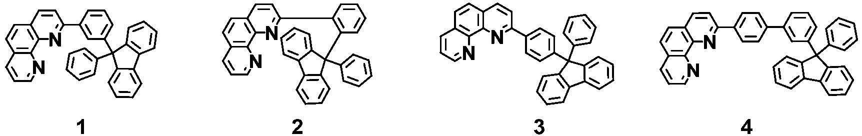

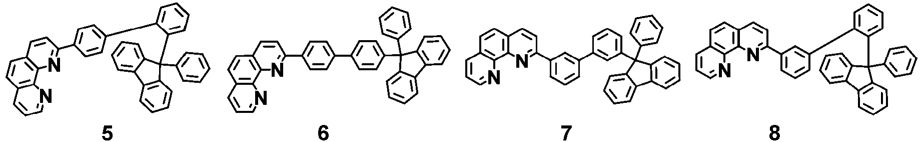

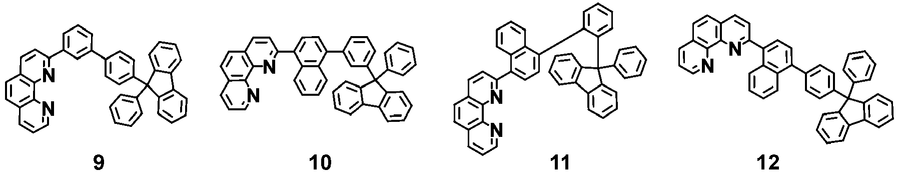

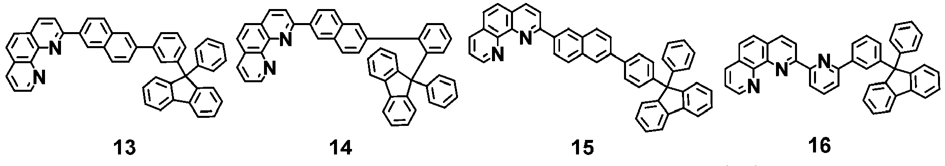

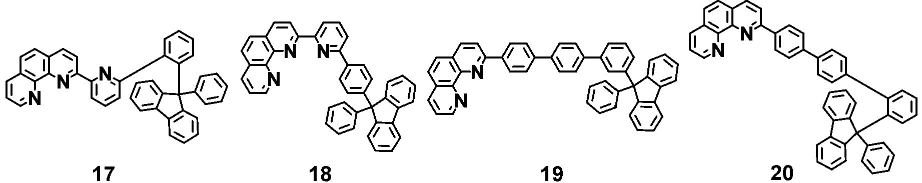

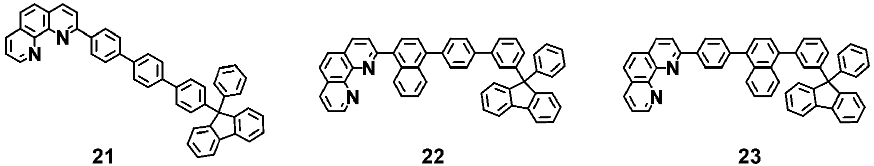

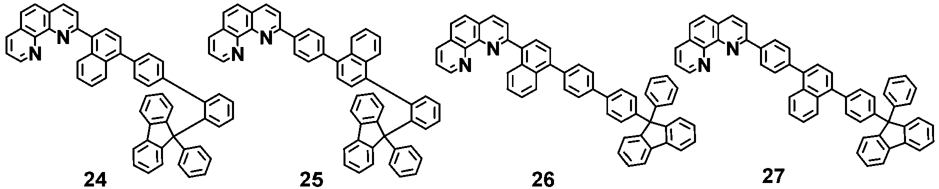

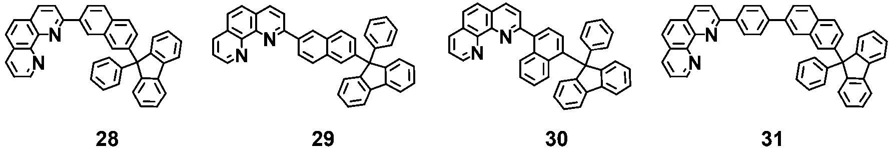

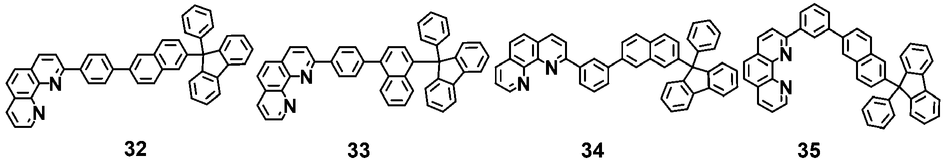

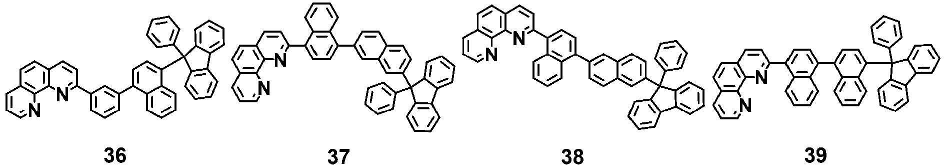

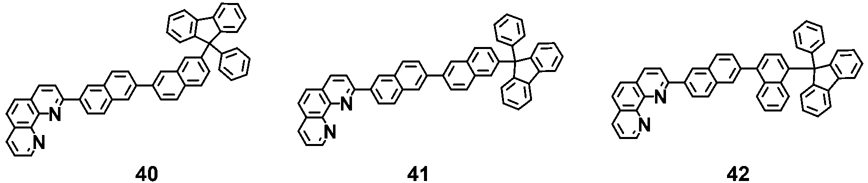

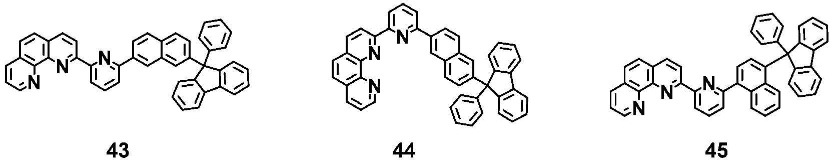

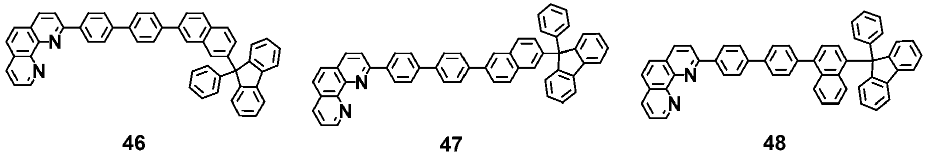

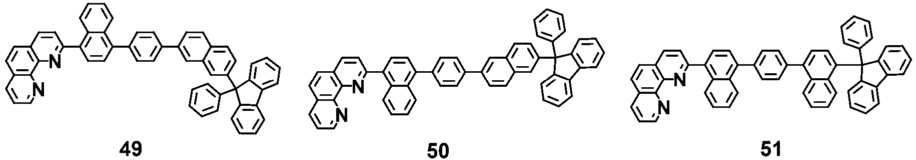

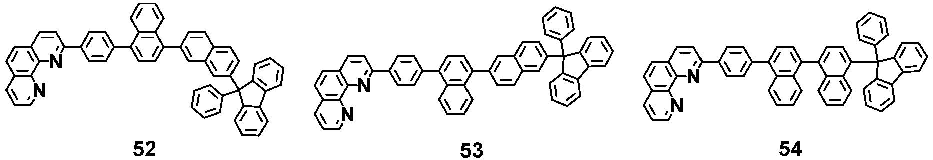

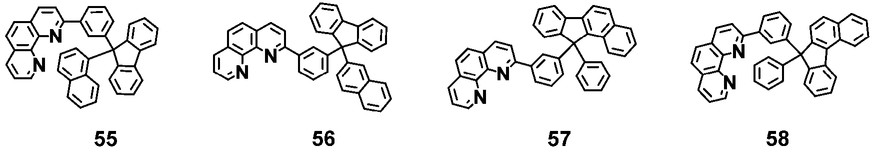

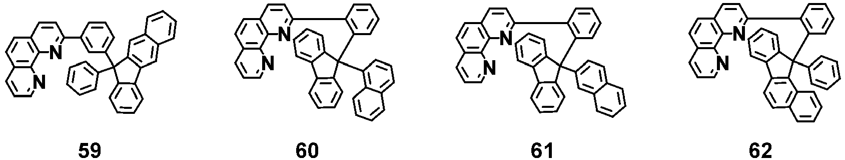

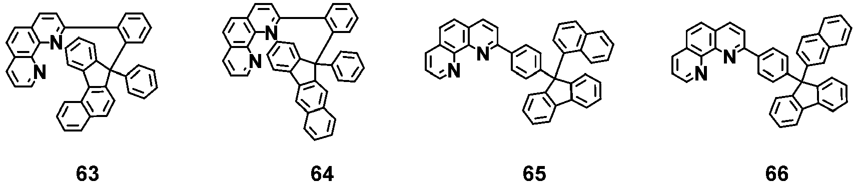

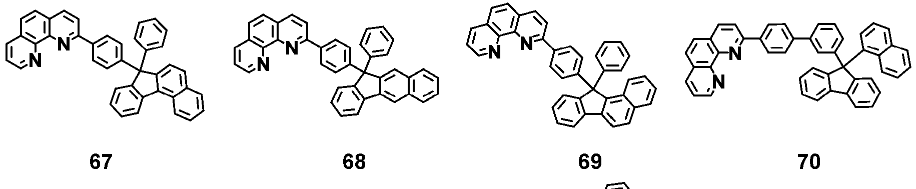

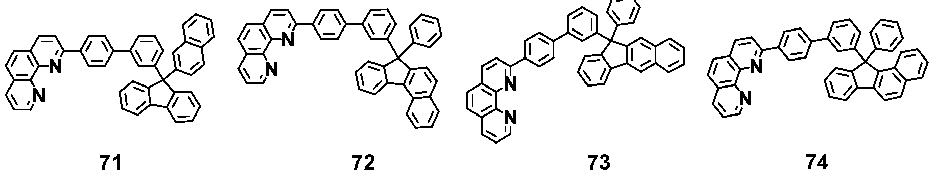

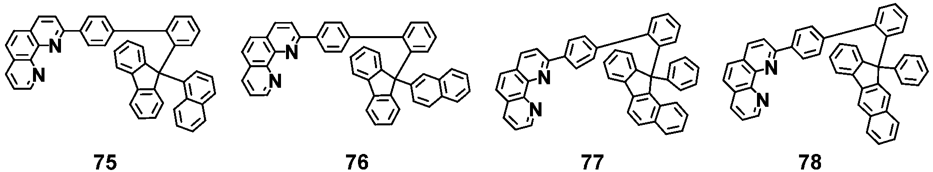

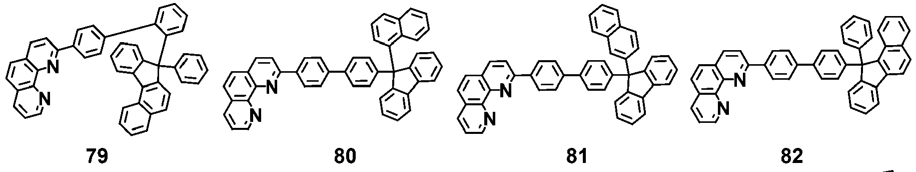

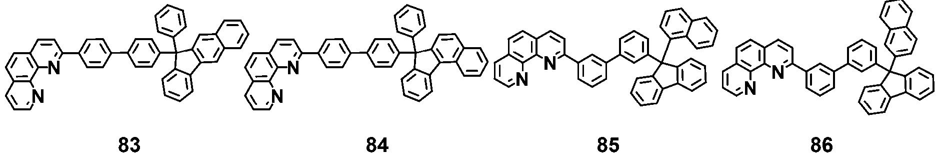

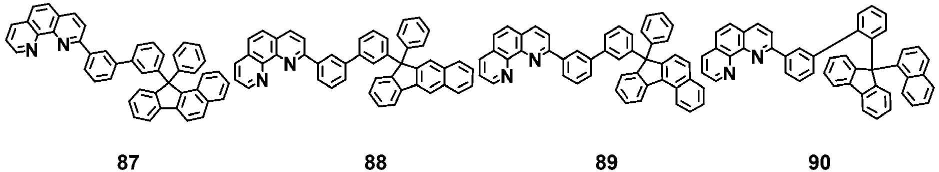

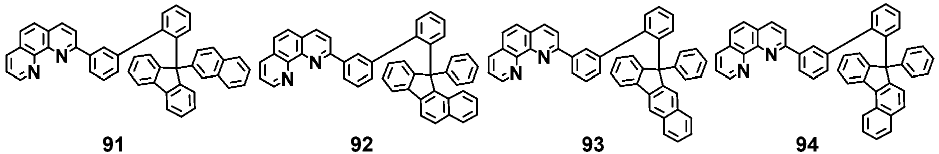

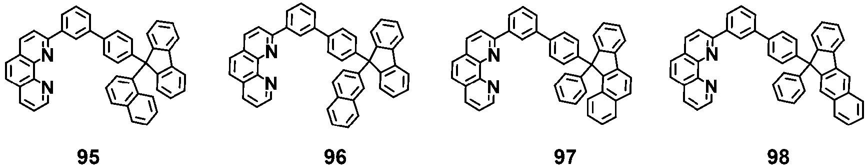

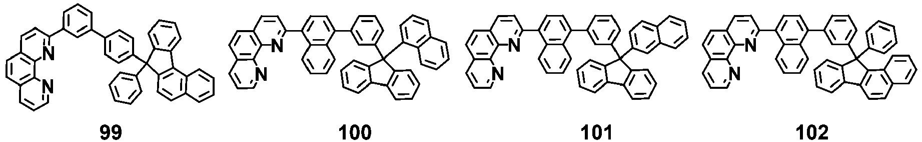

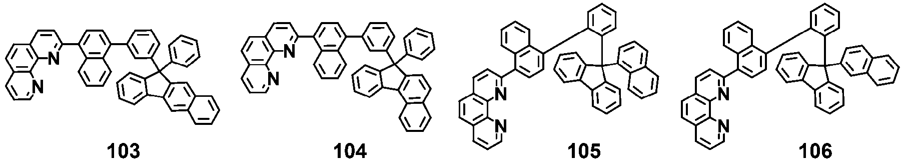

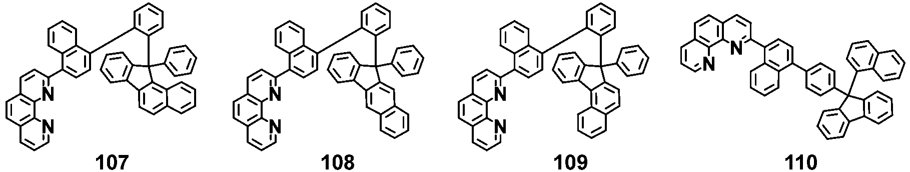

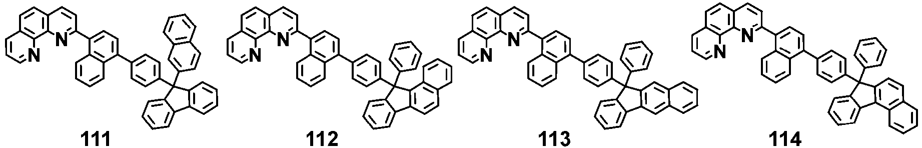

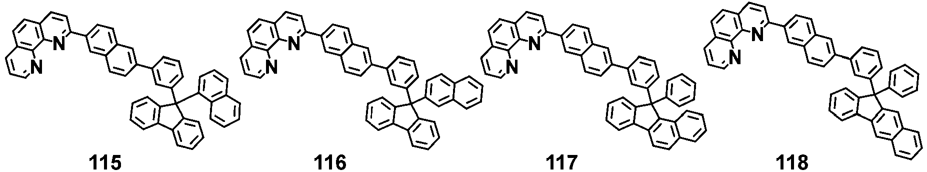

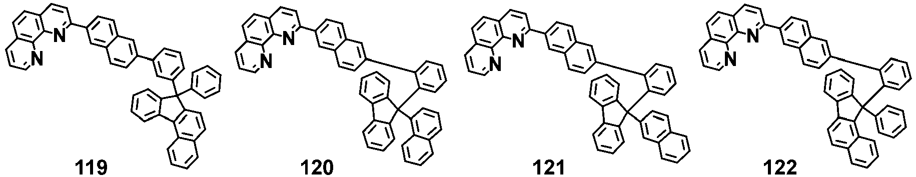

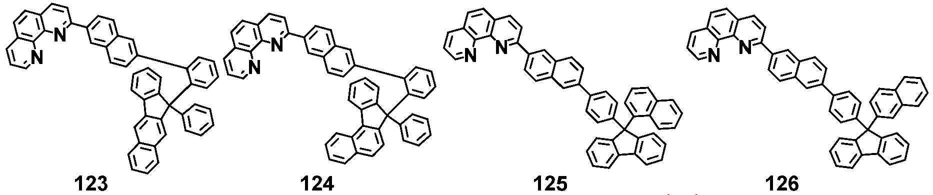

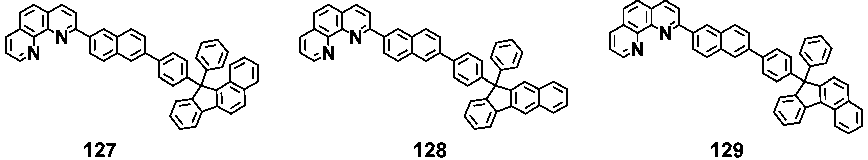

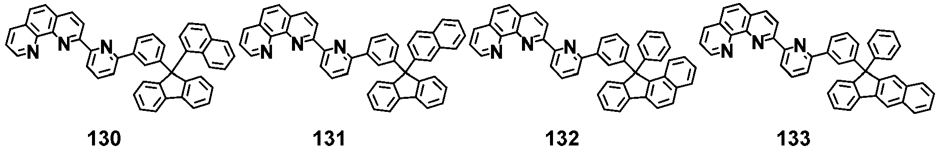

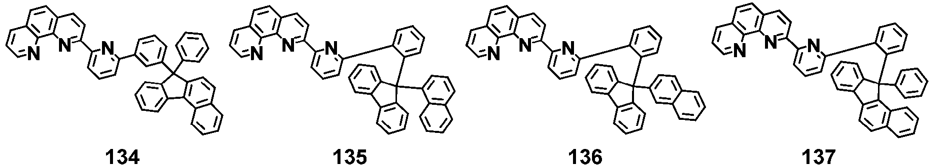

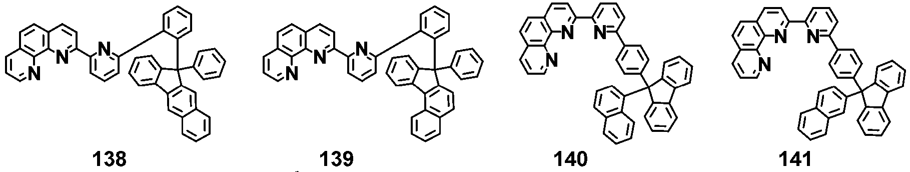

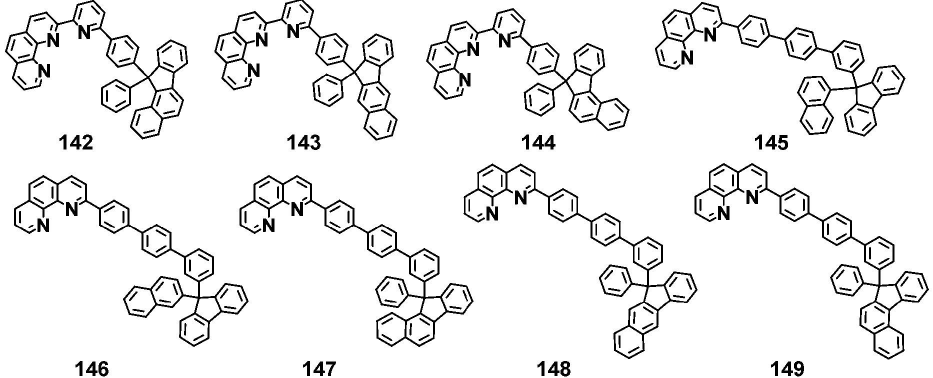

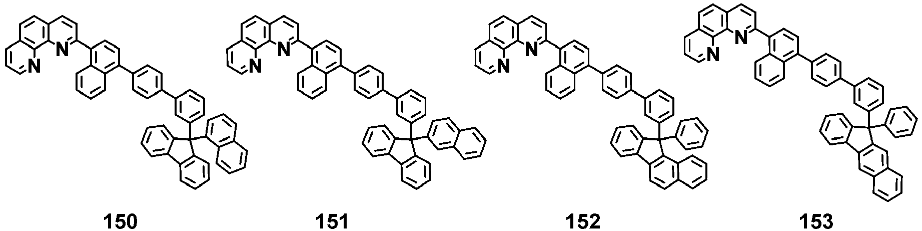

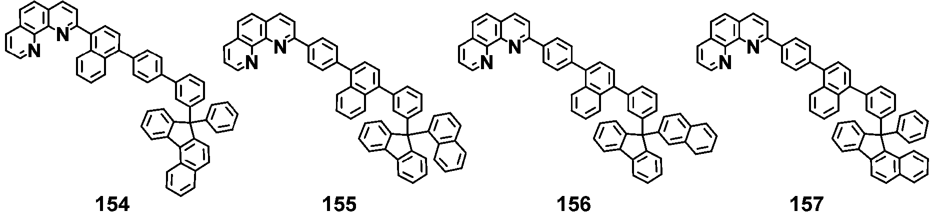

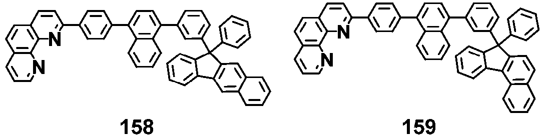

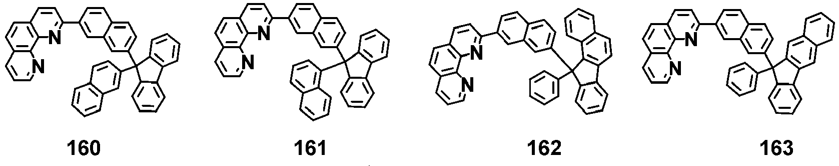

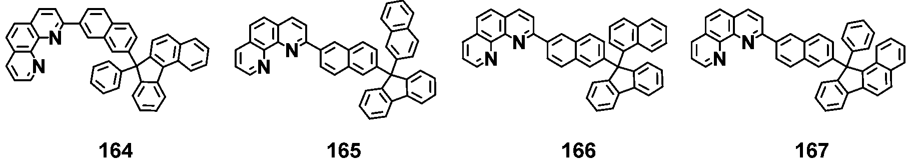

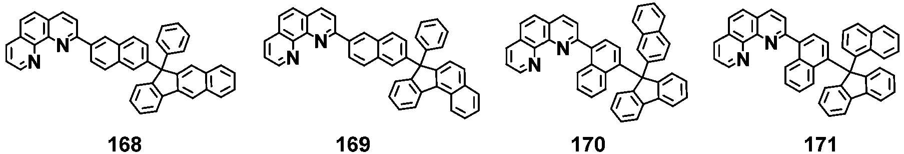

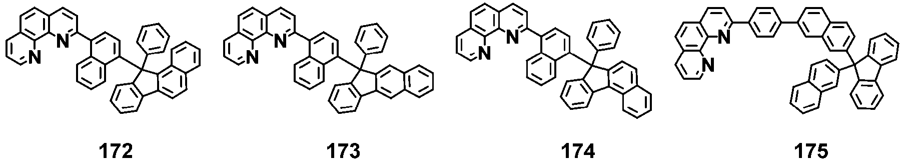

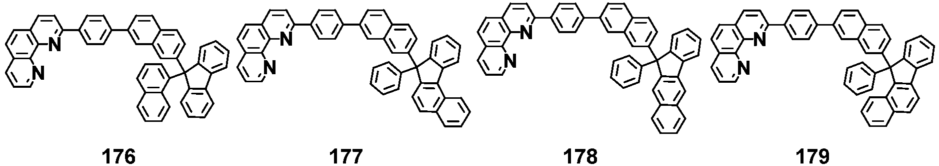

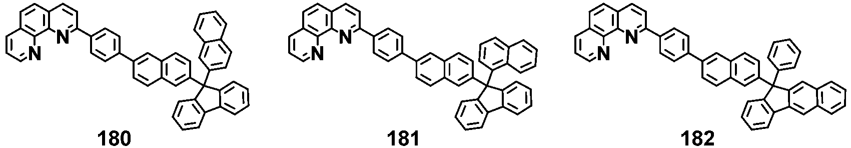

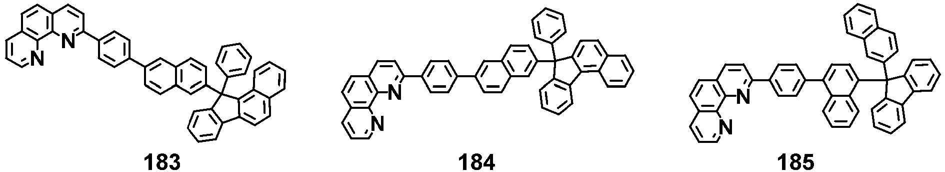

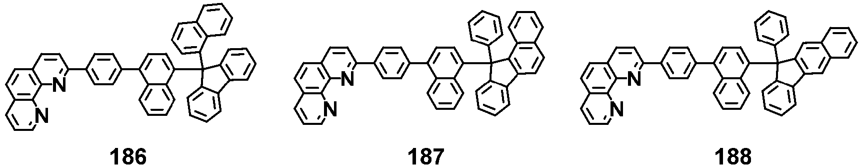

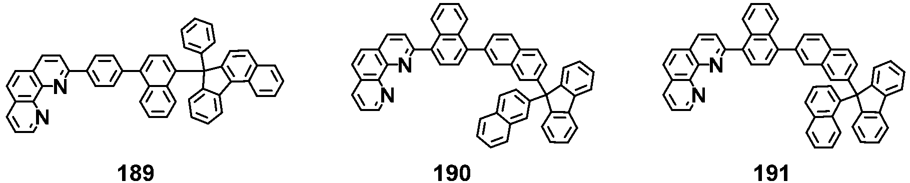

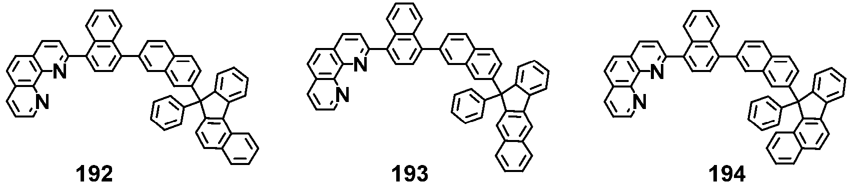

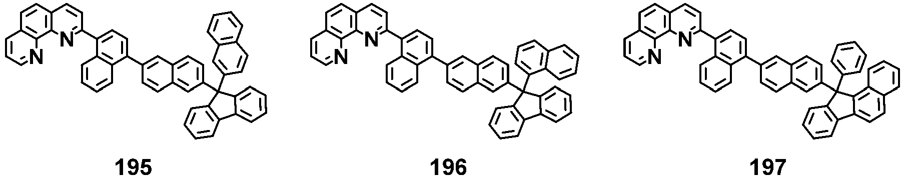

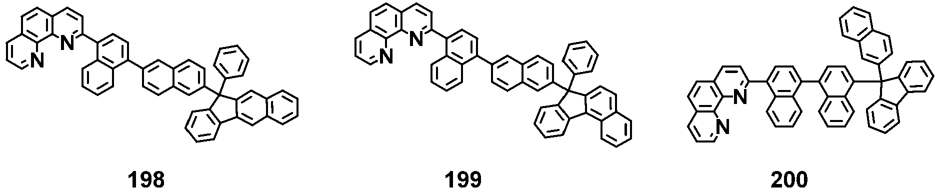

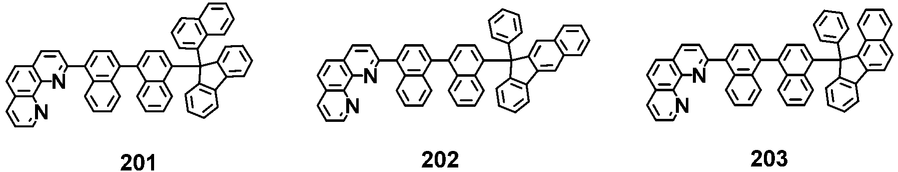

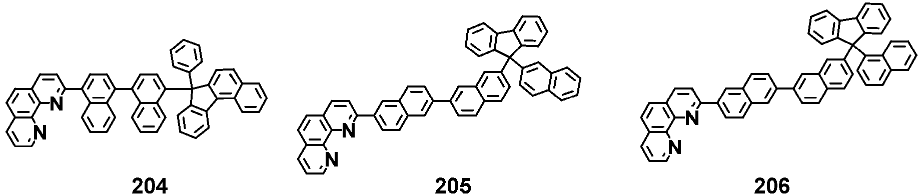

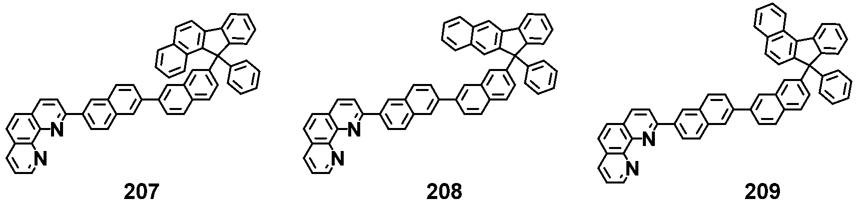

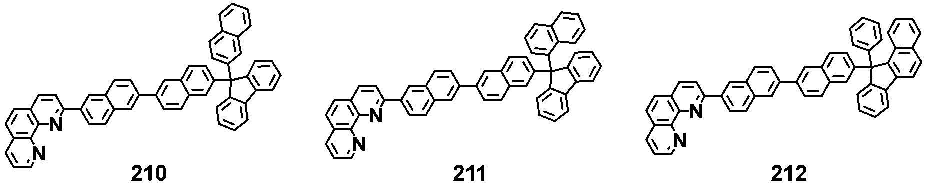

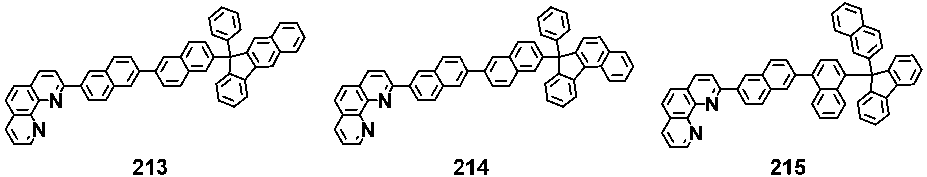

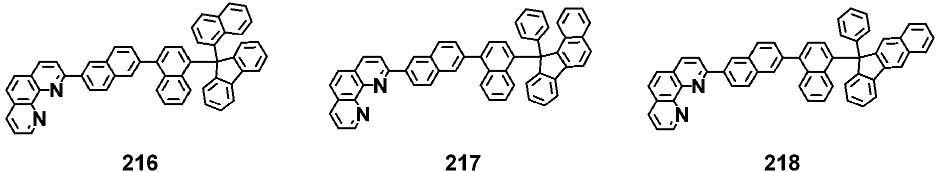

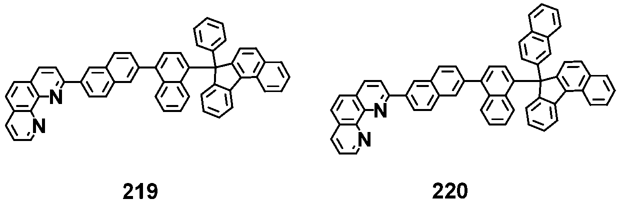

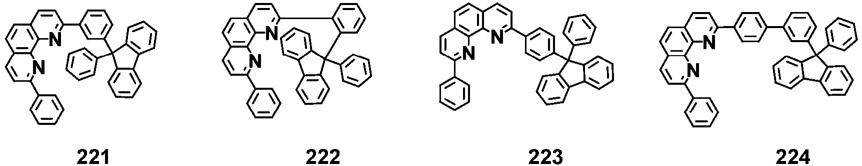

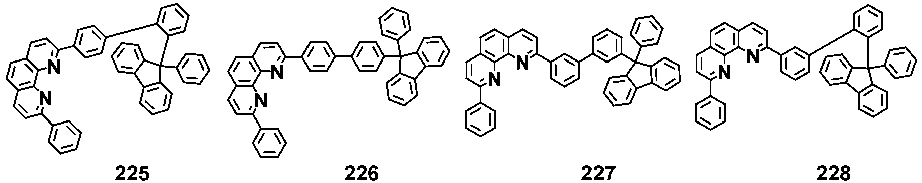

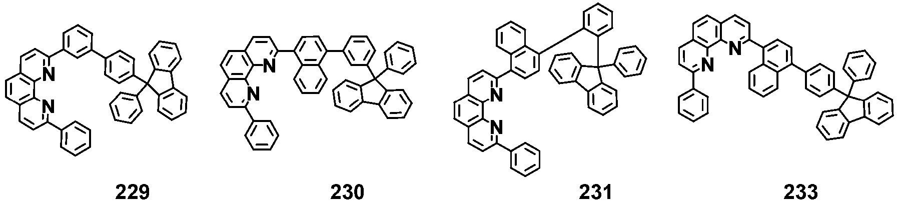

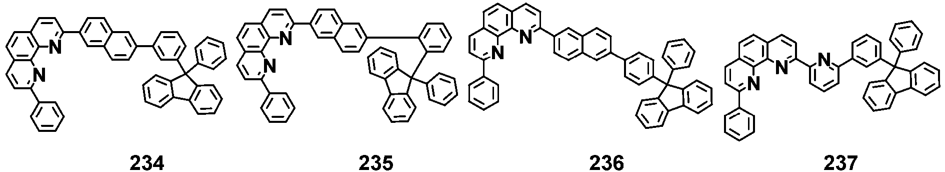

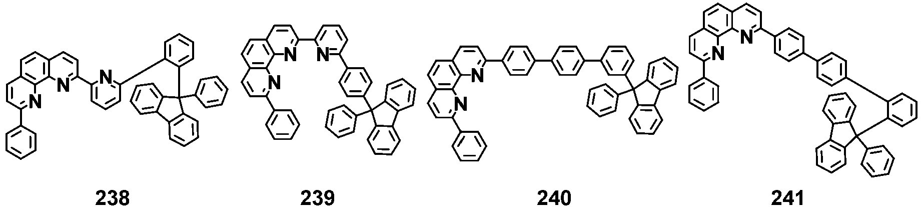

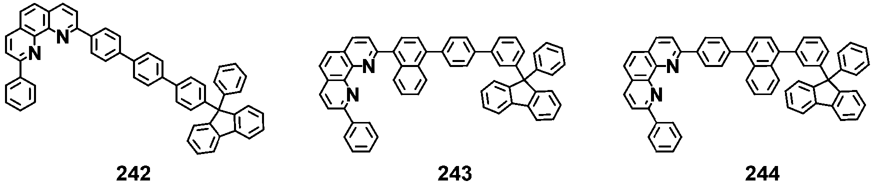

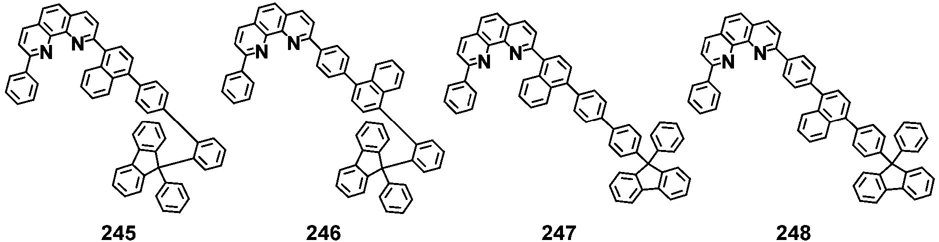

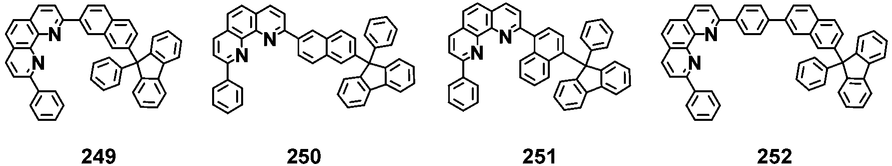

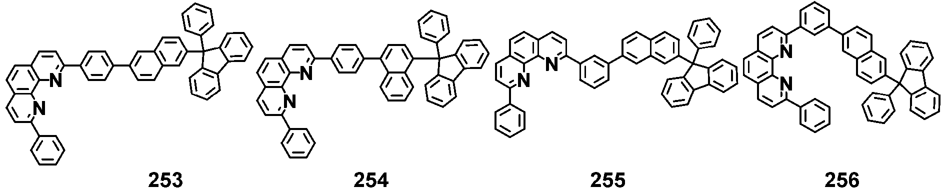

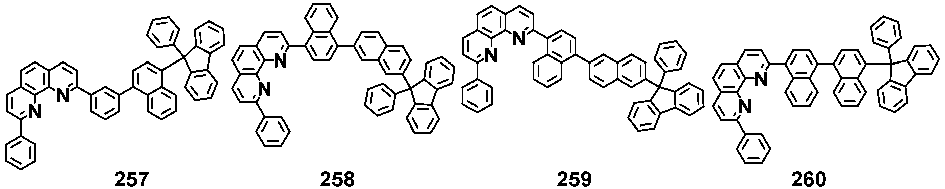

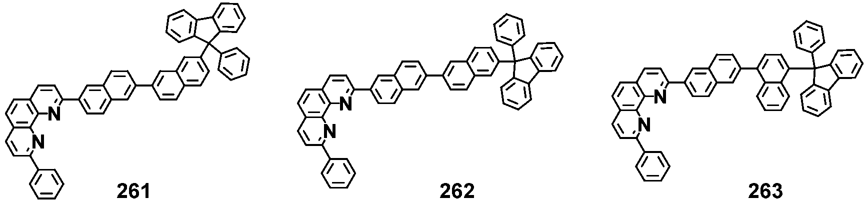

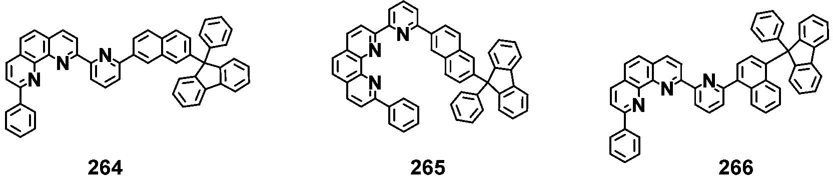

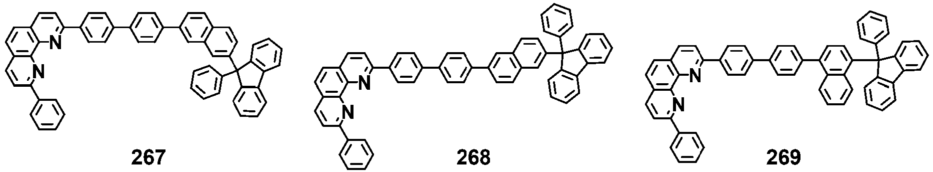

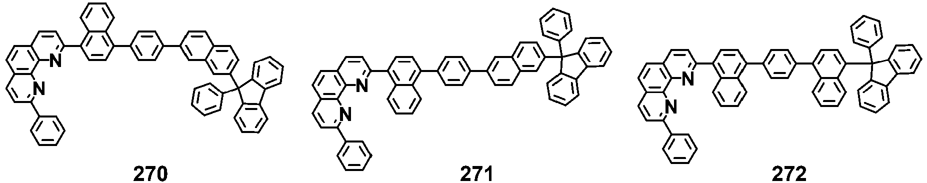

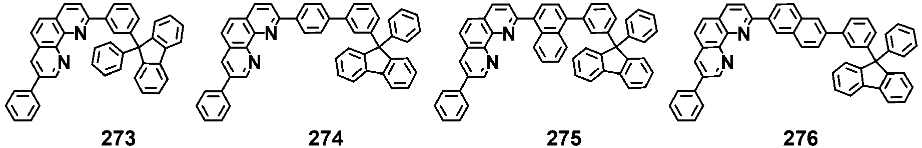

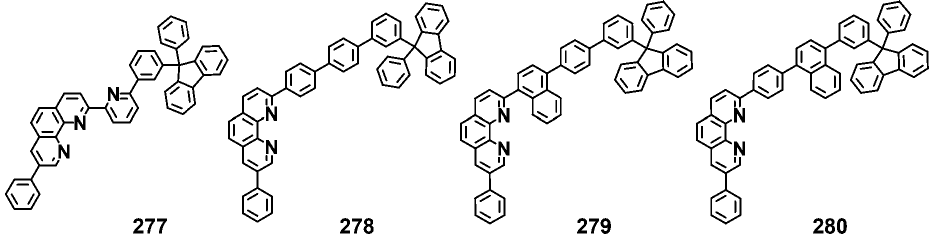

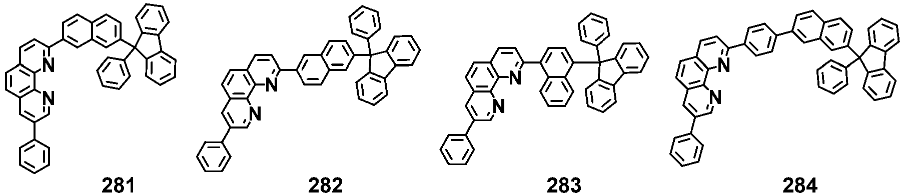

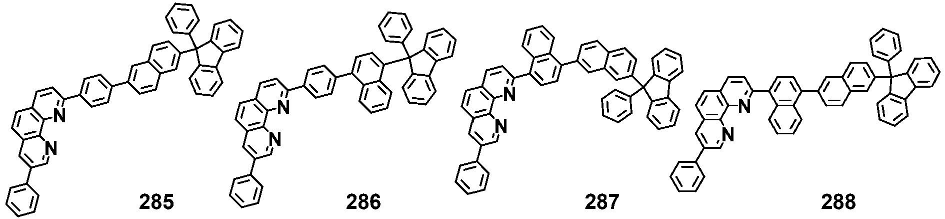

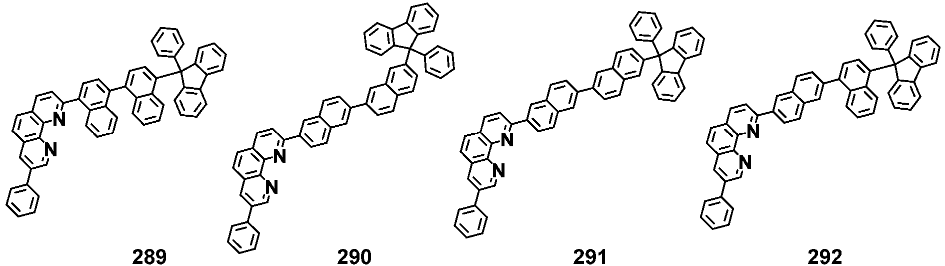

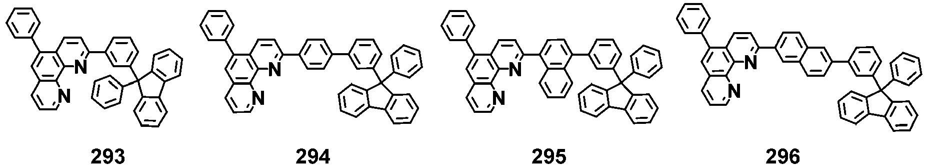

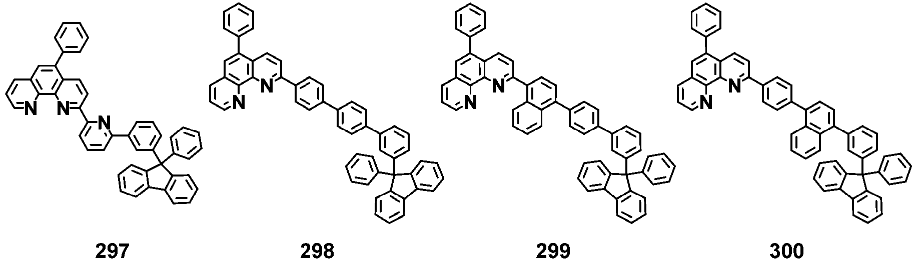

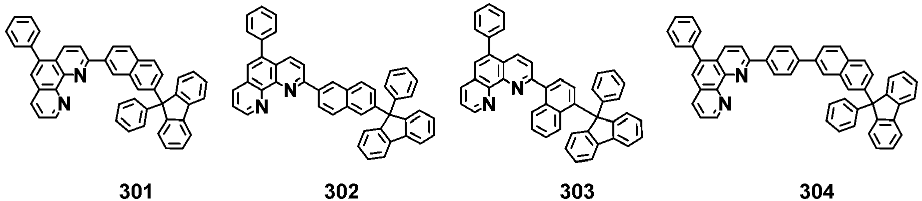

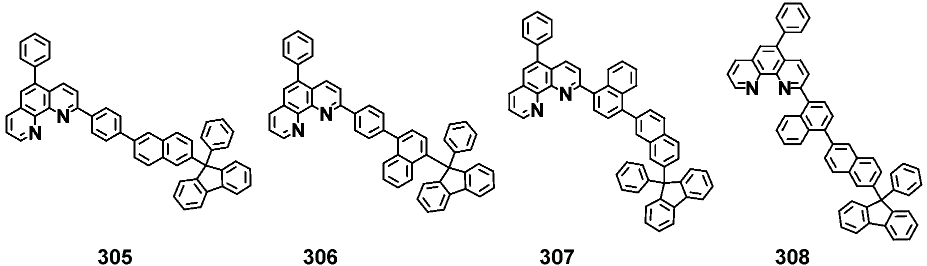

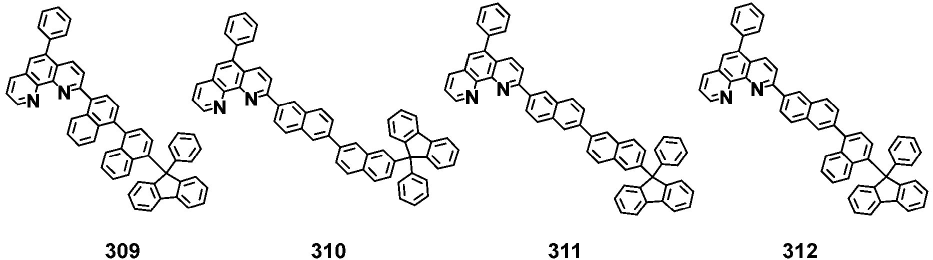

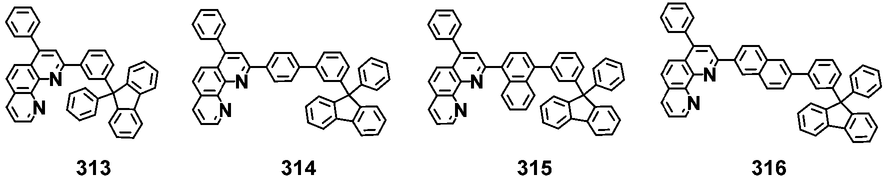

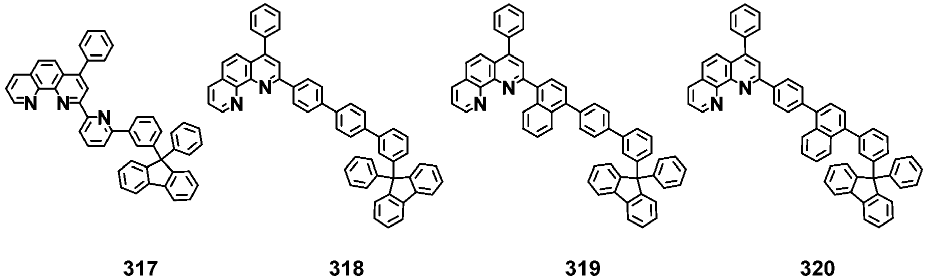

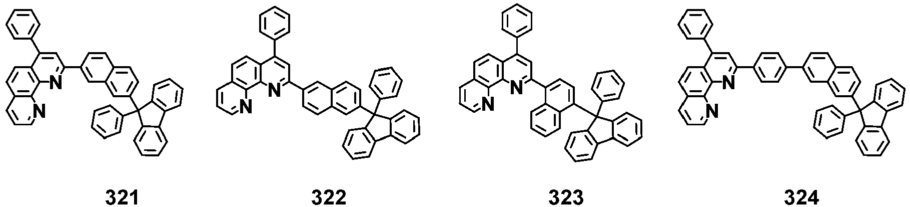

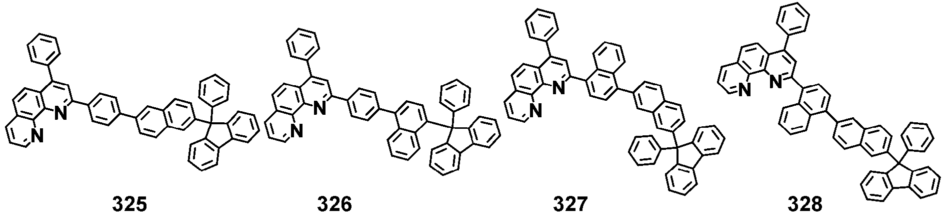

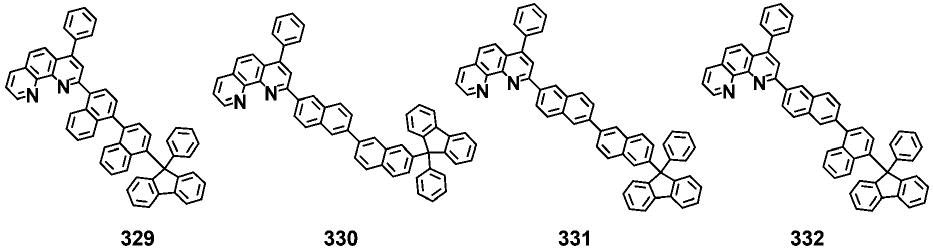

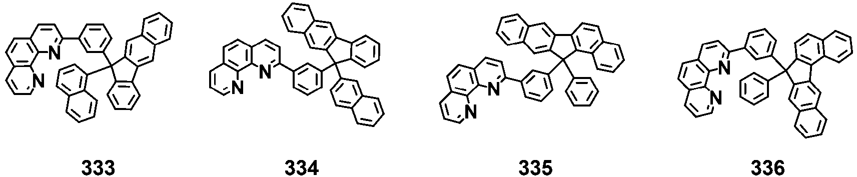

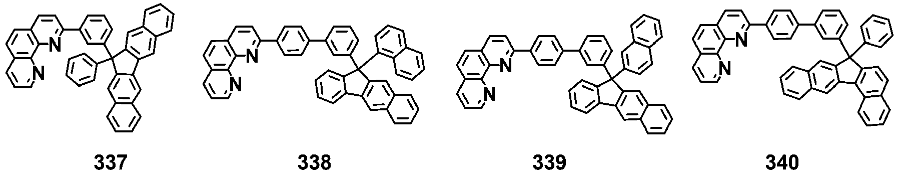

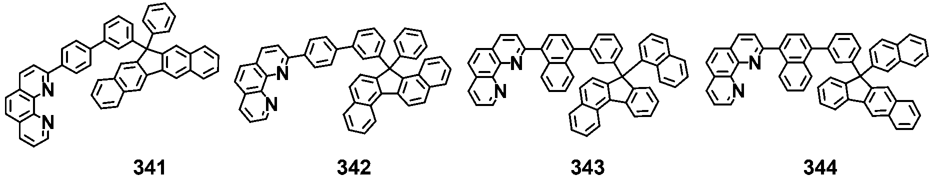

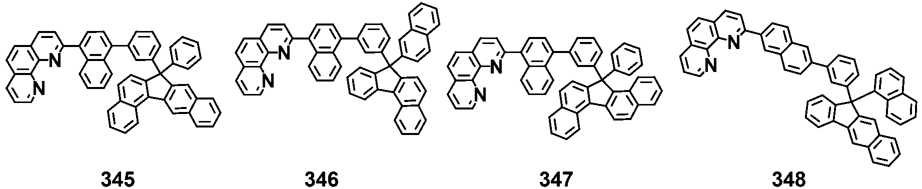

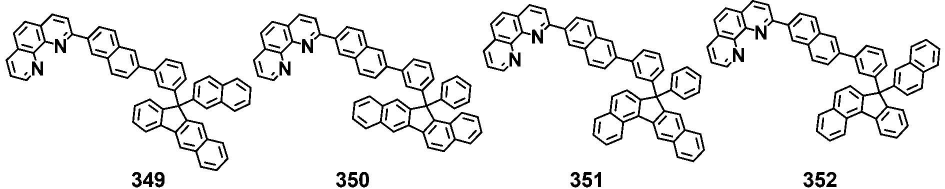

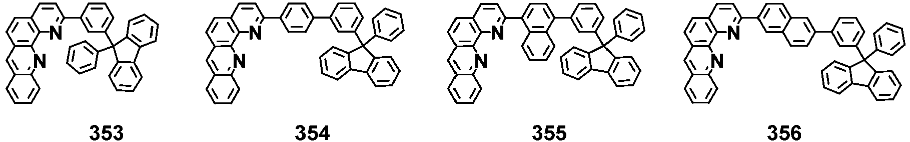

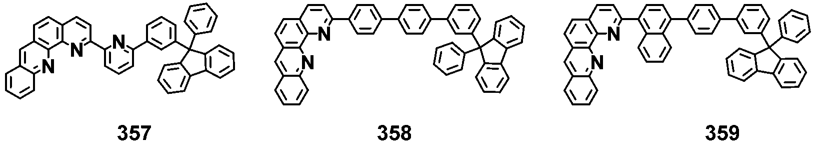

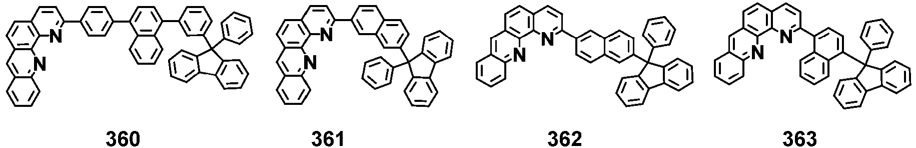

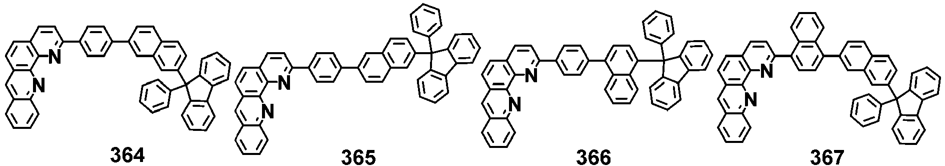

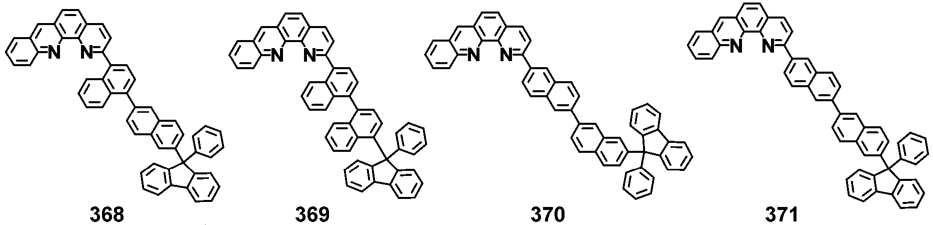

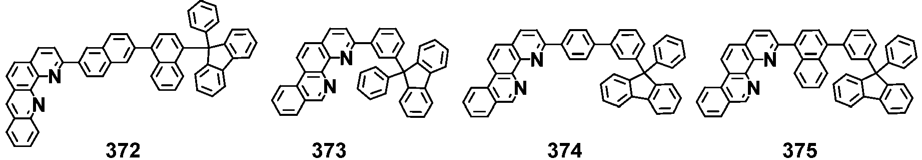

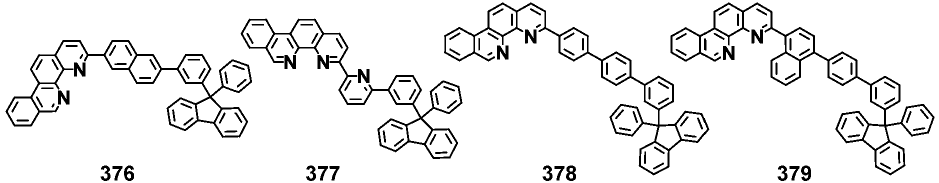

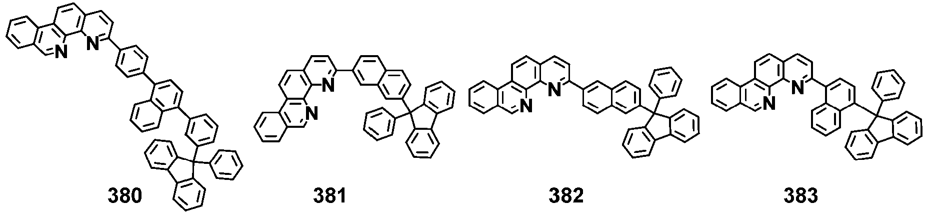

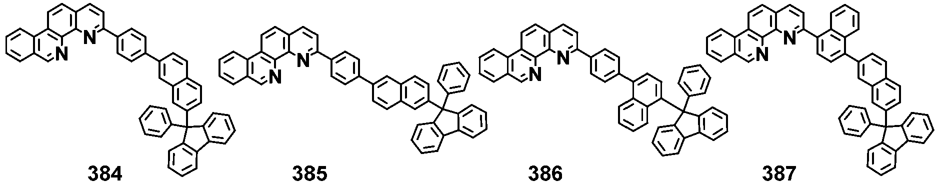

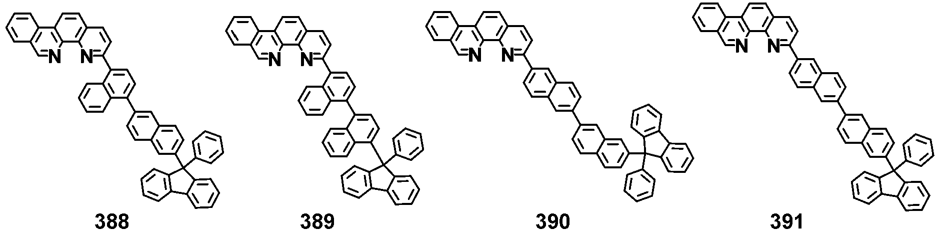

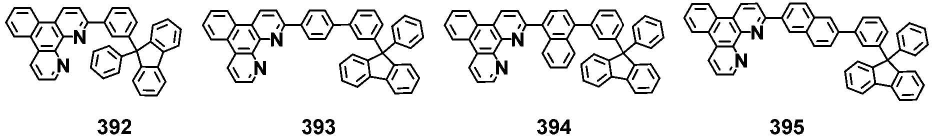

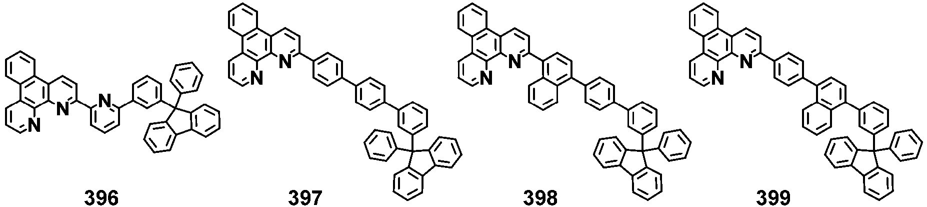

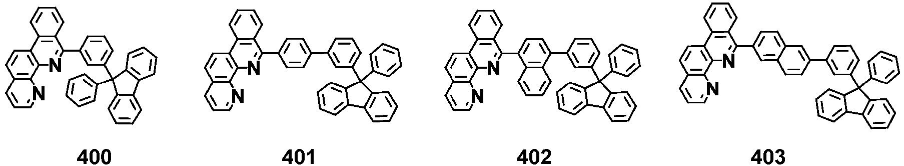

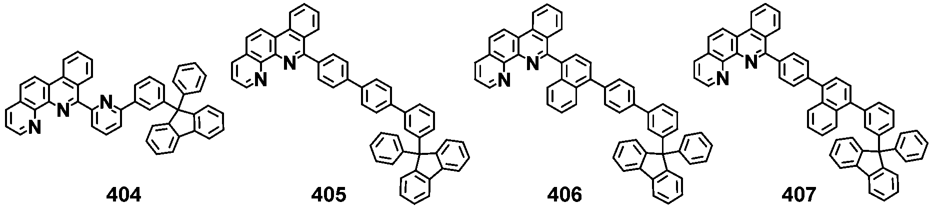

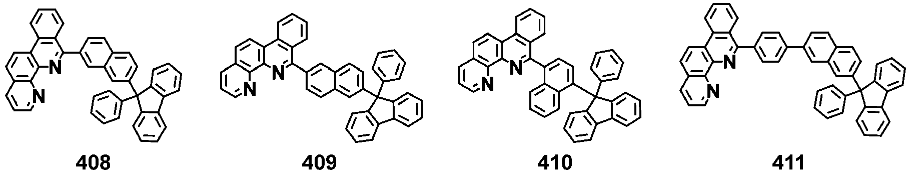

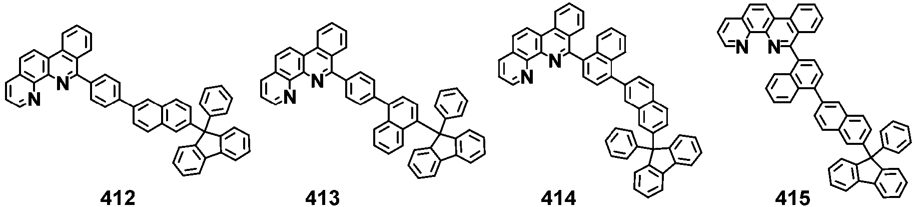

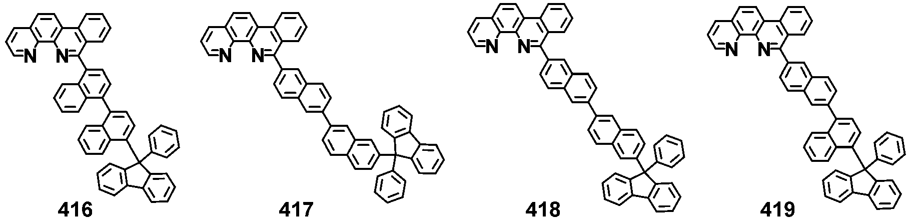

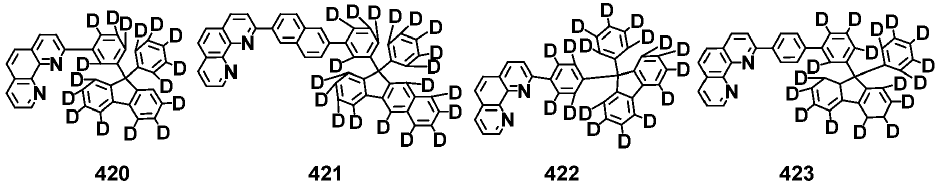

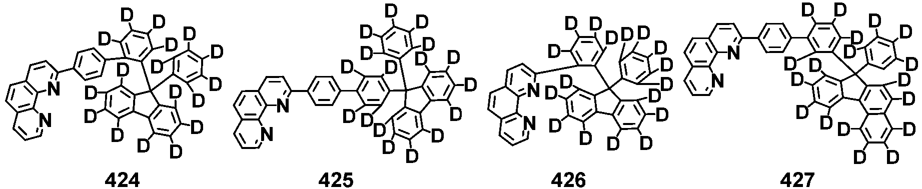

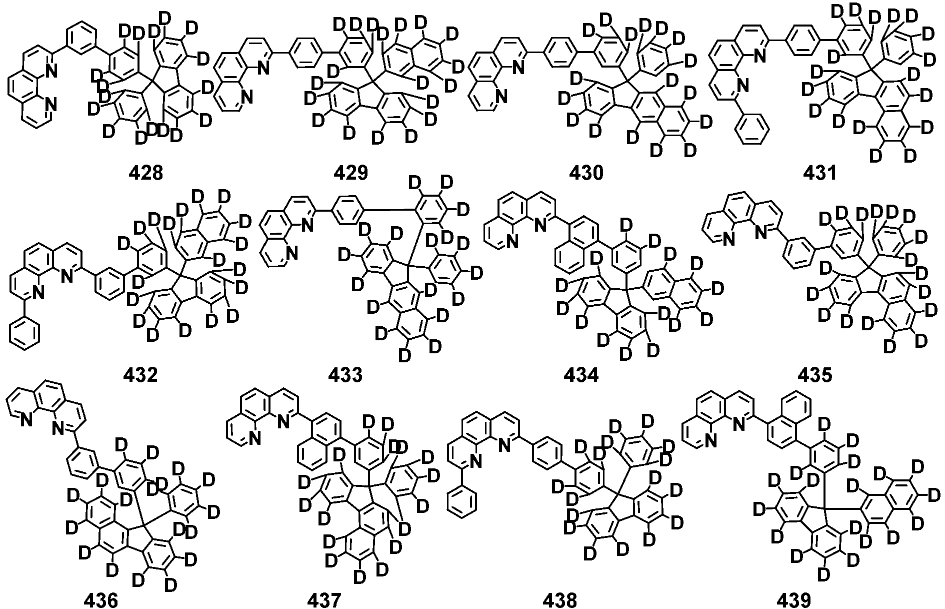

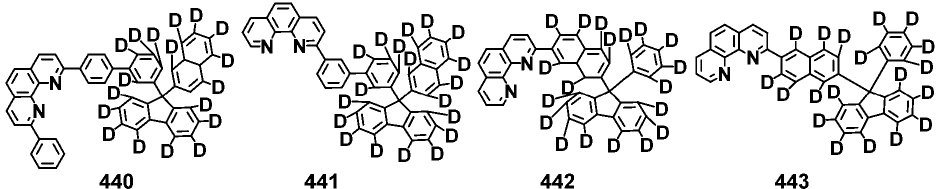

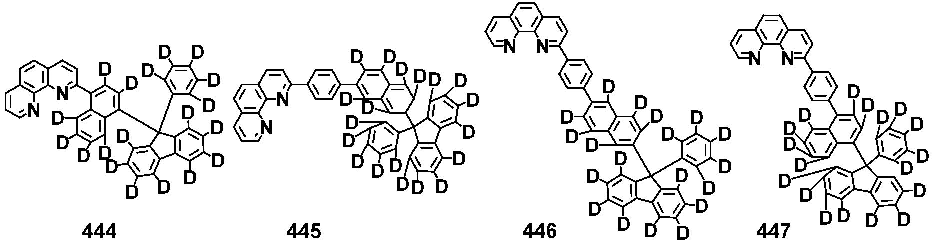

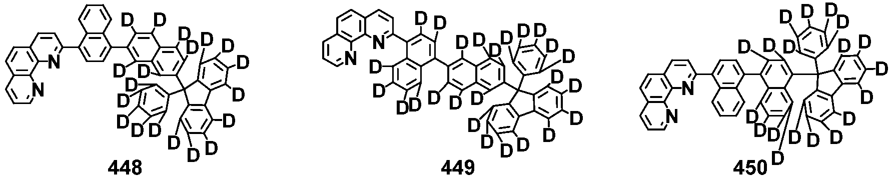

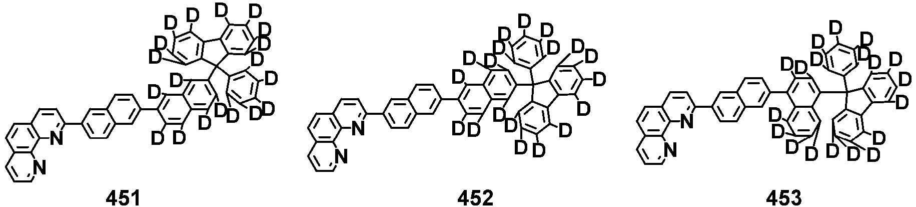

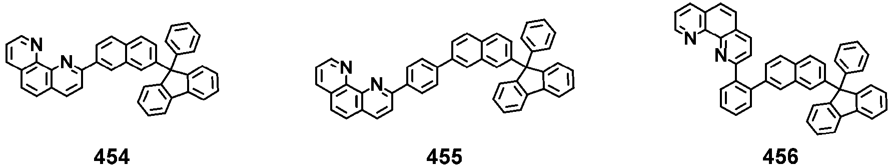

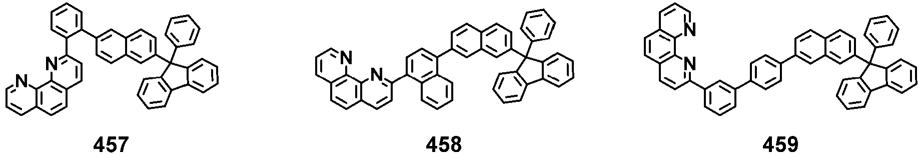

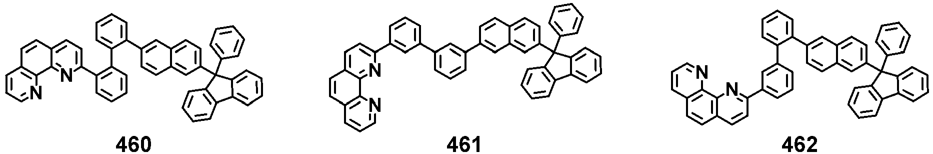

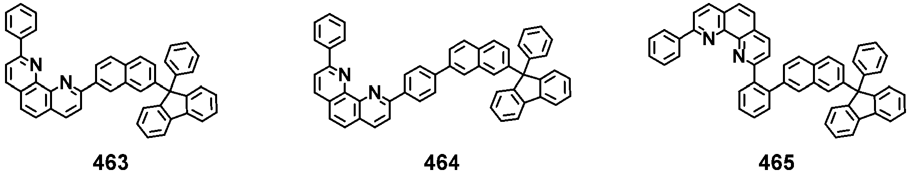

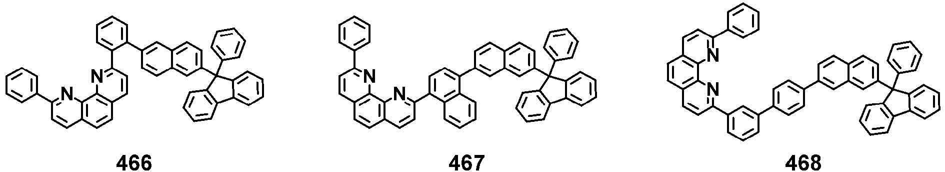

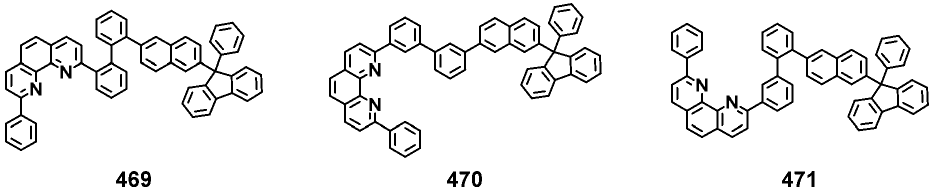

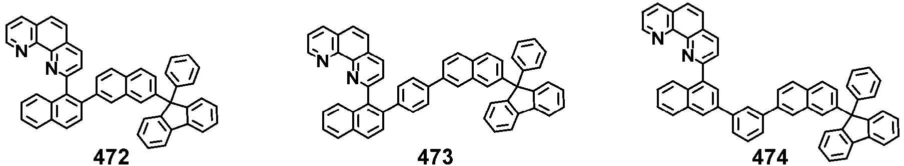

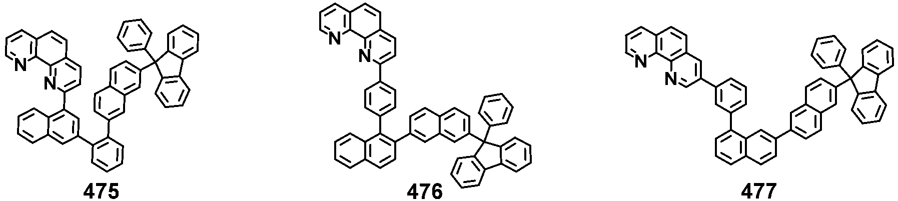

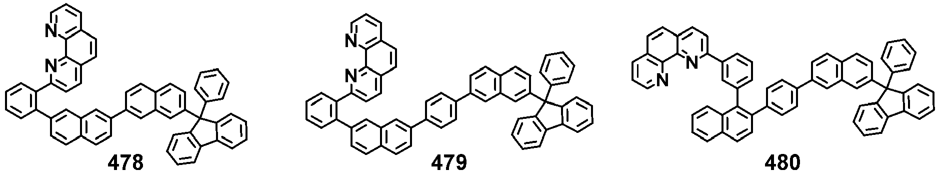

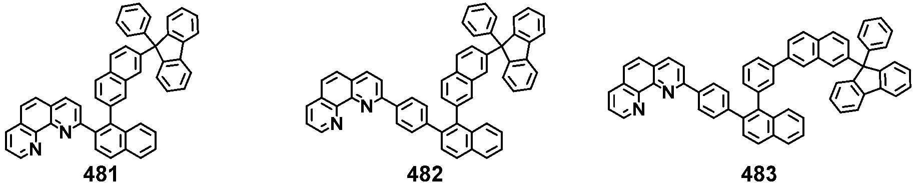

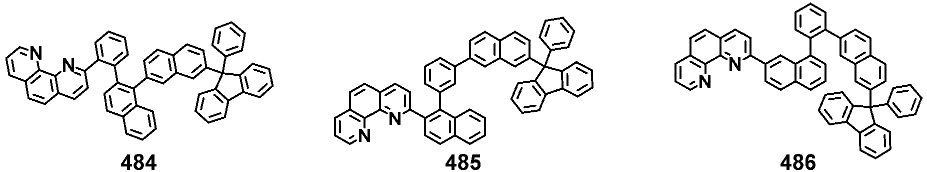

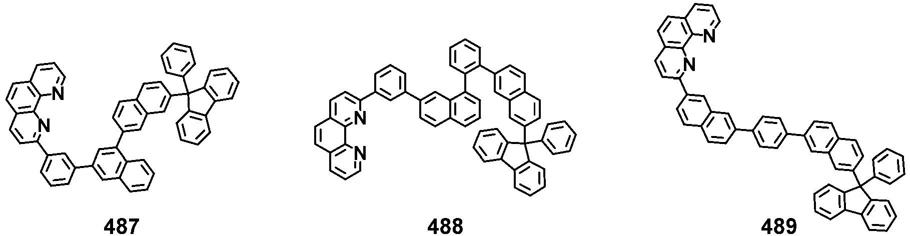

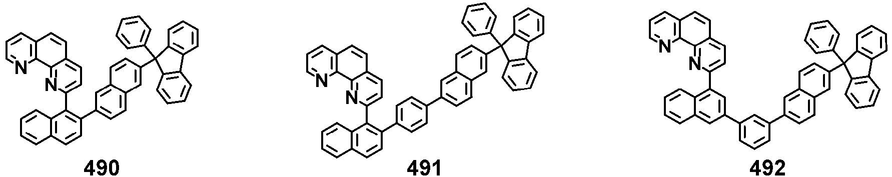

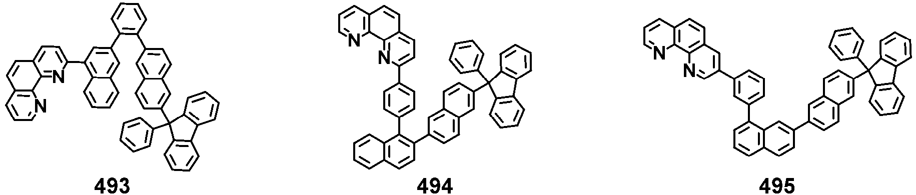

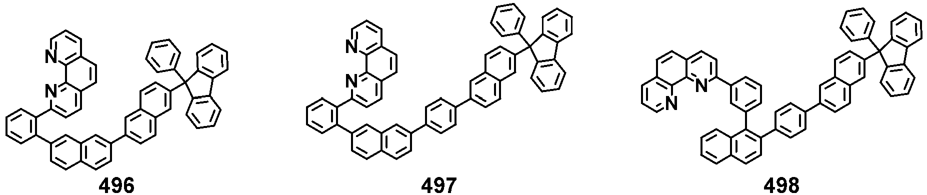

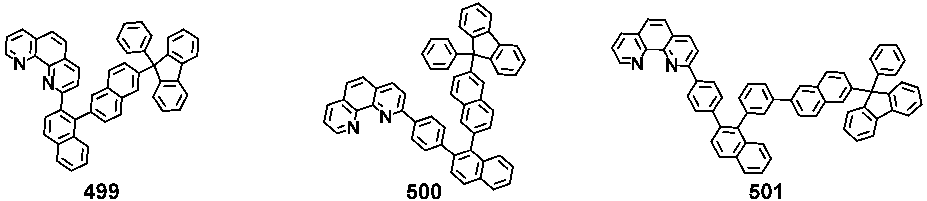

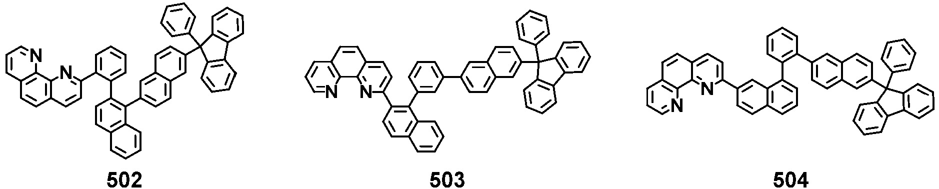

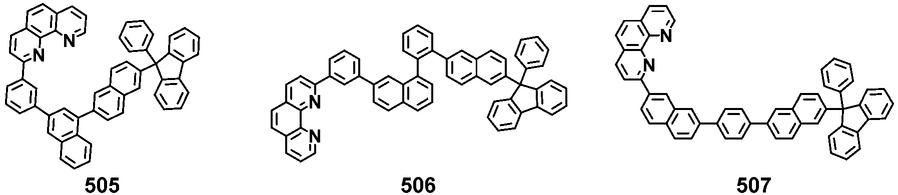

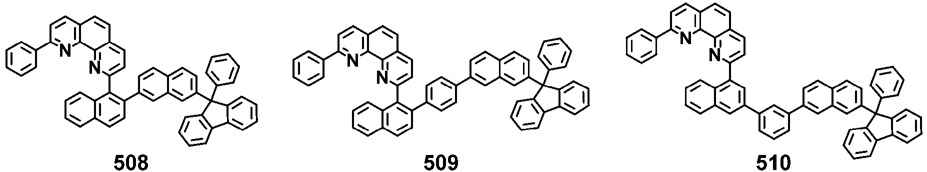

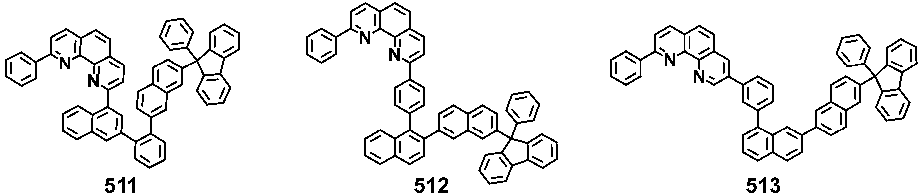

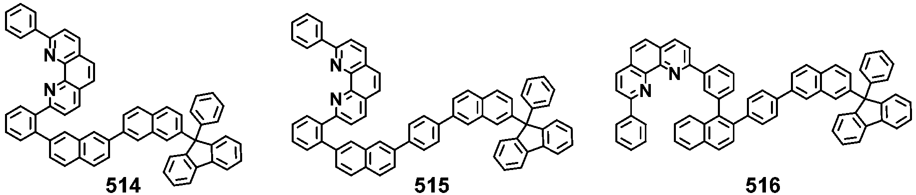

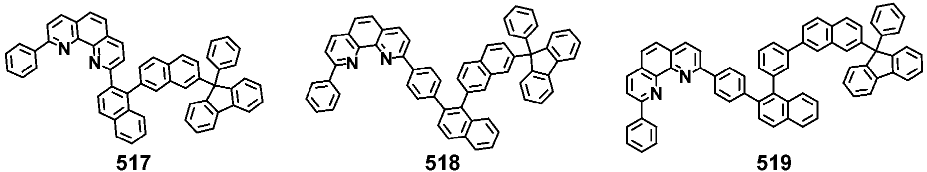

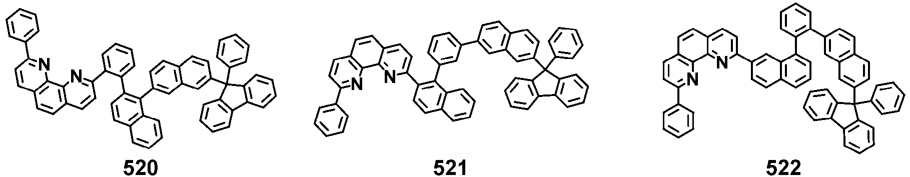

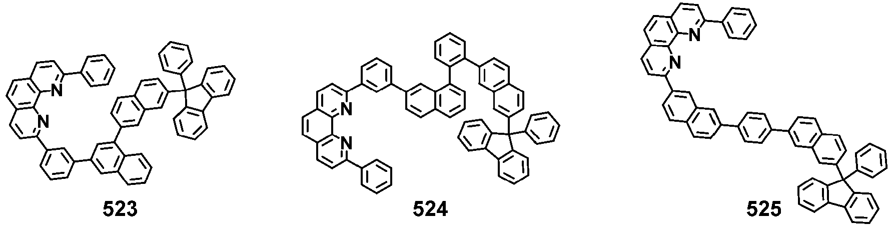

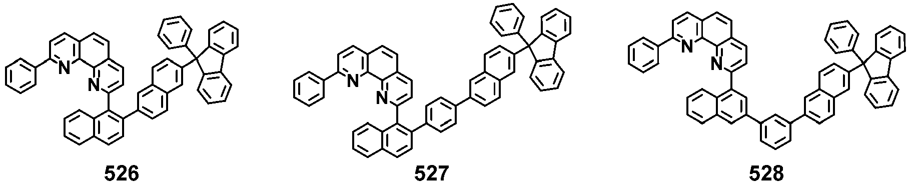

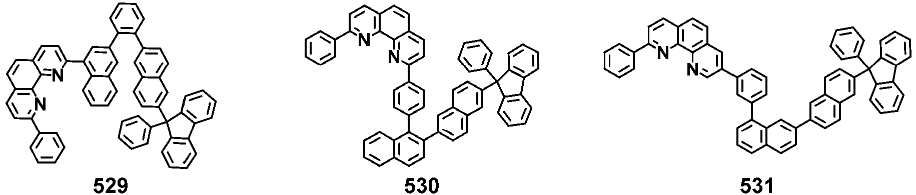

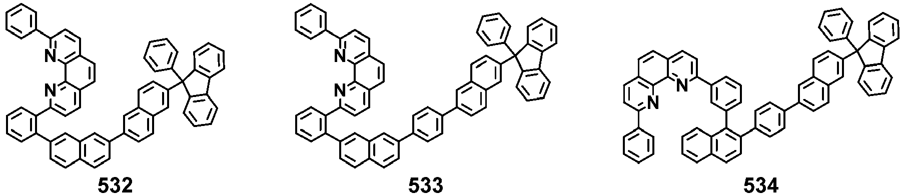

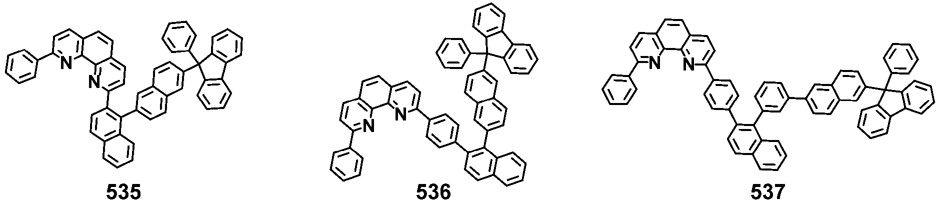

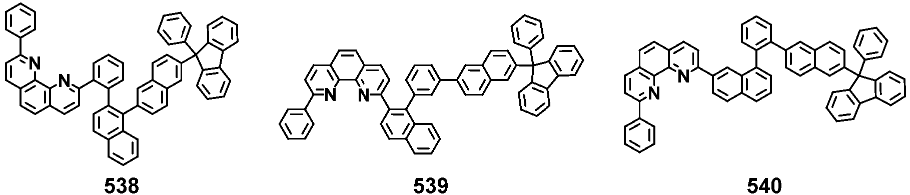

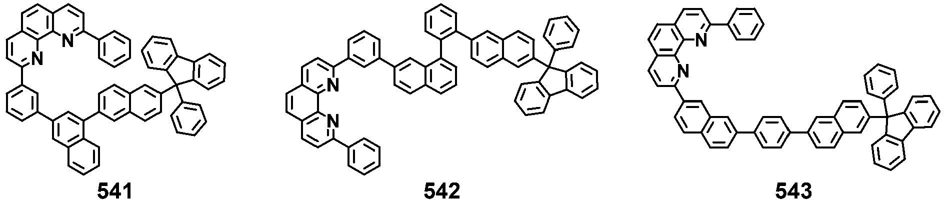

The novel compound according to an embodiment of the present invention described so far is particularly preferable for application to an N-type charge generation layer because it can easily combine with dopants such as lithium and ytterbium by introducing 1,10-phenanthroline having a non-shared electron pair, and also has excellent thermal stability by introducing a rigid fluorene-based substituent.

In addition, the compound has a reverse structure in which 1,10-phenanthroline is bonded to phenyl or naphthyl located above diphenylfluorene or naphthylphenylfluorene, and thus has a lower RE (Reorganization Energy) value compared to a structure in which it is bonded to the lower fluorene, thereby increasing the efficiency of the device.

In addition, the compound has a structure in which the position immediately adjacent to N of 1,10-phenanthroline is bonded, thereby expanding the conjugation and lowering the RE value compared to other positions, thereby increasing the efficiency of the device.

Furthermore, fluorene is not further substituted except for hydrogen and deuterium, so fluorene is located at the molecular terminal. This increases the packing density and refractive index, which in turn increases the lifespan of the device.

The novel compound of the present invention can be usefully applied to one or more organic layers of an electron transport layer (ETL), a hole blocking layer (HBL), and an N-type charge generation layer (N-CGL) among organic layers of an organic light-emitting device, and in particular, when applied to a host of an N-type charge generation layer of a tandem organic light-emitting device, it can enable the implementation of low voltage, high efficiency, and long life of the device.

In this specification, the RE value (reorganization energy) is calculated as (electron extraction potential, EEP) - (electron affinity, EA), where EEP is the energy when the structure changes from an anionic state to a cationic state, and EA refers to the anion energy in the ground state (neutral). When this RE value is lowered below a certain level, it may be desirable to improve the performance of the device, such as current efficiency.

The novel compound according to the present invention relates to a novel compound applicable to a charge generation layer (CGL) in a tandem device, particularly an N-type charge generation layer (nCGL), and can exhibit effects of increased efficiency, reduced operating voltage, and prolonged life of the device. In addition, it is not limited thereto, and can also be applied to organic layers such as an electron transport layer (ETL) and a hole blocking layer (HBL) where compounds with excellent electron mobility must be applied.

The present invention may include an organic light-emitting device comprising a first electrode, a second electrode, and at least one organic layer disposed between the first electrode and the second electrode, wherein at least one of the organic layers comprises a compound according to the Chemical Formula 1. The organic layer comprising the compound may be specifically an electron transport layer (ETL), a hole blocking layer (HBL), or a charge generation layer (CGL).

In addition, the present invention may include a tandem organic light-emitting device including a first electrode and a second electrode, a plurality of light-emitting members positioned between the first electrode and the second electrode, and a charge generation layer respectively positioned at one or more locations between any two adjacent light-emitting members, wherein at least one of the charge generation layers comprises an N-type charge generation layer including a compound represented by the Chemical Formula 1.

Hereinafter, the organic light-emitting device and tandem organic light-emitting device according to the present invention will be described in more detail.

The organic light-emitting device has an organic layer positioned between a first electrode and a second electrode. The organic layer may be configured to include one or more organic layers, and specifically, may be configured to include one or more layers selected from known organic layers that constitute a light-emitting member, such as a hole injection layer (HIL), a hole transport layer (HTL), an emission layer (EML), an electron transport layer (ETL), and an electron injection layer (EIL).

The hole injection layer (HIL) is a layer that injects holes from an electrode. As the hole injection material, a compound having the ability to transport holes, which has an excellent hole injection effect at the anode and an excellent hole injection effect for the light-emitting layer or light-emitting material, and which prevents the movement of excitons generated in the light-emitting layer to the electron injection layer or electron injection material, and which also has excellent thin-film forming ability is preferable.

The hole transport layer (HTL) is a layer that receives holes from the hole injection layer and transports them to the light-emitting layer. As the hole transport material, a material with high hole mobility is suitable, as it can transport holes from the anode or the hole injection layer and transfer them to the light-emitting layer.

The emission layer (EML) is a layer that emits light through the recombination of electrons and holes. The emission material can emit light in the visible spectrum by receiving holes and electrons from the hole transport layer and electron transport layer and recombining them. A material with high quantum efficiency for fluorescence or phosphorescence is preferred. The emission layer may specifically include a host and a dopant.

The electron transport layer (ETL) is a layer that receives electrons from the electron injection layer and transports them to the light-emitting layer. The electron transport material is a material that can easily receive electrons from the cathode and transfer them to the light-emitting layer, and a material with high electron mobility is preferable.

The electron injection layer (EIL) is a layer that injects electrons from an electrode, and a compound having the ability to transport electrons, an electron injection effect from a cathode, an excellent electron injection effect for a light-emitting layer or a light-emitting material, and preventing the movement of excitons generated in the light-emitting layer to the hole injection layer, and also having an excellent thin film forming ability is preferable.

Meanwhile, the organic layer may further include a hole-blocking layer (HBL). The HBL may be positioned between the emitting layer and the electron transport layer, and reduces the problem of holes from the hole injection layer invading the electron transport layer. The HBL is also one of the electron transport layers, and a material capable of transporting electrons is suitable.

Among the organic layers of such organic light-emitting devices, the novel compound according to the Chemical Formula 1 of the present invention is a material having excellent electron transport capability, and is therefore preferable to be applied to an electron transport layer or hole blocking layer in an electron transport region.

Meanwhile, the configuration of an organic light-emitting device can be varied or modified in various ways. According to one embodiment, a tandem organic light-emitting device may be formed by stacking two or more light-emitting members (or light-emitting units) each containing a light-emitting layer between a first electrode and a second electrode. In this tandem structure, in addition to the aforementioned organic layers, a charge generation layer (CGL) that regulates charge balance may be further included as one of the organic layers, and may be positioned at one or more locations between two adjacent light-emitting members. This charge generation layer may be composed of multiple layers, including an N-type charge generation layer that injects electrons and a P-type charge generation layer that injects holes, but is not limited thereto and may also be composed of a single layer.

The novel compound of the present invention can also be applied to the charge generation layer of such a tandem organic light-emitting device, and specifically, can be applied to the N-type charge generation layer. The N-type charge generation layer can be formed of an organic layer doped with a dopant such as a metal, and specifically, can be configured to include a host and a dopant.

At this time, the novel compound of the present invention can be applied as a host, and the compound of the present invention can smoothly interact with the dopant by including 1,10-phenanthroline having a lone pair of electrons. Meanwhile, the dopant may be a material containing a metal element, and specifically, may be at least one of a metal, a metal compound, and an organic metal complex. The metal element may be at least one selected from lithium (Li), sodium (Na), potassium (K), rubidium (Rb), cesium (Cs), beryllium (Be), magnesium (Mg), calcium (Ca), strontium (Sr), barium (Ba), ytterbium (Yb), samarium (Sm), tin (Sn), copper (Cu), titanium (Ti), cadmium (Cd), mercury (Hg), lead (Pb), bismuth (Bi), zinc (Zn), iron (Fe), cobalt (Co), nickel (Ni), indium (In), gallium (Ga), thorium (Th), uranium (U), silver (Ag), aluminum (Al), gold (Au), molybdenum (Mo), niobium (Nb), palladium (Pd), platinum (Pt), and europium (Eu), and may be lithium or ytterbium according to one embodiment. The ratio of these dopants may be doped at 0.1 to 20 wt% relative to the entire host material, specifically 0.5 to 15 wt%, and this is not limited and may vary depending on the type of metal element.

The present invention is described in more detail below, using synthetic examples, experimental examples, and practical examples. However, the following descriptions do not limit the scope of the present invention.

### Synthesis Example: Synthesis of a Compound

### Synthesis of Intermediate 1

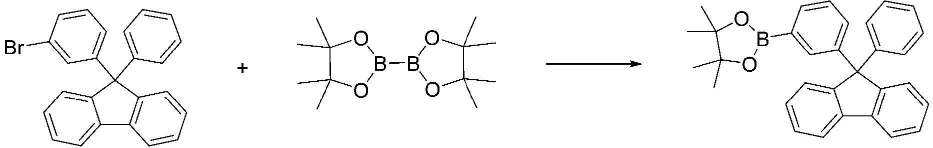

In a round-bottomed flask, 9-(3-bromophenyl)-9-phenyl-9H-fluorene (70 g, 176.75 mmol), bis(pinacolato)diboron (53.86 g, 212.09 mmol), sphos (4.35 g, 10.60 mmol), potassium acetate (34.70 g, 353.49 mmol), and [1,1'-Bis(diphenylphosphino)ferrocene]dichloropalladium(II) (3.88 g, 5.30 mmol) were added and refluxed in 600 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 1 (64.8 g, 82.53%).

### Synthetic Example 1. Synthesis of Compound 4

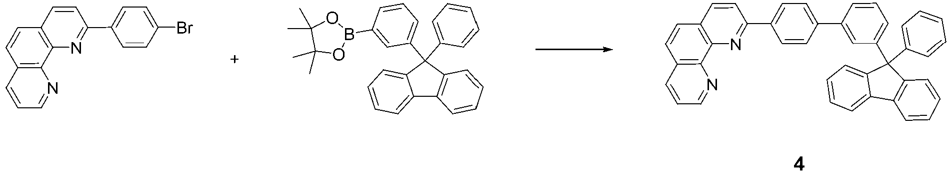

Intermediate 1 (10 g, 22.50 mmol), 2-(4-bromophenyl)-1,10-phenanthroline (9.05 g, 27.00 mmol), potassium carbonate (6.22 g, 45.01 mmol), and tetrakis(triphenylphophine)palladium (0.78 g, 0.68 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 4 (9.4 g, 72.94%).

### Synthesis of intermediate 2

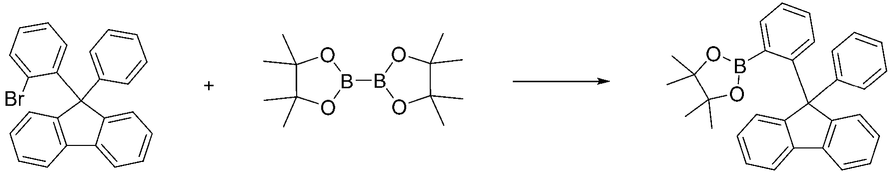

In a round-bottomed flask, 9-(2-bromophenyl)-9-phenyl-9H-fluorene (15 g, 37.87 mmol), bis(pinacolato)diboron (11.54 g, 45.45 mmol), sphos (0.93 g, 2.27 mmol), potassium acetate (7.43 g, 75.75 mmol), and [1,1'-Bis(diphenylphosphino)ferrocene]dichloropalladium(II) (0.93 g, 2.27 mmol) were added and stirred overnight under reflux in 150 mL of 1,4-dioxane. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 2 (12.5 g, 74.29%).

### Synthesis Example 2. Synthesis of Compound 5

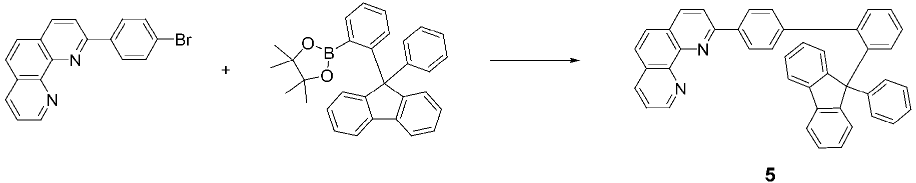

Intermediate 2 (12.5 g, 26.11 mmol), 2-(4-bromophenyl)-1,10-phenanthroline (10.50 g, 31.34 mmol), potassium carbonate (7.22 g, 52.23 mmol), and tetrakis(triphenylphophine)palladium (0.91 g, 0.78 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 5 (11.4 g, 76.23%).

### Synthesis of intermediate 3

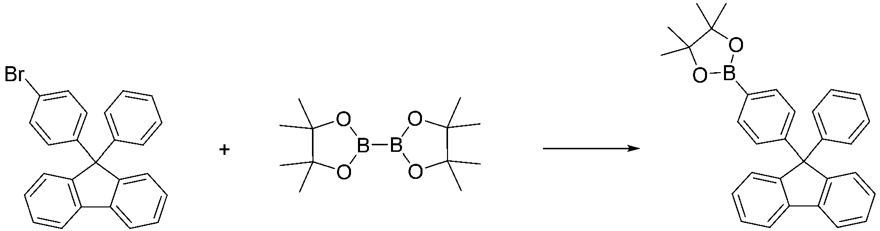

In a round-bottomed flask, 9-(4-bromophenyl)-9-phenyl-9H-fluorene (30 g, 75.51 mmol), bis(pinacolato)diboron (23.01 g, 90.61 mmol), sphos (1.86 g, 4.53 mmol), potassium acetate (14.82 g, 151.01 mmol), and [1,1'-Bis(diphenylphosphino)ferrocene]dichloropalladium(II) (1.66 g, 2.27 mmol) were added and refluxed in 300 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 3 (31.2 g, 92.72%).

### Synthesis Example 3. Synthesis of Compound 6

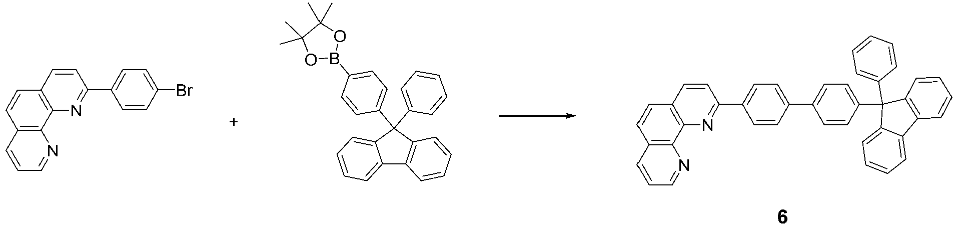

Intermediate 3 (10 g, 22.50 mmol), 2-(4-bromophenyl)-1,10-phenanthroline (9.05 g, 27.00 mmol), potassium carbonate (6.22 g, 45.01 mmol), and tetrakis(triphenylphophine)palladium (0.78 g, 0.68 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered through silica gel and Celite under reduced pressure to obtain a filtrate. The filtrate was concentrated under reduced pressure, dissolved in chloroform, and recrystallized with ethanol to obtain compound 6 (9.4 g, 72.91%).

### Synthesis Example 4. Synthesis of Compound 7

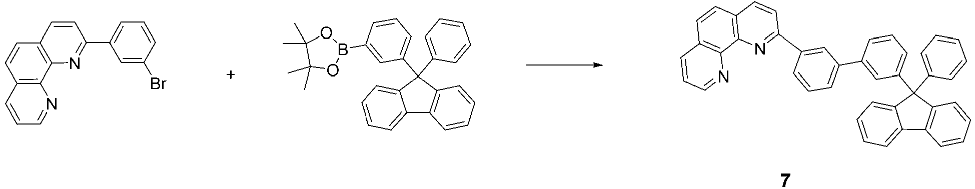

Intermediate 1 (10 g, 22.50 mmol), 2-(3-bromophenyl)-1,10-phenanthroline (9.05 g, 27.00 mmol), potassium carbonate (6.22 g, 45.01 mmol), and tetrakis(triphenylphophine)palladium (0.78 g, 0.68 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 7 (8.9 g, 69.03%).

### Synthesis Example 5. Synthesis of Compound 10

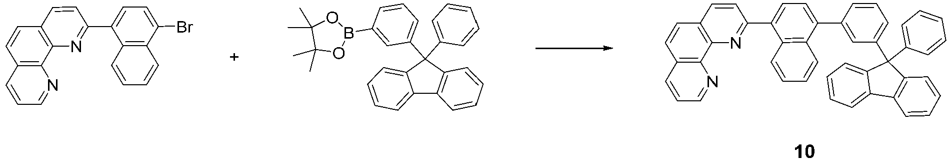

Intermediate 1 (10 g, 22.50 mmol), 2-(4-bromonaphthalen-1-yl)-1,10-phenanthroline (10.40 g, 27.00 mmol), potassium carbonate (6.22 g, 45.01 mmol), and tetrakis(triphenylphophine)palladium (0.78 g, 0.68 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 10 (11.6 g, 82.74%).

### Synthesis Example 6. Synthesis of Compound 13

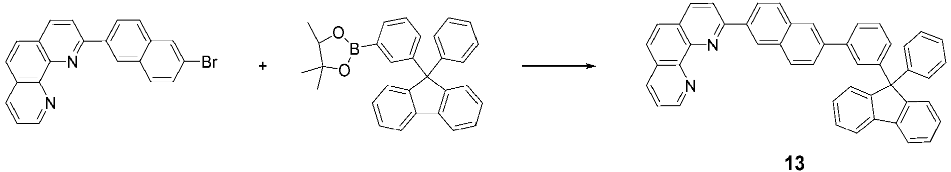

Intermediate 1 (10 g, 22.50 mmol), 2-(6-bromonaphthalen-2-yl)-1,10-phenanthroline (10.40 g, 27.00 mmol), potassium carbonate (6.22 g, 45.01 mmol), and tetrakis(triphenylphophine)palladium (0.78 g, 0.68 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 13 (10.2 g, 72.76%).

### Synthesis of intermediate 4

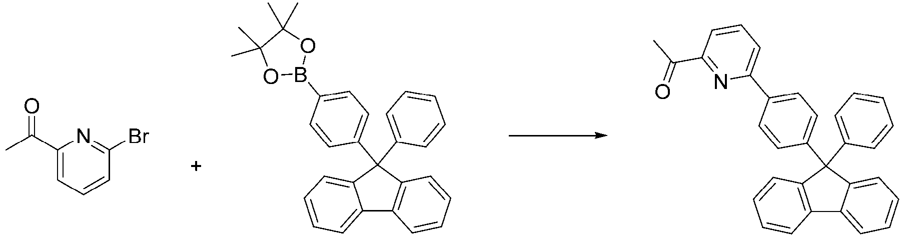

Intermediate 3 (15 g, 33.75 mmol), 1-(6-bromopyridin-2-yl)ethan-1-one (8.10 g, 40.51 mmol), potassium carbonate (9.33 g, 67.51 mmol), and tetrakis(triphenylphophine)palladium (1.17 g, 1.01 mmol) were added to a round-bottomed flask and stirred overnight under reflux in 125 mL of 1,4-dioxane and 60 mL of water. After the reaction was complete, the mixture was cooled to room temperature and concentrated under reduced pressure to obtain a filtrate. The obtained filtrate was extracted with ethyl acetate and water. The organic layer was dried over MgSO4 and concentrated under reduced pressure to obtain a filtrate. The obtained filtrate was purified by column chromatography to obtain intermediate 4 (10.4 g, 70.39%).

### Synthesis Example 7. Synthesis of Compound 18

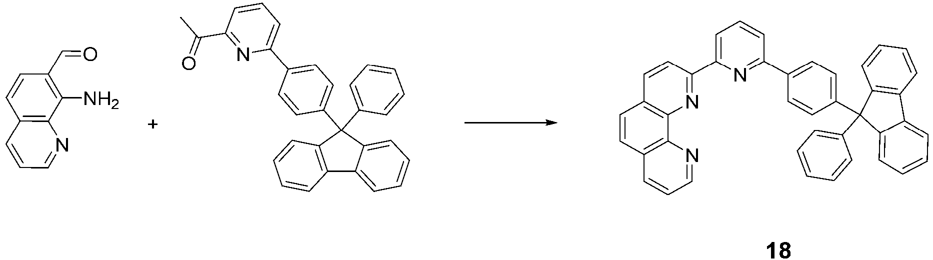

8-aminoquinoline-7-carbaldehyde (4.91 g, 28.52 mmol), intermediate 4 (10.4 g, 23.77 mmol), and 70 mL of toluene were added to a round-bottomed flask. Potassium hydroxide (2.56 g, 45.71 mmol) dissolved in 50 mL of ethanol was added dropwise, and the mixture was stirred under reflux overnight. After the reaction was complete, the mixture was cooled to room temperature, methanol was added, filtered, and the solid was washed with methionine. The solid was dissolved in toluene, and the filtrate was filtered through silica gel and Celite under reduced pressure to obtain a filtrate. The filtrate was concentrated under reduced pressure and recrystallized from toluene to obtain compound 18 (8.5 g, 62.33%).

### Synthesis of intermediate 5

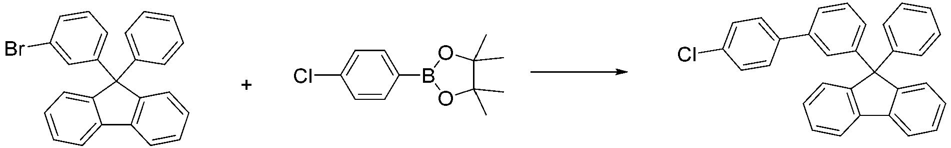

9-(3-bromophenyl)-9-phenyl-9H-fluorene (15 g, 37.87 mmol), 2-(4-chlorophenyl)-4,4,5,5-tetramethyl-1,3,2-dioxaborolane (9.92 g, 41.66 mmol), potassium carbonate (10.47 g, 75.75 mmol), and tetrakis(triphenylphophine)palladium (1.32 g, 1.14 mmol) were added to a round-bottomed flask and stirred overnight under reflux in 125 mL of 1,4-dioxane and 60 mL of water. After the reaction was complete, the mixture was cooled to room temperature and concentrated under reduced pressure to obtain a filtrate. The obtained filtrate was extracted with ethyl acetate and water. The organic layer was dried over MgSO4 and concentrated under reduced pressure to obtain a filtrate. The obtained filtrate was purified by column chromatography to obtain intermediate 5 (12.6 g, 77.56%).

### Synthesis of intermediate 6

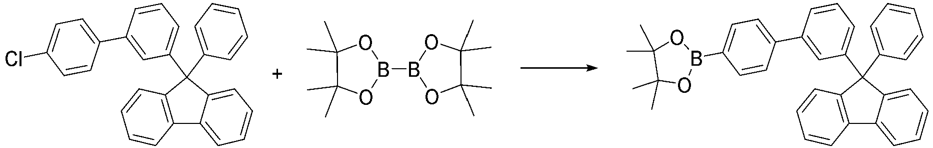

A round-bottomed flask was charged with 9-(4'-chloro-[1,1'-biphenyl]-3-yl)-9-phenyl-9H-fluorene (12.6 g, 29.37 mmol), bis(pinacolato)diboron (8.95 g, 35.25 mmol), sphos (0.72 g, 1.76 mmol), potassium acetate (5.77 g, 58.75 mmol), and [1,1'-Bis(diphenylphosphino)ferrocene]dichloropalladium(II) (0.64 g, 0.88 mmol), and the mixture was stirred overnight under reflux in 150 mL of 1,4-dioxane. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 6 (12.8 g, 83.72%).

### Synthesis Example 8. Synthesis of Compound 22

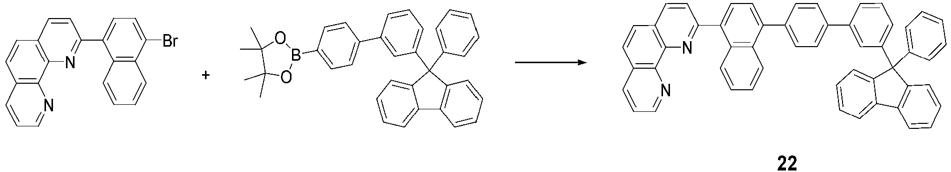

Intermediate 6 (12.8 g, 24.59 mmol), 2-(4-bromonaphthalen-1-yl)-1,10-phenanthroline (10.42 g, 27.05 mmol), potassium carbonate (6.80 g, 49.19 mmol), and tetrakis(triphenylphophine)palladium (0.85 g, 0.74 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered through silica gel and Celite under reduced pressure to obtain a filtrate. The obtained filtrate was concentrated under reduced pressure, dissolved in chloroform, and recrystallized with ethanol to obtain compound 22 (11.8 g, 68.66%).

### Synthesis of intermediate 7

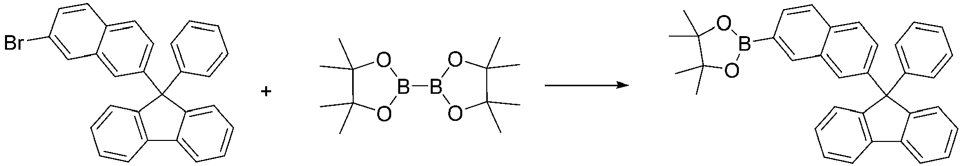

In a round-bottomed flask, 9-(7-bromonaphthalen-2-yl)-9-phenyl-9H-fluorene (40 g, 89.41 mmol), bis(pinacolato)diboron (27.25 g, 107.29 mmol), sphos (2.20 g, 5.36 mmol), potassium acetate (17.55 g, 178.82 mmol), and [1,1'-Bis(diphenylphosphino)ferrocene]dichloropalladium(II) (1.96 g, 2.68 mmol) were added and stirred overnight under reflux in 300 mL of 1,4-dioxane. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 7 (41.2 g, 93.20%).

### Synthetic Example 9. Synthesis of Compound 28

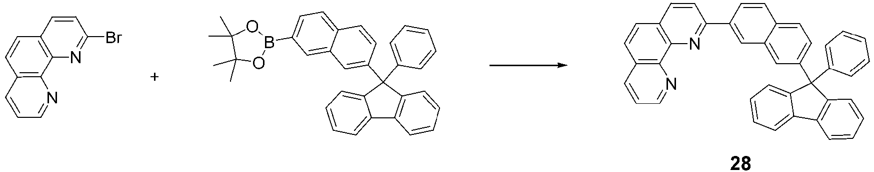

Intermediate 7 (10 g, 20.22 mmol), 2-bromo-1,10-phenanthroline (5.76 g, 22.25 mmol), potassium carbonate (5.60 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered through silica gel and Celite under reduced pressure to obtain a filtrate. The filtrate was concentrated under reduced pressure, dissolved in chloroform, and recrystallized with ethanol to obtain compound 28 (8.5 g, 76.88%).

### Synthesis of intermediate 8

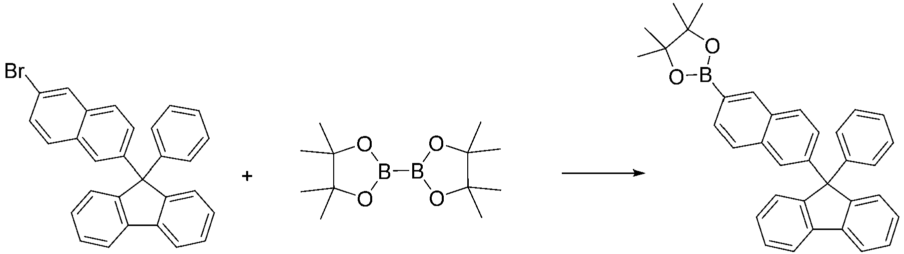

In a round-bottomed flask, 9-(6-bromonaphthalen-2-yl)-9-phenyl-9H-fluorene (30 g, 67.06 mmol), bis(pinacolato)diboron (20.43 g, 80.47 mmol), sphos (1.65 g, 4.02 mmol), potassium acetate (13.16 g, 134.11 mmol), and [1,1'-Bis(diphenylphosphino)ferrocene]dichloropalladium(II) (1.47 g, 2.01 mmol) were added and refluxed in 300 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 8 (30.9 g, 93.2%).

### Synthetic Example 10. Synthesis of Compound 29

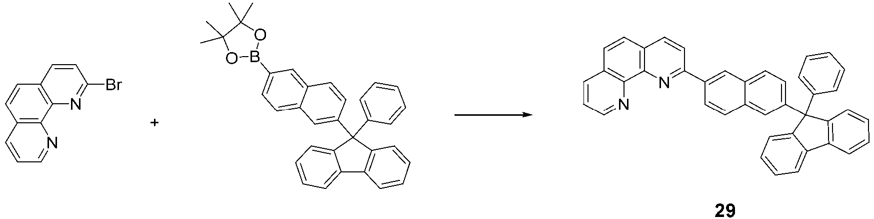

Intermediate 8 (10 g, 20.22 mmol), 2-bromo-1,10-phenanthroline (5.76 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 29 (7.9 g, 71.45%).

### Synthesis of intermediate 9

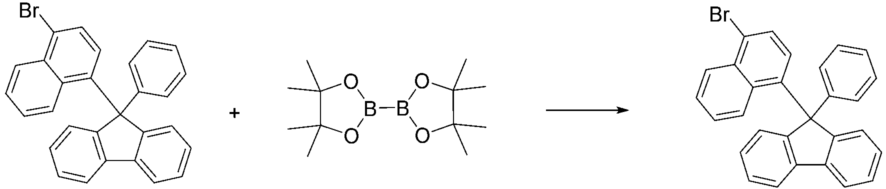

In a round-bottomed flask, 9-(4-bromonaphthalen-1-yl)-9-phenyl-9H-fluorene (15 g, 33.53 mmol), bis(pinacolato)diboron (10.22 g, 40.23 mmol), sphos (0.83 g, 2.01 mmol), potassium acetate (6.58 g, 67.06 mmol), and [1,1'-Bis(diphenylphosphino)ferrocene]dichloropalladium(II) (0.74 g, 1.01 mmol) were added and refluxed in 300 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 9 (12.7 g, 76.61%).

### Synthesis Example 11. Synthesis of Compound 30

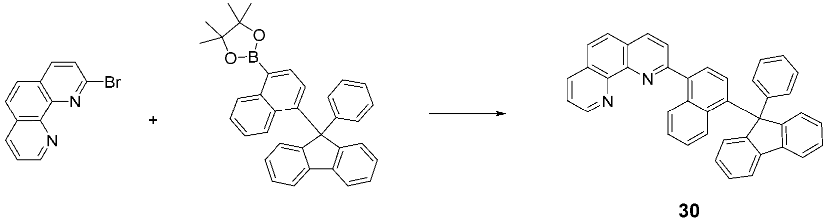

Intermediate 9 (12.7 g, 25.69 mmol), 2-bromo-1,10-phenanthroline (7.32 g, 28.25 mmol), potassium carbonate (7.10 g, 51.37 mmol), and tetrakis(triphenylphophine)palladium (0.89 g, 0.77 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered through silica gel and Celite under reduced pressure to obtain a filtrate. The filtrate was concentrated under reduced pressure, dissolved in chloroform, and recrystallized with ethanol to obtain compound 30 (9.2 g, 65.52%).

### Synthesis Example 12. Synthesis of Compound 32

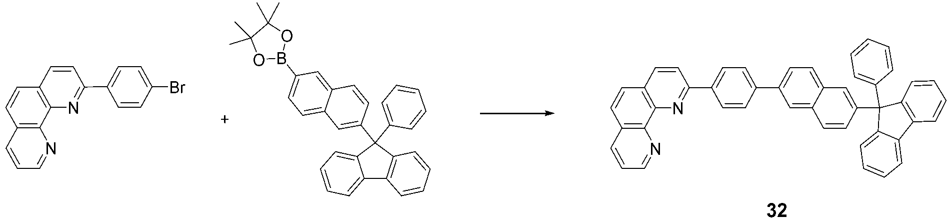

Intermediate 8 (10 g, 20.22 mmol), 2-(4-bromophenyl)-1,10-phenanthroline (7.46 g, 22.25 mmol), potassium carbonate (5.59 g, 44.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 32 (8.7 g, 69.07%).

### Synthetic Example 13. Synthesis of Compound 34

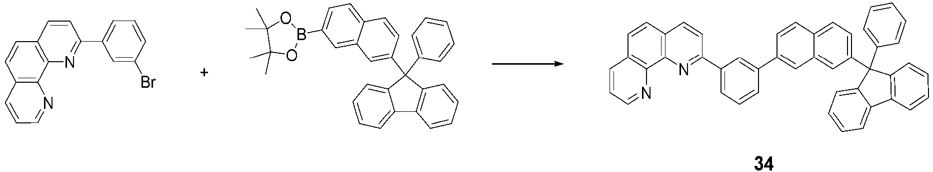

Intermediate 7 (10 g, 20.22 mmol), 2-(3-bromophenyl)-1,10-phenanthroline (7.46 g, 22.25 mmol), potassium carbonate (5.59 g, 44.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 34 (9.2 g, 73.04%).

### Synthetic Example 14. Synthesis of Compound 37

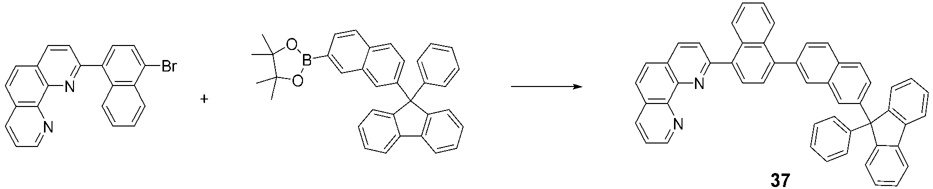

Intermediate 7 (10 g, 20.22 mmol), 2-(4-bromonaphthalen-1-yl)-1,10-phenanthroline (8.57 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 37 (10.3 g, 75.69%).

### Synthesis of intermediate 10

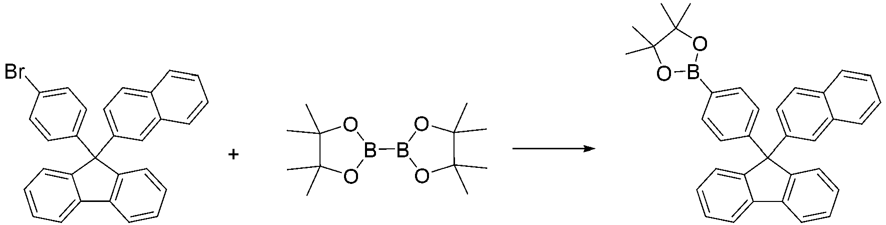

In a round-bottomed flask, 9-(4-bromophenyl)-9-(naphthalen-2-yl)-9H-fluorene (10 g, 22.35 mmol), bis (pinacolato) diboron (6.81 g, 26.82 mmol), sphos (0.55 g, 1.34 mmol), potassium acetate (4.39 g, 44.70 mmol), and [1,1'-Bis(diphenylphosphino)ferrocene]dichloropalladium(II) (0.49 g, 0.67 mmol) were added and refluxed in 120 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 10 (9.8 g, 88.67%).

### Synthetic Example 15. Synthesis of Compound 66

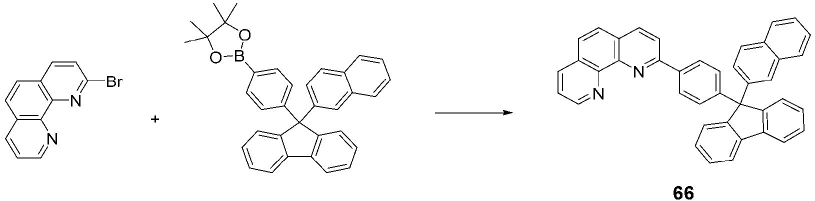

Intermediate 10 (9.8 g, 19.82 mmol), 2-bromo-1,10-phenanthroline (5.65 g, 21.80 mmol), potassium carbonate (5.47 g, 39.64 mmol), and tetrakis(triphenylphophine)palladium (0.69 g, 0.59 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 66 (8.6 g, 79.37%).

### Synthesis of intermediate 11

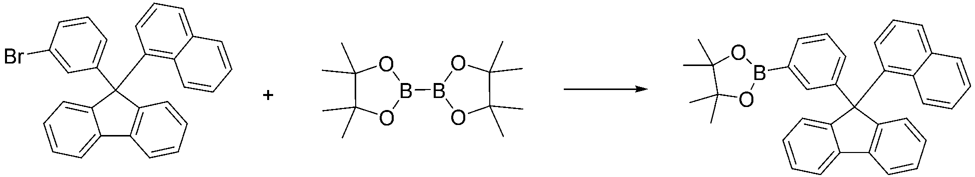

In a round-bottomed flask, 9-(4-bromophenyl)-9-(naphthalen-1-yl)-9H-fluorene (10 g, 22.35 mmol), bis (pinacolato) diboron (6.81 g, 26.82 mmol), sphos (0.55 g, 1.34 mmol), potassium acetate (4.39 g, 44.70 mmol), and [1,1'-Bis(diphenylphosphino)ferrocene]dichloropalladium(II) (0.49 g, 0.67 mmol) were added and stirred overnight under reflux in 120 mL of 1,4-dioxane. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 11 (9.7 g, 87.77%).

### Synthesis Example 16. Synthesis of Compound 70

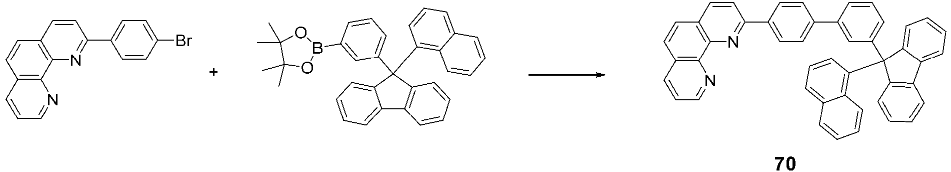

Intermediate 11 (9.7 g, 19.62 mmol), 2-(4-bromophenyl)-1,10-phenanthroline (7.23 g, 21.58 mmol), potassium carbonate (5.42 g, 39.24 mmol), and tetrakis(triphenylphophine)palladium (0.68 g, 0.59 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 70 (9.1 g, 74.48%).

### Synthesis of intermediate 12

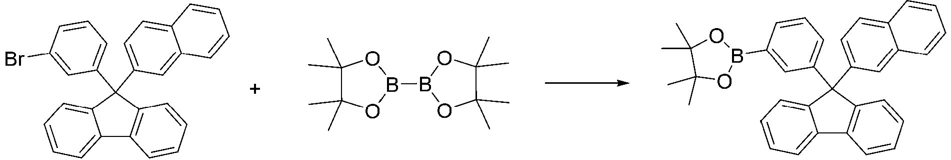

In a round-bottomed flask, 9-(3-bromophenyl)-9-(naphthalen-2-yl)-9H-fluorene (35 g, 78.23 mmol), bis (pinacolato) diboron (23.84 g, 93.88 mmol), sphos (1.92 g, 4.69 mmol), potassium acetate (15.35 g, 156.47 mmol), and [1,1'-Bis(diphenylphosphino)ferrocene]dichloropalladium(II) (1.71 g, 2.35 mmol) were added and refluxed in 300 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 12 (32.4 g, 83.76%).

### Synthetic Example 17. Synthesis of Compound 71

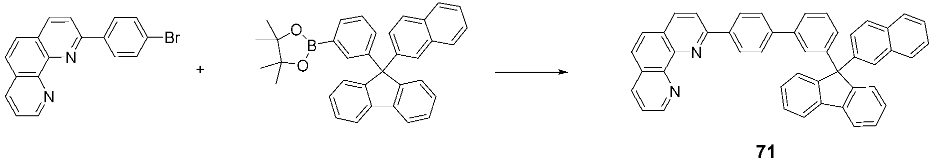

Intermediate 12 (10 g, 20.22 mmol), 2-(4-bromophenyl)-1,10-phenanthroline (7.46 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 71 (8.8 g, 69.87%).

### Synthesis of intermediate 13

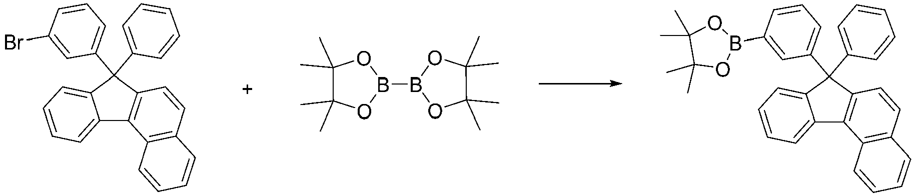

In a round-bottomed flask, 7-(3-bromophenyl)-7-phenyl-7H-benzo [c] fluorene (35 g, 78.23 mmol), bis (pinacolato) diboron (23.84 g, 93.88 mmol), sphos (1.92 g, 4.69 mmol), potassium acetate (15.35 g, 156.47 mmol), and [1,1'-Bis(diphenylphosphino)ferrocene]dichloropalladium(II) (1.71 g, 2.35 mmol) were added and refluxed in 300 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 13 (31.0 g, 80.14%).

### Synthesis Example 18. Synthesis of Compound 72

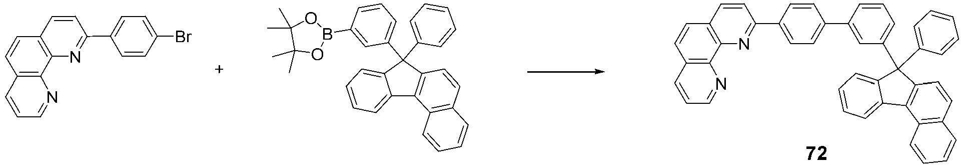

Intermediate 13 (10 g, 20.22 mmol), 2-(4-bromophenyl)-1,10-phenanthroline (7.46 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 72 (8.6 g, 68.28%).

### Synthesis of intermediate 14

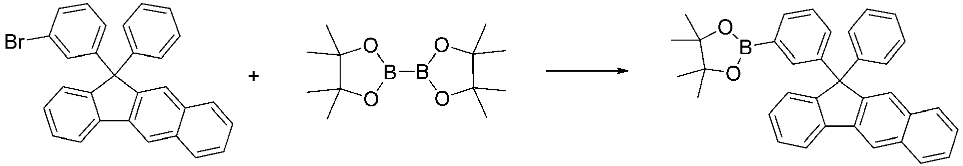

A round-bottomed flask was charged with 11-(3-bromophenyl)-11-phenyl-11H-benzo[b]fluorene (25 g, 55.88 mmol), bis (pinacolato) diboron (17.03 g, 67.06 mmol), sphos (1.38 g, 3.35 mmol), potassium acetate (10.97 g, 111.76 mmol), and [1,1'-Bis(diphenylphosphino)ferrocene]dichloropalladium(II) (1.23 g, 1.68 mmol), and the mixture was stirred overnight under reflux in 300 mL of 1,4-dioxane. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 14 (21.1 g, 76.37%).

### Synthetic Example 19. Synthesis of Compound 73

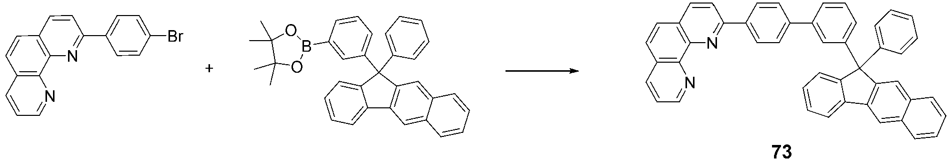

Intermediate 14 (10 g, 20.22 mmol), 2-(4-bromophenyl)-1,10-phenanthroline (7.46 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 73 (7.9 g, 62.72%).

### Synthesis of intermediate 15

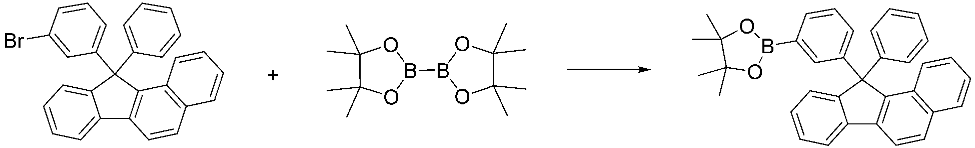

In a round-bottomed flask, 11-(3-bromophenyl)-11-phenyl-11H-benzo[a]fluorene (10 g, 22.35 mmol), bis(pinacolato)diboron (6.81 g, 26.82 mmol), sphos (0.55 g, 1.34 mmol), potassium acetate (4.39 g, 44.70 mmol), and [1,1'-Bis(diphenylphosphino)ferrocene]dichloropalladium(II) (0.49 g, 0.67 mmol) were added and stirred overnight under reflux in 300 mL of 1,4-dioxane overnight. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 15 (8.7 g, 78.72%).

### Synthesis Example 20. Synthesis of Compound 74

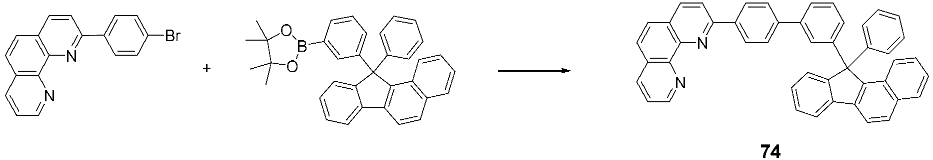

Intermediate 15 (8.7 g, 17.60 mmol), 2-(4-bromophenyl)-1,10-phenanthroline (6.49 g, 19.36 mmol), potassium carbonate (4.86 g, 35.19 mmol), and tetrakis(triphenylphophine)palladium (0.61 g, 0.53 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and then filtered through silica gel and Celite under reduced pressure to obtain a filtrate. The obtained filtrate was concentrated under reduced pressure, dissolved in chloroform, and recrystallized with ethanol to obtain compound 74 (7.9 g, 72.09%).

### Synthesis of intermediate 16

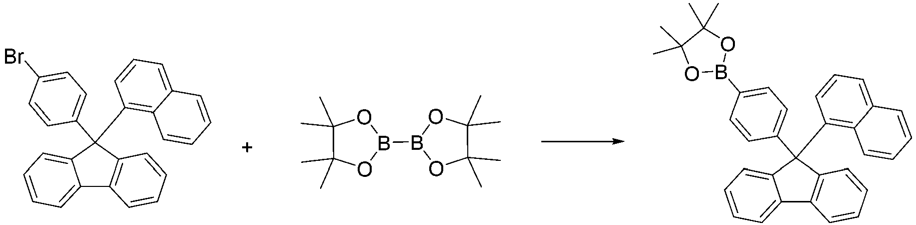

In a round-bottomed flask, 9-(4-bromophenyl)-9-(naphthalen-1-yl)-9H-fluorene (10 g, 22.35 mmol), bis(pinacolato)diboron (6.81 g, 26.82 mmol), sphos (0.55 g, 1.34 mmol), potassium acetate (4.39 g, 44.70 mmol), and [1,1'-Bis(diphenylphosphino)ferrocene]dichloropalladium(II) (0.49 g, 0.67 mmol) were added and stirred overnight under reflux in 300 mL of 1,4-dioxane. After the reaction was complete, the mixture was cooled to room temperature and concentrated under reduced pressure to obtain a filtrate. The obtained filtrate was extracted with dichloromethane and water. The organic layer was dried over MgSO4 and concentrated under reduced pressure to obtain a filtrate. The obtained filtrate was dissolved in dichloromethane, filtered under reduced pressure through silica gel and celite, and concentrated under reduced pressure to obtain intermediate 16 (8.1 g, 73.29%).

### Synthesis Example 21. Synthesis of Compound 80

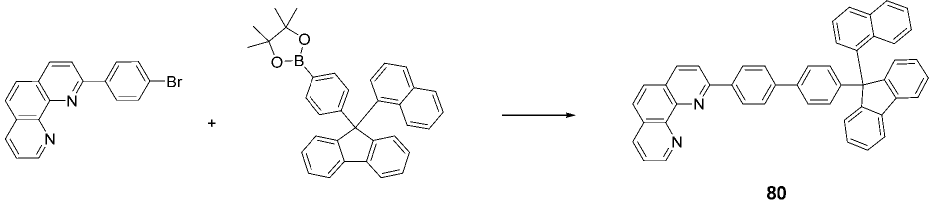

Intermediate 16 (8.1 g, 16.38 mmol), 2-(4-bromophenyl)-1,10-phenanthroline (6.04 g, 18.02 mmol), potassium carbonate (4.53 g, 32.76 mmol), and tetrakis(triphenylphophine)palladium (0.57 g, 0.49 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 80 (8.2 g, 80.37%).

### Synthetic Example 22. Synthesis of Compound 86

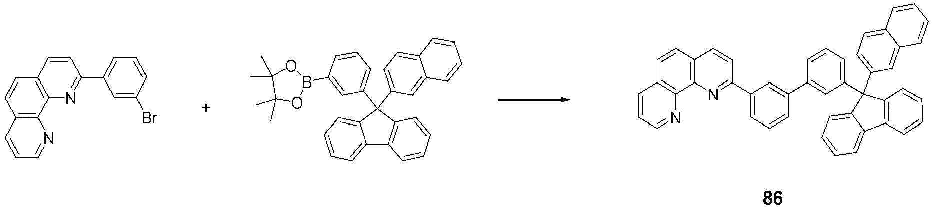

Intermediate 12 (10 g, 20.22 mmol), 2-(3-bromophenyl)-1,10-phenanthroline (7.46 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 86 (8.1 g, 64.31%).

### Synthesis Example 23. Synthesis of Compound 88

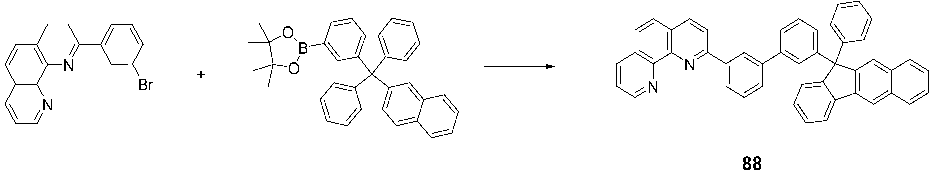

Intermediate 14 (10 g, 20.22 mmol), 2-(3-bromophenyl)-1,10-phenanthroline (7.46 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 88 (9.0 g, 71.45%).

### Synthesis Example 24. Synthesis of Compound 89

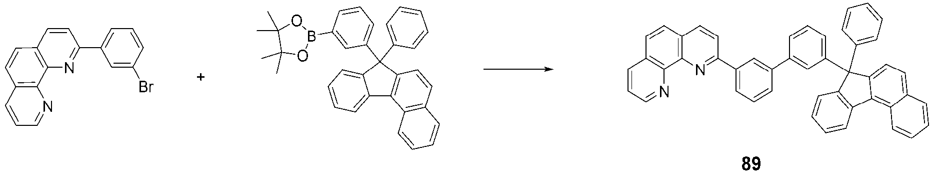

Intermediate 13 (10 g, 20.22 mmol), 2-(3-bromophenyl)-1,10-phenanthroline (7.46 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 89 (7.8 g, 61.93%).

### Synthesis Example 25. Synthesis of Compound 101

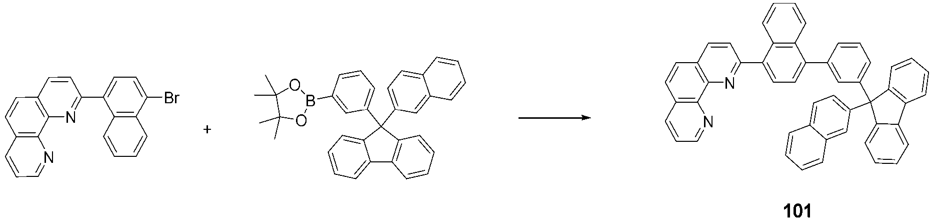

Intermediate 12 (10 g, 20.22 mmol), 2-(4-bromonaphthalen-1-yl)-1,10-phenanthroline (8.57 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 101 (9.4 g, 69.13%).

### Synthetic Example 26. Synthesis of Compound 226

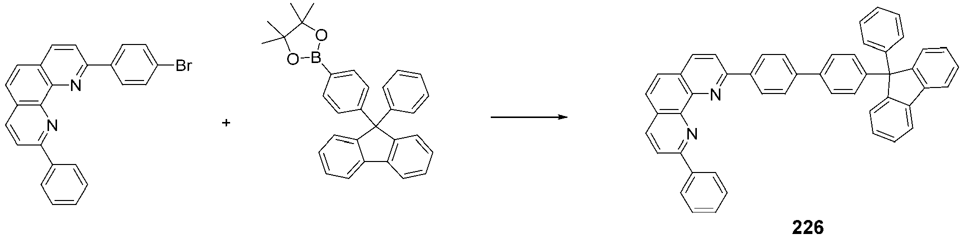

Intermediate 3 (10 g, 22.51 mmol), 2-(4-bromophenyl)-9-phenyl-1,10-phenanthroline (10.18 g, 24.76 mmol), potassium carbonate (6.22 g, 45.01 mmol), and tetrakis (triphenylphophine)palladium (0.78 g, 0.68 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 226 (9.8 g, 67.1%).

### Synthetic Example 27. Synthesis of Compound 227

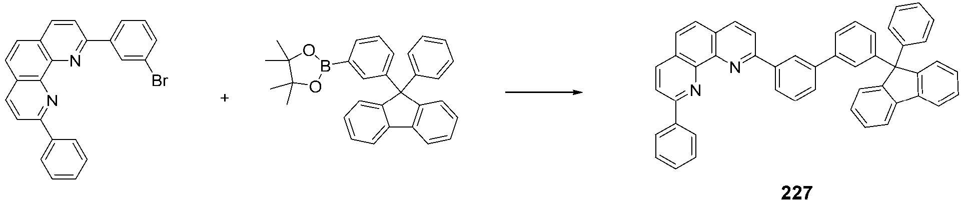

Intermediate 1 (10 g, 22.51 mmol), 2-(3-bromophenyl)-1,10-phenanthroline (10.18 g, 24.76 mmol), potassium carbonate (6.22 g, 45.01 mmol), and tetrakis(triphenylphophine)palladium (0.78 g, 0.68 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The obtained filtrate was concentrated under reduced pressure, dissolved in chloroform, and recrystallized with ethanol to obtain compound 227 (10.1 g, 69.15%).

### Synthetic Example 28. Synthesis of Compound 230

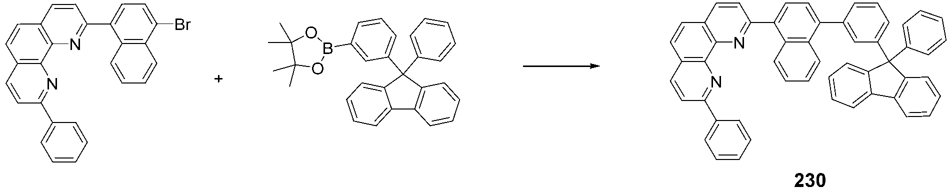

Intermediate 1 (10 g, 21.68 mmol), 2-(4-bromonaphthalen-1-yl)-9-phenyl-1,10-phenanthroline (9.81 g, 23.84 mmol), potassium carbonate (5.99 g, 43.35 mmol), and tetrakis(triphenylphophine)palladium (0.75 g, 0.65 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 230 (9.5 g, 62.71%).

### Synthesis Example 29. Synthesis of Compound 250

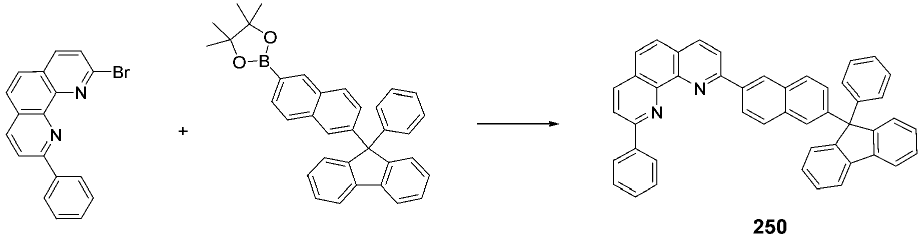

Intermediate 8 (10 g, 20.22 mmol), 2-bromo-9-phenyl-1,10-phenanthroline (7.46 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 250 (9.2 g, 73.04%).

### Synthesis Example 30. Synthesis of Compound 252

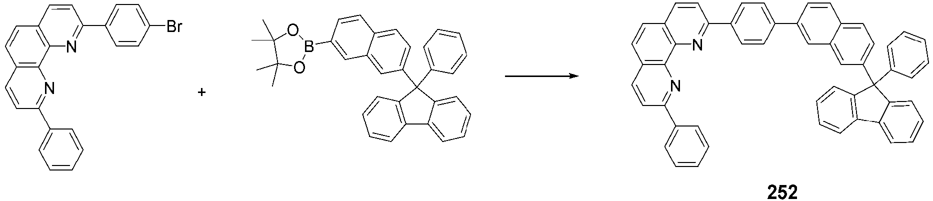

Intermediate 7 (10 g, 20.22 mmol), 2-(4-bromophenyl)-9-phenyl-1,10-phenanthroline (9.15 g, 22.25 mmol), potassium carbonate (5.59 g, 40.45 mmol), and tetrakis(triphenylphophine)palladium (0.70 g, 0.61 mmol) were added to a round-bottomed flask and stirred overnight under reflux in 125 mL of 1,4-dioxane and 60 mL of water. After the reaction was complete, the mixture was cooled to room temperature, filtered, and the solid was washed with water and ethanol. The solid was dissolved in chloroform, and the filtrate was filtered under reduced pressure through silica gel and Celite to obtain a filtrate. The filtrate was concentrated under reduced pressure, dissolved in chloroform, and recrystallized with ethanol to obtain compound 252 (10.6 g, 74.99%).

### Experimental Example: Fabrication and Evaluation of Tandem Organic Light-Emitting Devices

### Comparative Example 1

An ITO substrate was patterned to form an anode with a light-emitting area of 2 mm X 2 mm, and then cleaned with isopropyl alcohol and UV ozone, respectively. Afterwards, the ITO substrate was mounted on a holder of a vacuum deposition device and pressure was applied so that the vacuum level became 1 X 10-7 torr. Afterwards, plasma treatment was performed for 3 minutes under a N2 atmosphere. Afterwards, a HAT-CN compound was vacuum-deposited on the substrate to a thickness of 5 nm to form a first hole injection layer, and a NPB material was vacuum-deposited thereon to a thickness of 20 nm to form a first hole transport layer. Afterwards, a GH-1 material as a hose and a GD-1 material as a dopant were co-deposited to a mass ratio of about 10% to form a 20 nm thick green first light-emitting layer.

Thereon, a TmPyPB material was vacuum-deposited to a thickness of 20 nm to form a first electron transport layer. Thereafter, a BPhen material as a host and a Li material as a dopant were co-deposited on the first electron transport layer at a mass ratio of approximately 2% to form a 10 nm thick N-type charge generation layer. The HAT-CN material was vacuum-deposited on top of it to form a 5 nm thick P-type charge generation layer. This P-type charge generation layer is also used as a second hole injection layer. Thereafter, the NPB material was vacuum-deposited to a thickness of 50 nm to form a second hole transport layer. Thereon, the GH-1 material as a host and the GD-1 material as a dopant were co-deposited at a mass ratio of approximately 10% to form a 20 nm thick green second emitting layer. Thereafter, the TmPyPB material as a host and the Liq material as a dopant were co-deposited at a mass ratio of approximately 33% to form a 20 nm thick second electron transport layer. The LiF material was vacuum-deposited thereon to a thickness of 1 nm to form an electron injection layer. After that, Al was deposited to a thickness of 50 nm to form a cathode, thereby completing the fabrication of a tandem-type organic light-emitting device. The structures of HAT-CN, NPB, GH-1, GD-1, TmPyPB, BPhen, and Liq materials used in the fabrication of organic light-emitting devices are shown in Table 1 below, respectively.

**[Table 1]**

| | | |
|---|---|---|
| 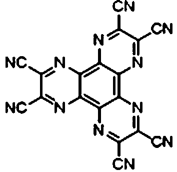 | 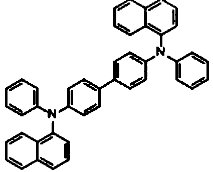 | |
| HAT-CN | NPB | GH-1 |
| 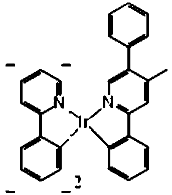 | 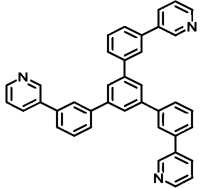 | 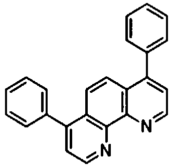 |
| GD-1 | TmPyPB | BPhen |
| | | |
| Liq | | |

### Comparative Examples 2 to 10

An organic light-emitting device was manufactured in the same manner as in Comparative Example 1, except that each of Comparative Compounds 1 to 9 in Table 2 below was used instead of BPhen as a host for the N-type charge generation layer.

**[Table 2]**

| | | |
|---|---|---|
| | | |
| Comparative Compound 1 | Comparative Compound2 | Comparative Compound 3 |
| | | |
| Comparative Compound 4 | Comparative Compound 5 | Comparative Compound 6 |
| 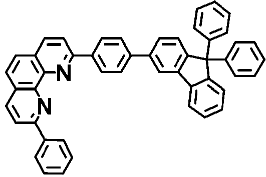 | 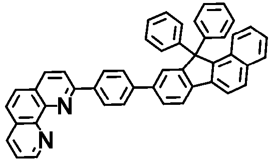 | 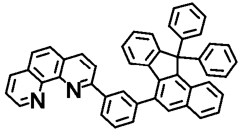 |
| Comparative Compound 7 | Comparative Compound 8 | Comparative Compound 9 |

### Example 1 to Example 30

Organic light-emitting devices were fabricated in the same manner as in Comparative Example 1, except that the previously synthesized compounds 1 through 30 were used instead of BPhen as the host for the N-type charge generation layer.

The operating voltage, current efficiency, and Lifespan of the previously fabricated organic light-emitting devices were evaluated. The evaluation results are shown in Table 3.

**[Table 3]**

| | N-type charge generation layer Host material | Operating current density J(mA/cm^2) | Operating voltage (Voltage) | Current efficiency (cd/A) | Lifespan (LT95) |
|---|---|---|---|---|---|
| Comparative Example 1 | BPhen | 10 | 6.74 | 116.85 | 179.50 |
| Comparative Example 2 | Comparative Compound 1 | 10 | 6.71 | 117.72 | 181.48 |
| Comparative Example 3 | Comparative Compound 2 | 10 | 6.73 | 117.20 | 180.90 |
| Comparative Example 4 | Comparative Compound 3 | 10 | 6.66 | 118.48 | 183.32 |
| Comparative Example 5 | Comparative Compound 4 | 10 | 6.69 | 118.89 | 184.53 |
| Comparative Example 6 | Comparative Compound 5 | 10 | 6.64 | 118.64 | 183.35 |
| Comparative Example 7 | Comparative Compound 6 | 10 | 6.67 | 119.34 | 182.77 |
| Comparative Example 8 | Comparative Compound 7 | 10 | 6.63 | 118.83 | 184.39 |
| Comparative Example 9 | Comparative Compound 8 | 10 | 6.60 | 119.06 | 184.24 |
| Comparative Example 10 | Comparative Compound 9 | 10 | 6.61 | 119.75 | 183.65 |
| Example 1 | Compound 4 | 10 | 6.58 | 121.74 | 196.17 |
| Example 2 | Compound 5 | 10 | 6.57 | 121.77 | 195.48 |
| Example 3 | Compound 6 | 10 | 6.54 | 122.68 | 196.40 |
| Example 4 | Compound 7 | 10 | 6.49 | 121.94 | 195.74 |
| Example 5 | Compound 10 | 10 | 6.55 | 121.71 | 197.01 |
| Example 6 | Compound 13 | 10 | 6.48 | 122.92 | 196.98 |
| Example 7 | Compound 18 | 10 | 6.52 | 121.90 | 197.84 |
| Example 8 | Compound 22 | 10 | 6.56 | 121.65 | 197.66 |
| Example 9 | Compound 28 | 10 | 6.23 | 125.14 | 211.87 |
| Example 10 | Compound 29 | 10 | 6.21 | 126.08 | 212.49 |
| Example 11 | Compound 30 | 10 | 6.24 | 125.52 | 211.03 |
| Example 12 | Compound 32 | 10 | 6.17 | 126.06 | 212.15 |
| Example 13 | Compound 34 | 10 | 6.19 | 126.18 | 212.59 |
| Example 14 | Compound 37 | 10 | 6.20 | 125.85 | 211.93 |
| Example 15 | Compound 66 | 10 | 6.28 | 125.04 | 210.87 |
| Example 16 | Compound 70 | 10 | 6.31 | 124.87 | 209.48 |
| Example 17 | Compound 71 | 10 | 6.33 | 125.15 | 209.25 |
| Example 18 | Compound 72 | 10 | 6.34 | 124.28 | 205.37 |
| Example 19 | Compound 73 | 10 | 6.38 | 123.91 | 206.16 |
| Example 20 | Compound 74 | 10 | 6.36 | 124.35 | 205.29 |
| Example 21 | Compound 80 | 10 | 6.29 | 125.01 | 208.96 |
| Example 22 | Compound 86 | 10 | 6.34 | 124.76 | 209.04 |
| Example 23 | Compound 88 | 10 | 6.37 | 123.91 | 204.83 |
| Example 24 | Compound 89 | 10 | 6.35 | 123.77 | 203.41 |
| Example 25 | Compound 101 | 10 | 6.30 | 124.58 | 208.83 |
| Example 26 | Compound 226 | 10 | 6.41 | 122.71 | 200.14 |
| Example 27 | Compound 227 | 10 | 6.42 | 122.46 | 199.58 |
| Example 28 | Compound 230 | 10 | 6.39 | 121.97 | 199.30 |
| Example 29 | Compound 250 | 10 | 6.18 | 126.20 | 212.87 |
| Example 30 | Compound 252 | 10 | 6.22 | 125.78 | 213.45 |

As shown in Table 3 above, it was confirmed that the organic light-emitting devices of the examples had lower driving voltages and better efficiency and lifespans than the organic light-emitting devices of the comparative examples.

Looking specifically at comparative examples and comparative compounds, it was observed that when a rigid fluorene-based substituent was introduced to 1,10-phenanthroline compared to BPhen, the operating voltage was lowered and the efficiency and lifespan were improved. However, when fluorene was substituted with an alkyl group or heteroaryl group, as in comparative compounds 1 and 2, there was a limit to the improvement in device performance. This is expected to be because the thermal stability was low and the packing density was low, which prevented smooth intermolecular packing.

In addition, in the case of a structure in which fluorene is not bonded to the position immediately adjacent to N of 1,10-phenanthroline, such as comparative compounds 3 and 4, the improvement in performance such as efficiency was low. This is expected to be because the structure has a relatively high RE value due to limited expansion of conjugation.

In the case of comparative compounds 5 to 9, in which 1,10-phenanthroline has a structure in which it is bonded to the lower side of fluorene rather than the upper side, there was also a limit to the improvement in device performance, and this is expected to be because the structure has a relatively high RE value.

Table 4 below shows the result of calculating RE value at the DFT b3lyp/6-31g(d) level using the Gaussian 09W program. When comparing the RE value of the compound of the present invention with that of a comparative compound having only a different actual bonding position, it was confirmed that the RE value of the compound of the present invention was lower than that of the comparative compound.

**[Table 4]**

| Comparative Compound | RE value | Compound of the present invention | RE value |
|---|---|---|---|
| (Comparative Compound 3) | 0.347 | 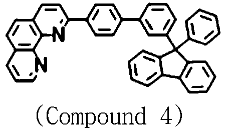 | 0.266 |
| (Comparative Compound 4) | 0.384 | 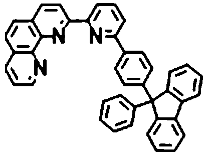 | 0.221 |
| | | 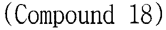 | |
| (Comparative Compound 6) | 0.273 | 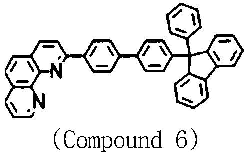 | 0.241 |

The novel compound of the present invention described so far particularly preferable for application to an N-type charge generation layer because it can easily combine with dopants such as lithium and ytterbium by introducing 1,10-phenanthroline having a non-shared electron pair, and also has excellent thermal stability by introducing a rigid fluorene-based substituent. In addition, the compound has a reverse structure in which 1,10-phenanthroline is bonded to phenyl or naphthyl located above diphenylfluorene or naphthylphenylfluorene, and thus has a lower RE (Reorganization Energy) value compared to a structure in which it is bonded to the lower fluorene, thereby increasing the efficiency of the device. In addition, the compound has a structure in which the position immediately adjacent to N of 1,10-phenanthroline is bonded, thereby expanding the conjugation and lowering the RE value compared to other positions, thereby increasing the efficiency of the device. Furthermore, fluorene is not further substituted except for hydrogen and deuterium, so fluorene is located at the molecular terminal. This increases the packing density and refractive index, which in turn increases the lifespan of the device.

The novel compound of the present invention can be usefully applied to one or more organic layers of an electron transport layer (ETL), a hole blocking layer (HBL), and an N-type charge generation layer (N-CGL) among organic layers of an organic light-emitting device, and in particular, when applied to a host of an N-type charge generation layer of a tandem organic light-emitting device, it can enable the implementation of low voltage, high efficiency, and long life of the device.

## Claims

1. A novel compound, wherein the compound is represented by Formula 1 below: 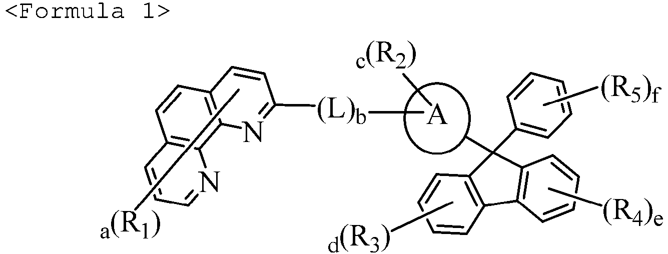 In the Chemical Formula 1,
ring A is phenyl or naphthyl,
R1 is each independently hydrogen, deuterium, a halogen group, a cyano group, a nitro group, a nitrile group, a hydroxyl group, a thiol group, a substituted or unsubstituted C0~C50 amine group, a substituted or unsubstituted C1~C50 alkyl group, a substituted or unsubstituted C1~C50 alkenyl group, a substituted or unsubstituted C1~C50 alkynyl group, a substituted or unsubstituted C1~C50 alkoxy group, a substituted or unsubstituted C1~C50 sulfide group, a substituted or unsubstituted C0~C50 silyl group, a substituted or unsubstituted C3~C50 cycloalkyl group, a substituted or unsubstituted C2~C50 heterocycloalkyl group, a substituted or unsubstituted C3~C50 cycloalkenyl group, a substituted or unsubstituted C1~C30 phosphine oxide group, a substituted or unsubstituted C2~C50 heterocycloalkenyl group, a substituted or unsubstituted C6~C50 aryl group, a substituted or unsubstituted C6~C50 aryloxy group, a substituted or unsubstituted C2~C50 heteroaryloxy group, or a substituted or unsubstituted C2~C50 heteroaryl group, wherein adjacent groups may or may not combine with each other to form a substituted or unsubstituted ring,
L is each independently a direct bond, a substituted or unsubstituted C6~C50 arylene group, or a substituted or unsubstituted C2~C50 heteroarylene group,
R2 to R5 are each independently hydrogen or deuterium, or may or may not be combined with each other to form a ring substituted or unsubstituted with one or more deuteriums,
a is an integer from 0 to 7,
b is an integer from 0 to 3,
c is an integer from 0 to 6,
d and e are each independently integers from 0 to 4, and
f is an integer from 0 to 5.

2. The compound of claim 1, wherein the Chemical Formula 1 is represented by one of the following Chemical Formula 2 to Chemical Formula 4: 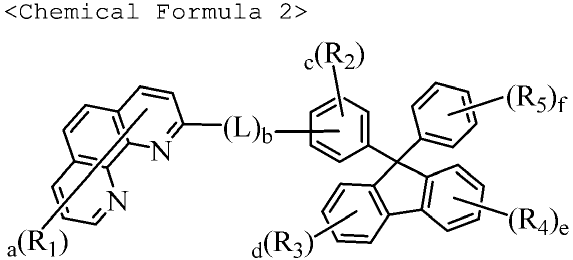 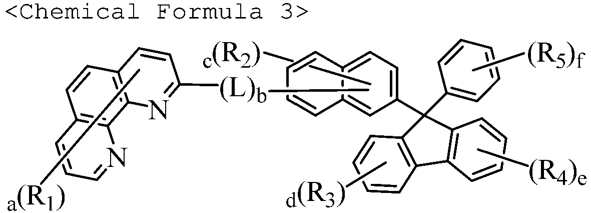 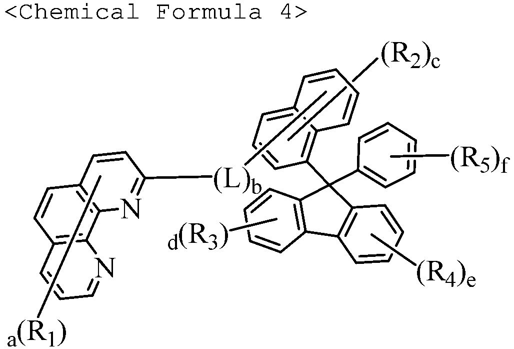 In the Chemical Formulas 2 to 4,
R1, L, R2 to R5, and a to f have the same definitions as in the Chemical Formula 1.

3. The compound of claim 1, wherein the Chemical Formula 1 is represented by one of the following Chemical Formulas 2-1 to 2-3, 3-1 to 3-3, 4-1 and 4-2: 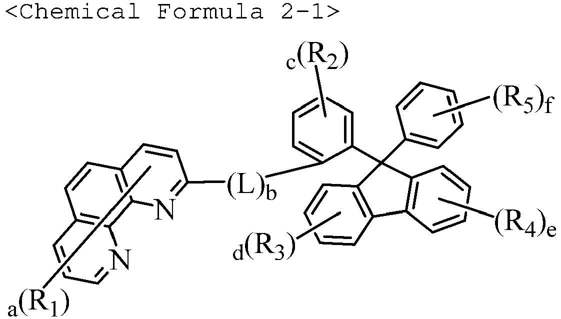 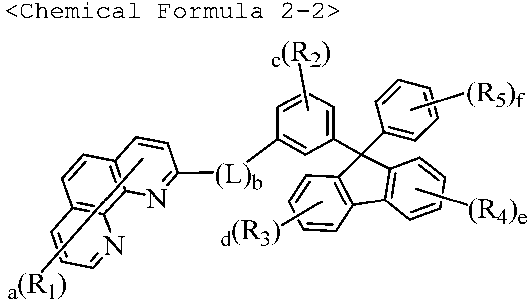 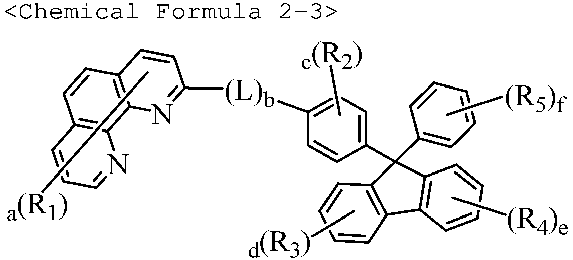 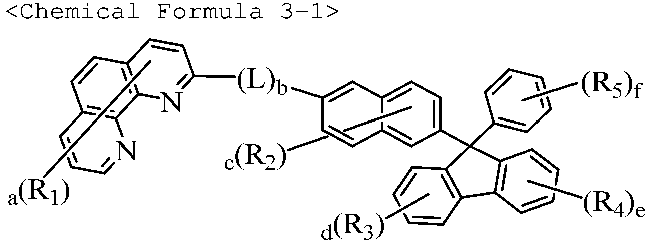 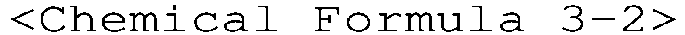 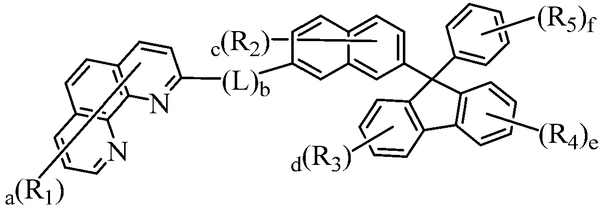 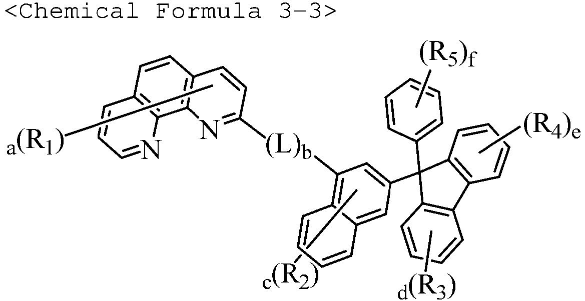 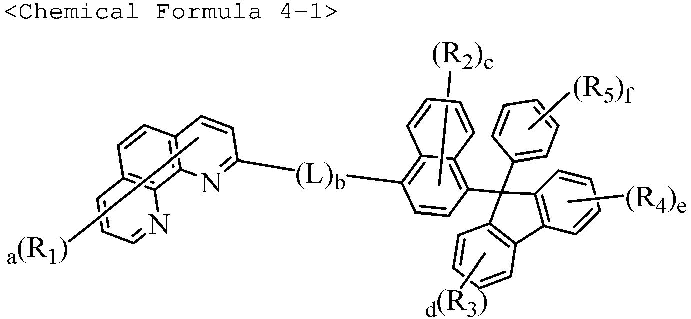 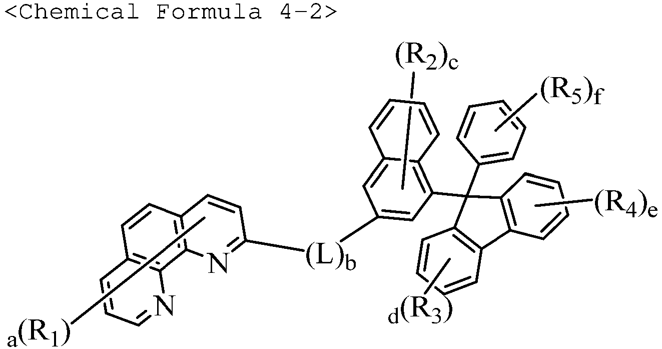 In the Chemical Formulas 2-1 to 2-3, 3-1 to 3-3, 4-1 and 4-2, R1, L, R2 to R5 and a to f are the same as defined in the Chemical Formula 1.

4. The compound of claim 1, wherein the Chemical Formula 1 is represented by the following Chemical Formula 5: 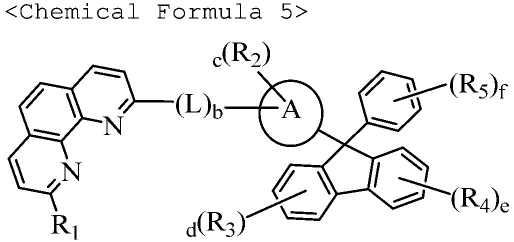 In the Chemical Formula 5,
L, R2 to R5 and b to f are the same as defined in the above Chemical Formula 1,
R1 is one of hydrogen, deuterium, a methyl group, -CD3, a phenyl group, a biphenyl group, a naphthyl group, a pyridine group, a pyrimidine group, a quinoline group, an isoquinoline group, a quinazoline group, and a naphthyridine group.

5. The compound of claim 1, wherein at least one group among R3, R4, and R5 is bonded to adjacent one to form a benzene unsubstituted or substituted with one or more deuterium atoms.

6. The compound of claim 5, wherein the fluorene moiety in the Chemical Formula 1 is represented by one of the following Structural Formula F-1 to Structural Formula F-5: 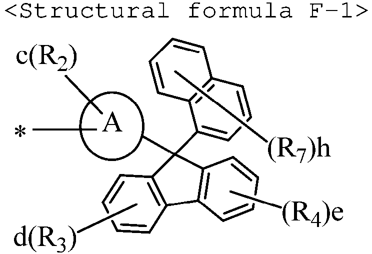 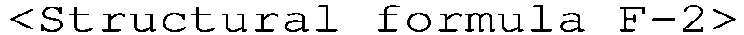 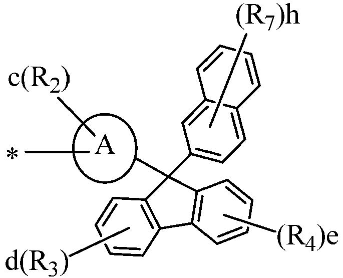 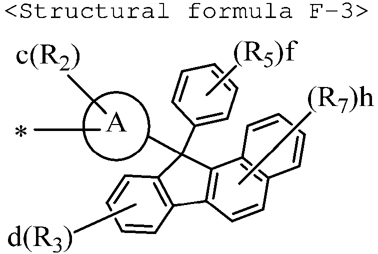 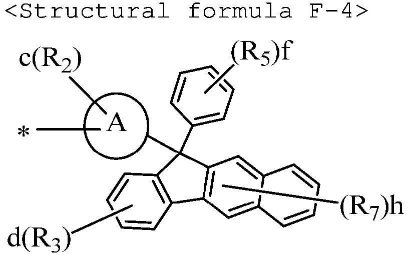 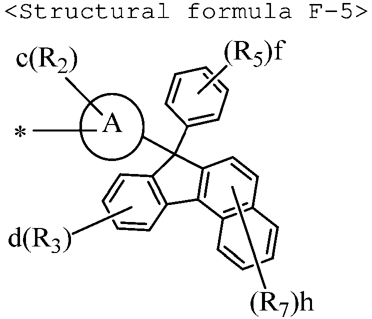 In the Structural formula F-1 to F-5, ring A is the same as defined in the Chemical Formula 1,
R2 to R5 and R7 are each independently hydrogen or deuterium, c to f are the same as defined in the Chemical Formula 1,
h is each independently an integer from 0 to 6, and
"*" indicates a position bonded to L, a linker in Chemical Formula 1.

7. The compound of claim 1, wherein the Chemical Formula 1 is represented by one of the following Chemical Formulas A to H: 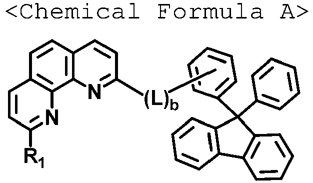 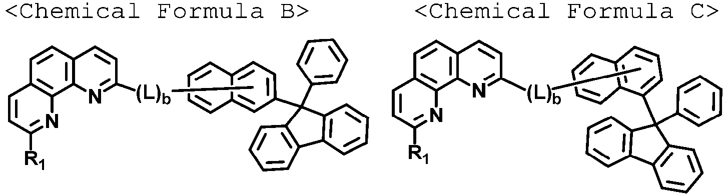 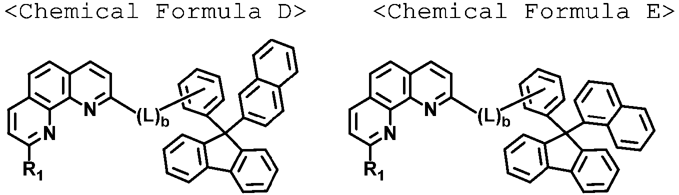 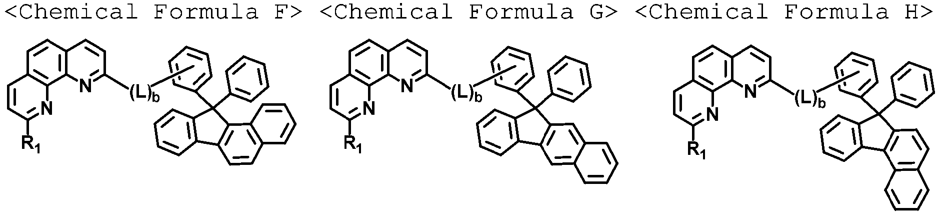 In the Chemical Formulas A to H,
R1, L and b are the same as defined in the Chemical Formula 1,

8. The compound of claim 1, wherein the compound represented by Chemical Formula 1 is one of the following compounds 1 to 543:

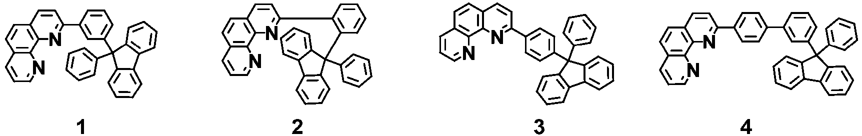

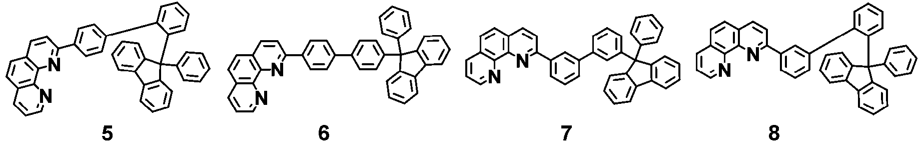

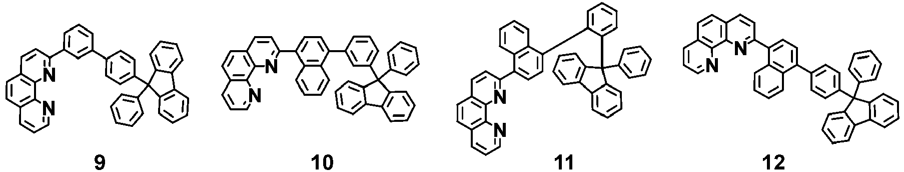

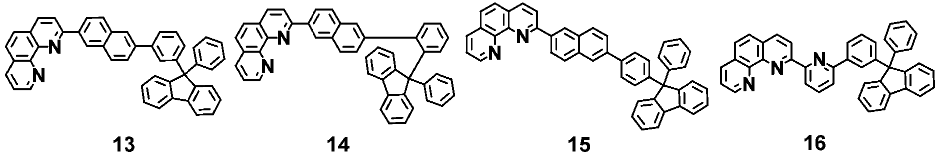

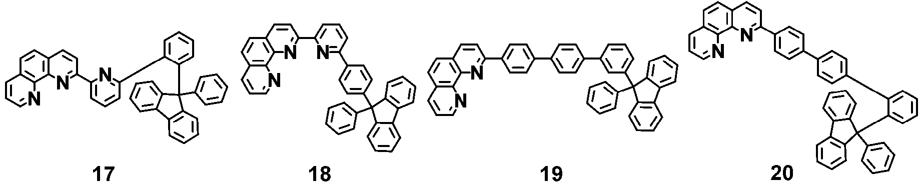

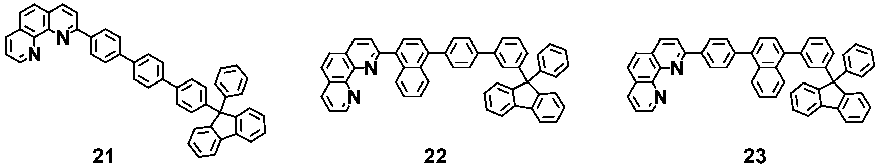

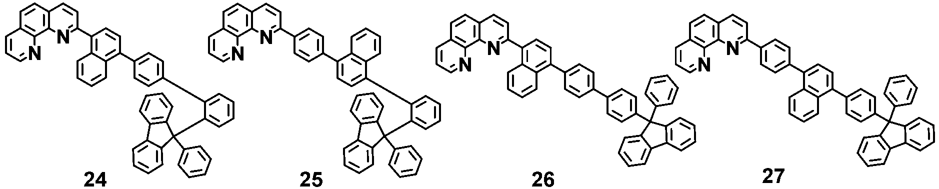

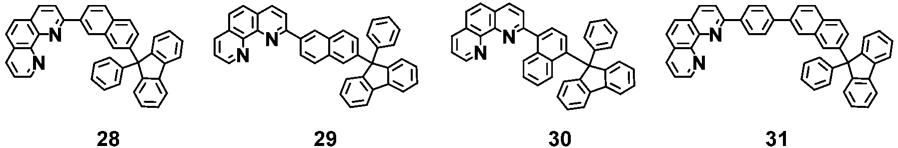

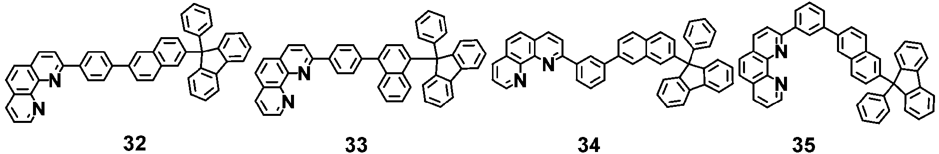

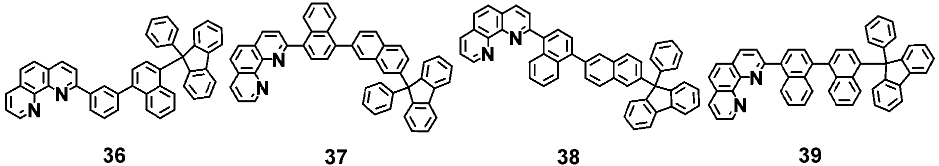

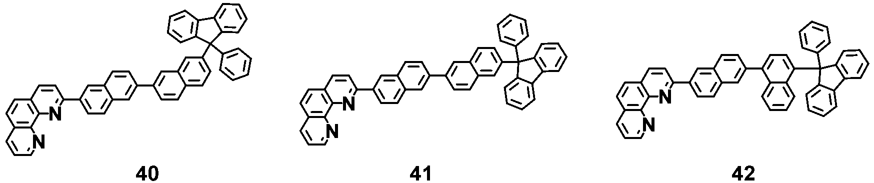

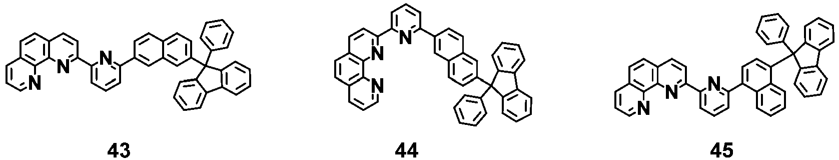

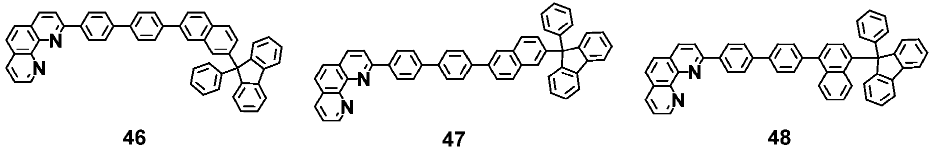

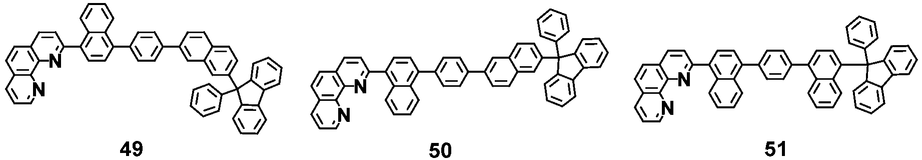

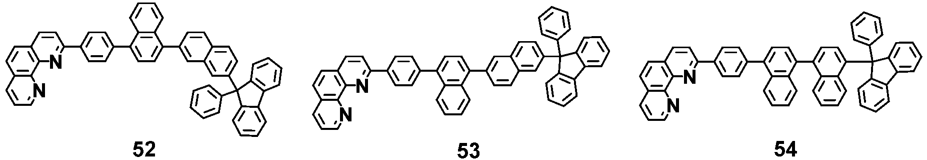

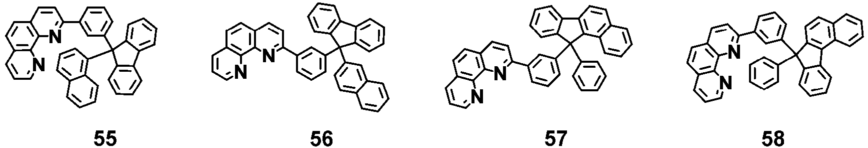

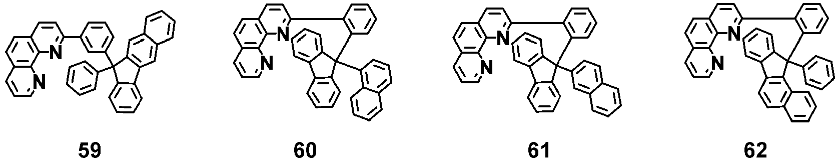

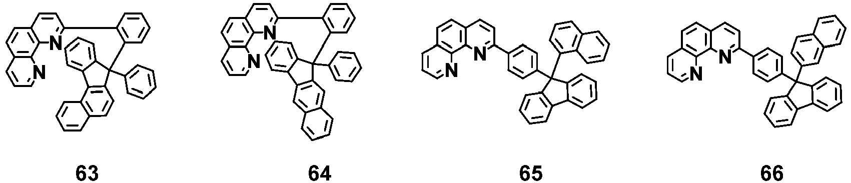

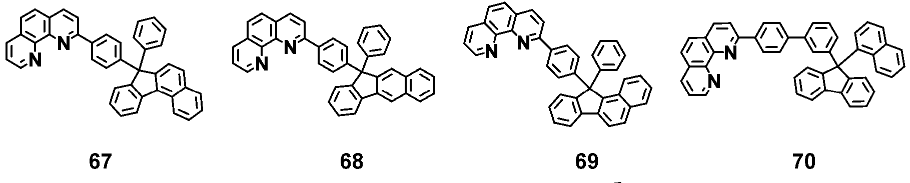

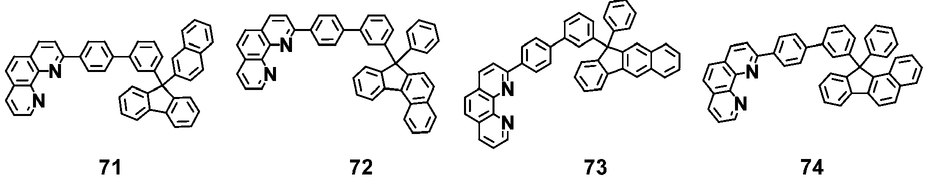

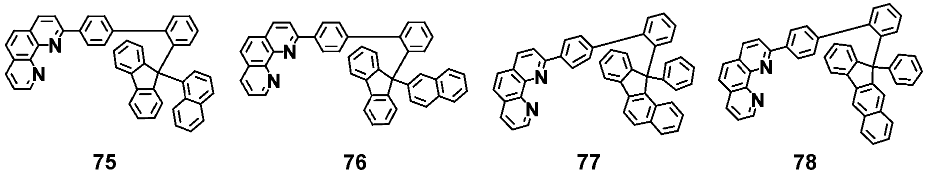

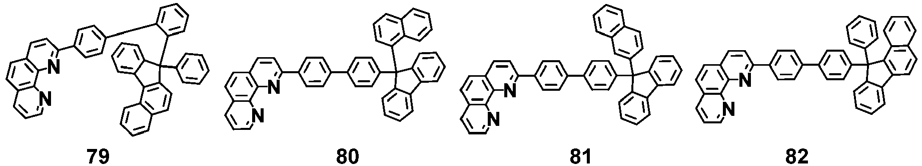

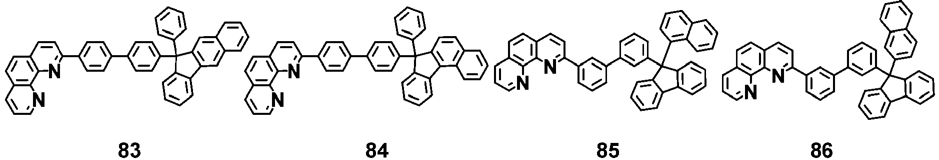

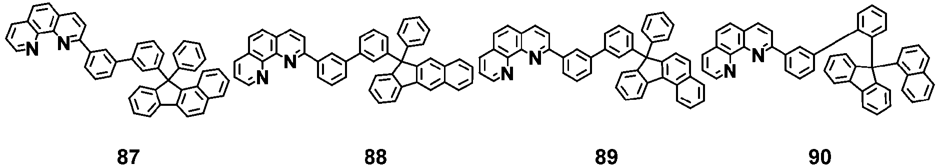

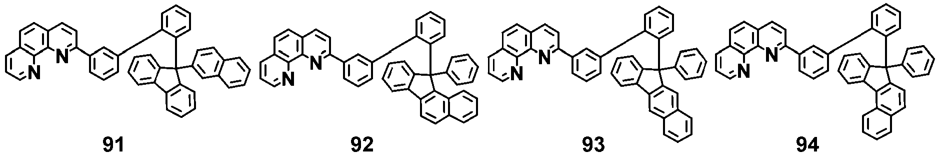

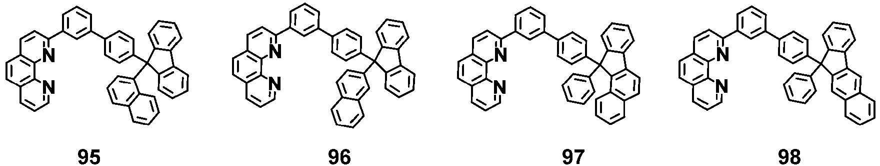

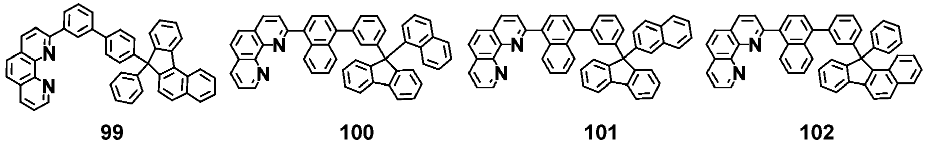

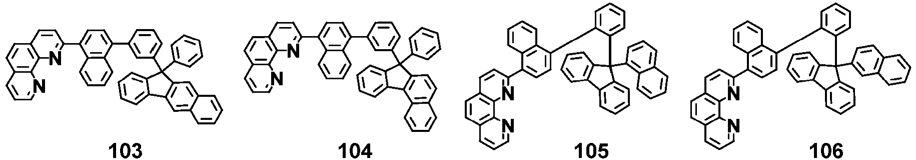

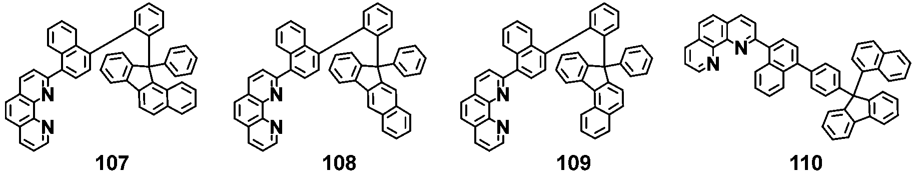

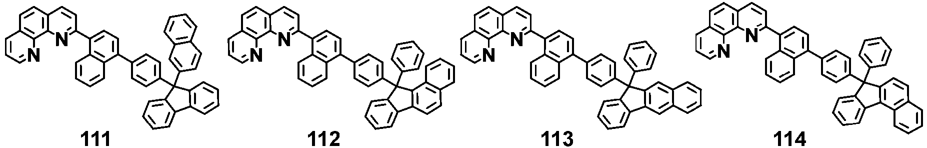

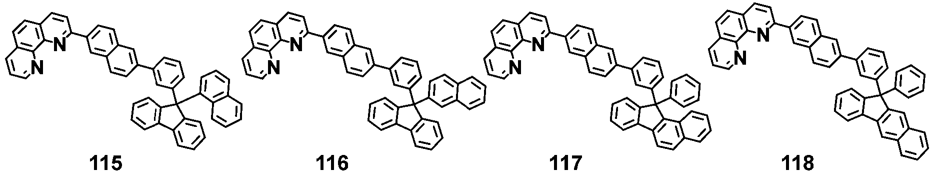

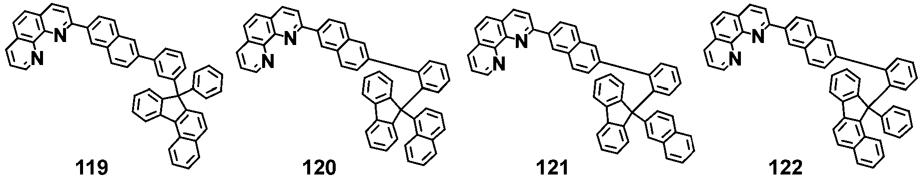

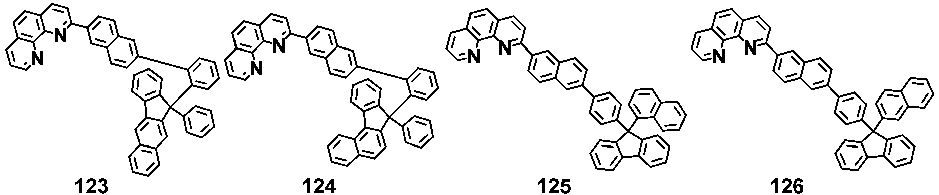

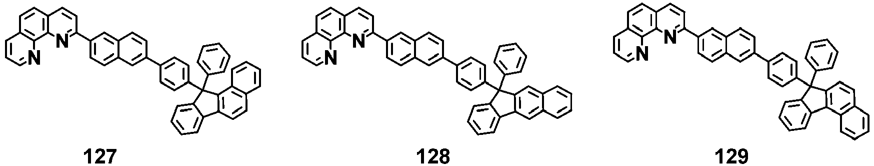

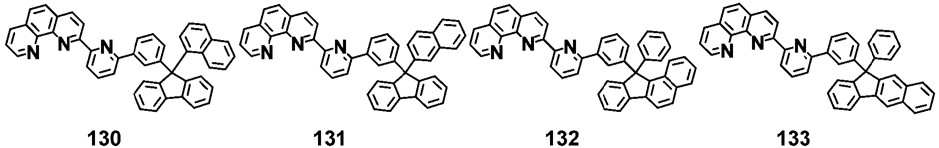

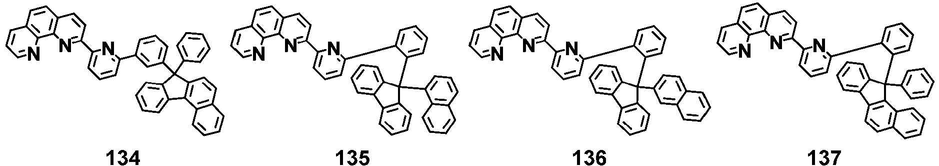

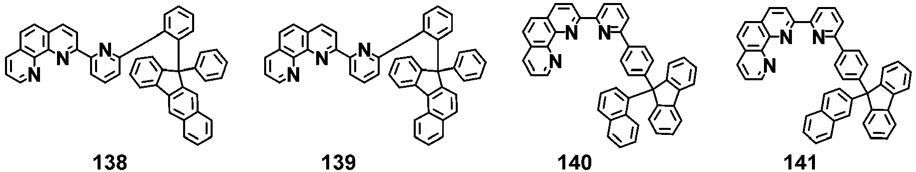

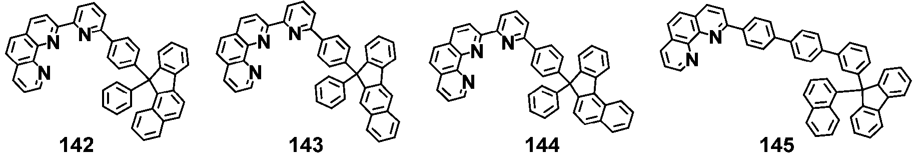

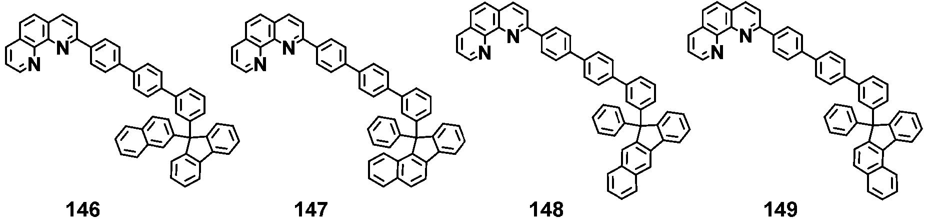

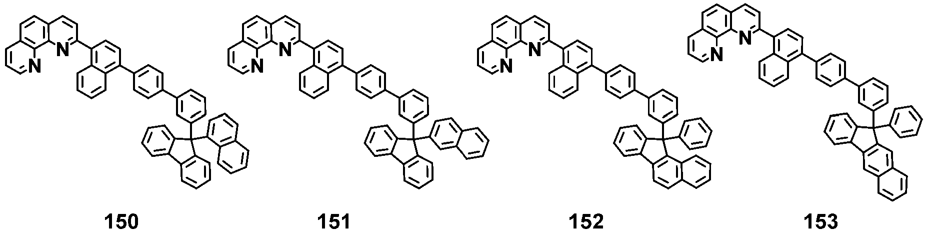

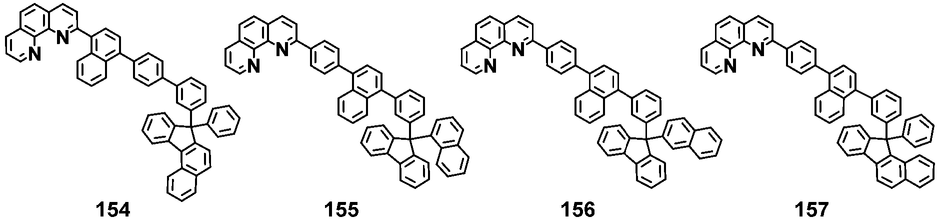

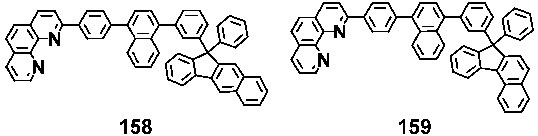

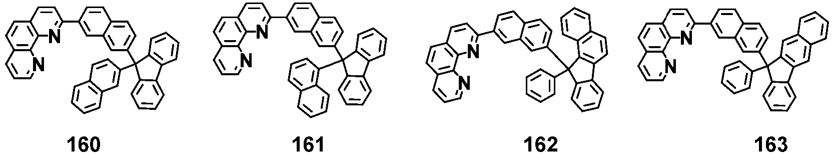

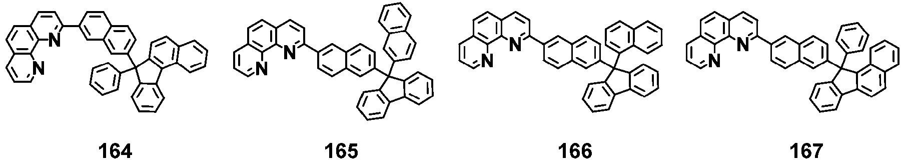

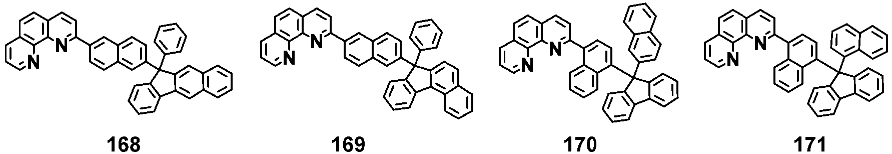

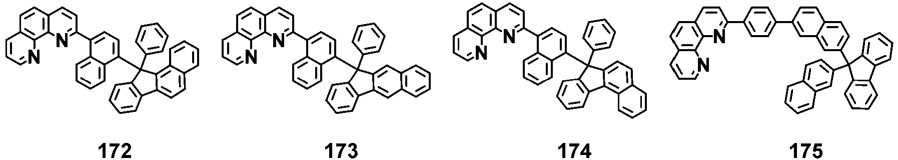

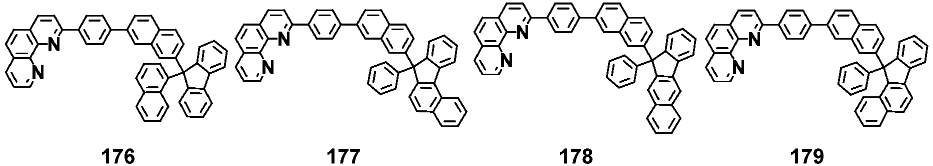

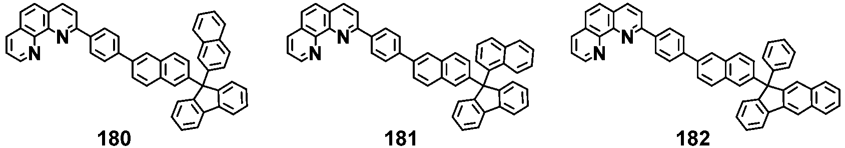

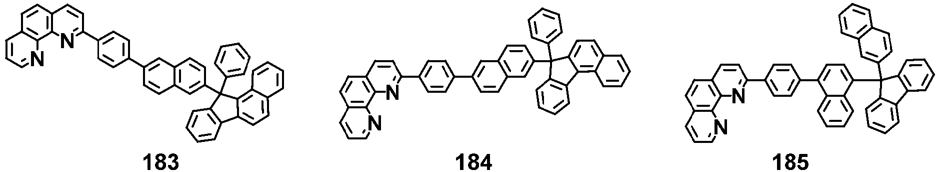

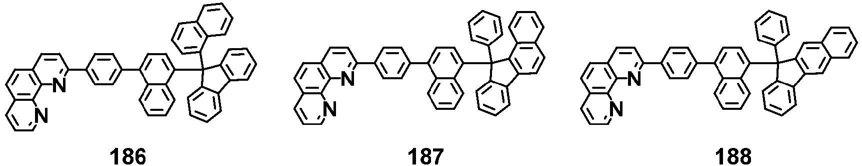

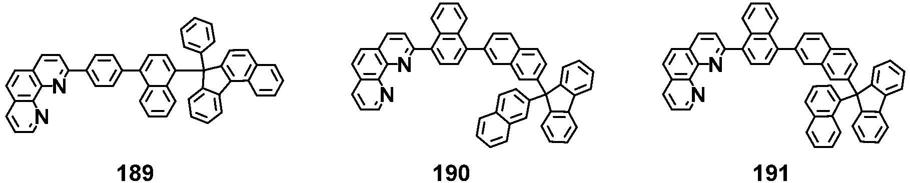

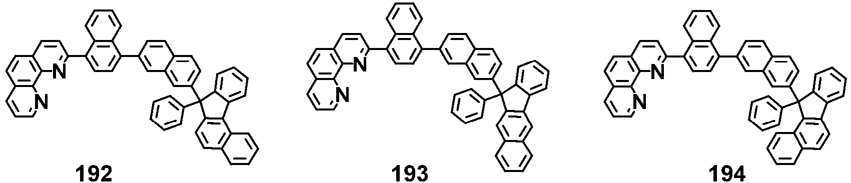

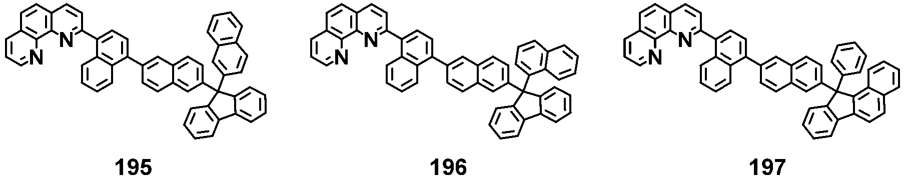

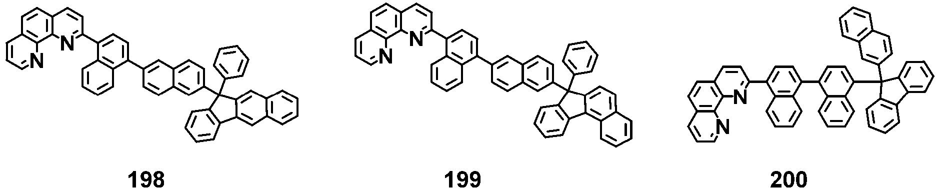

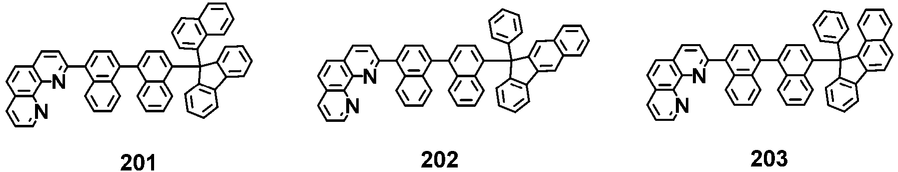

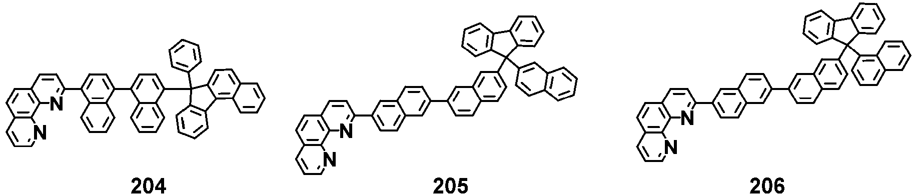

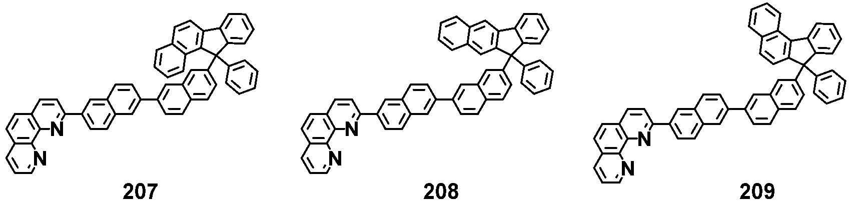

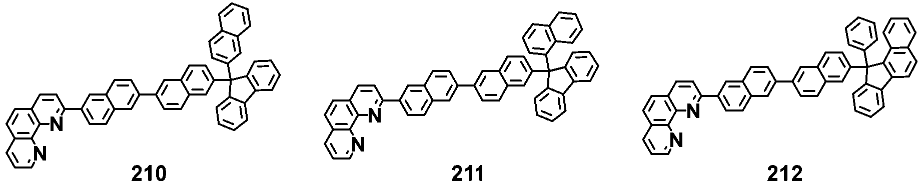

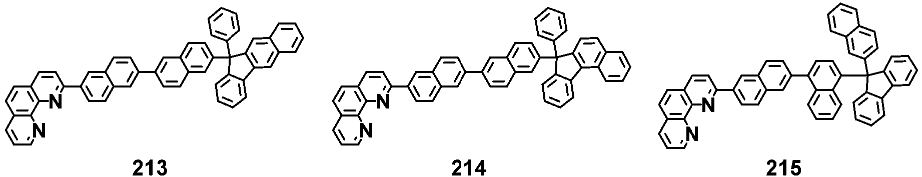

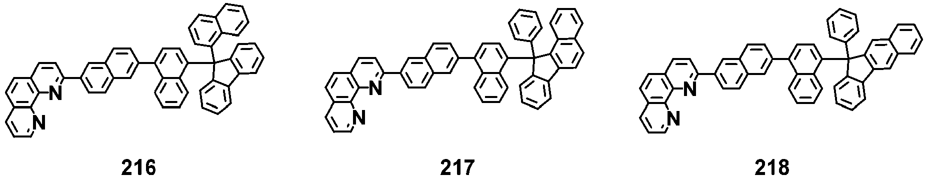

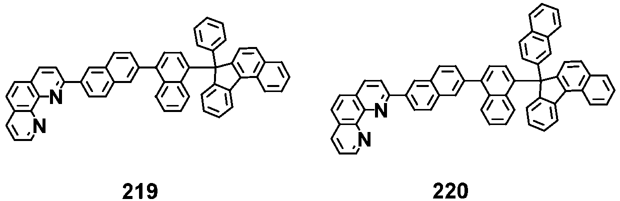

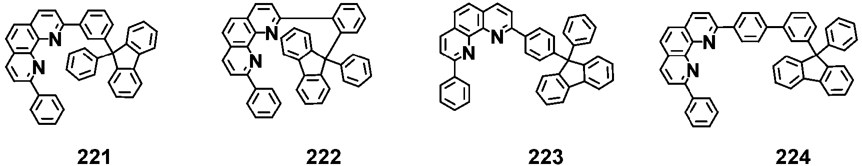

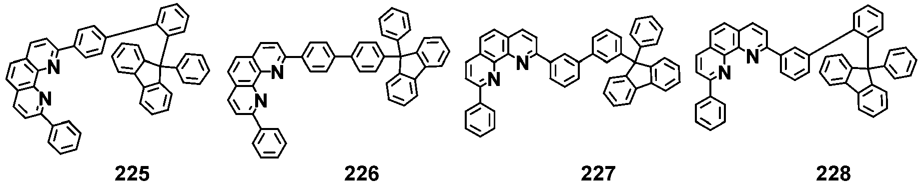

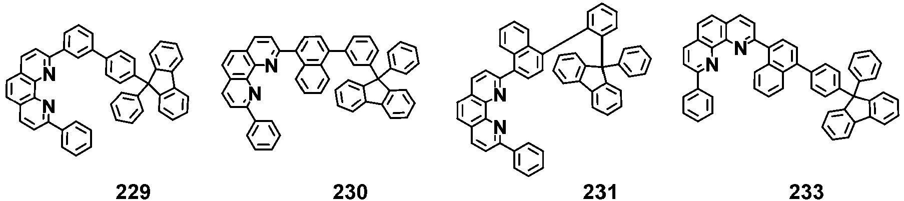

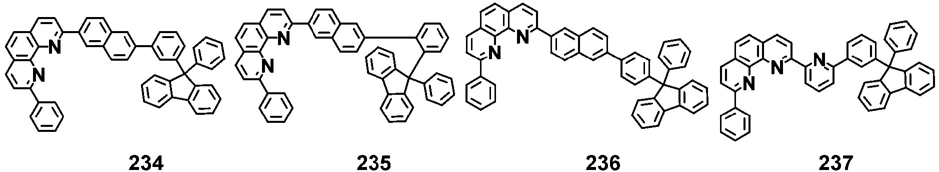

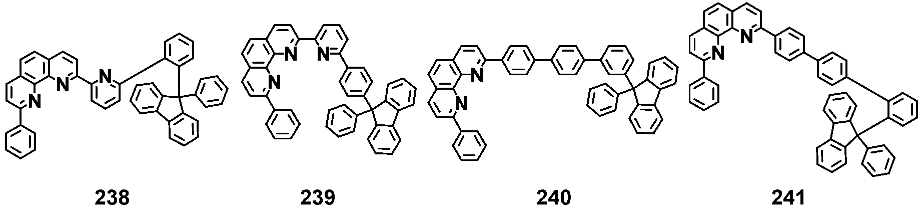

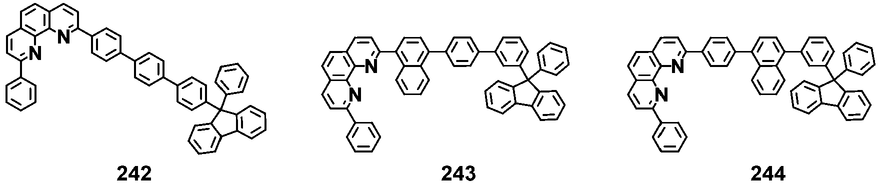

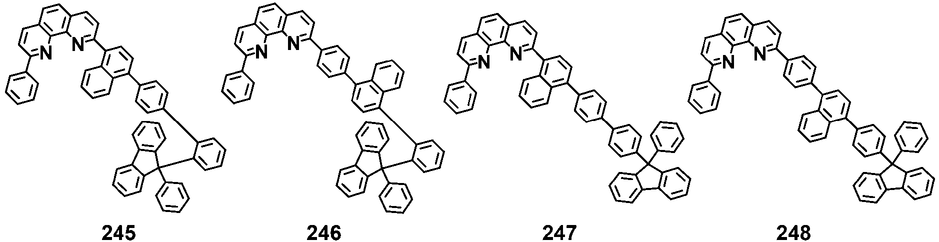

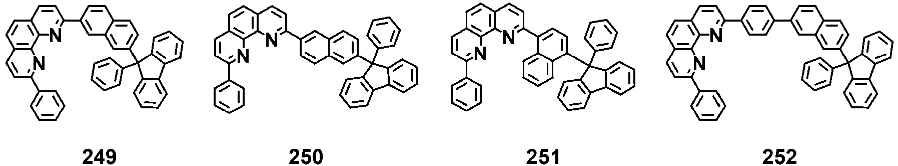

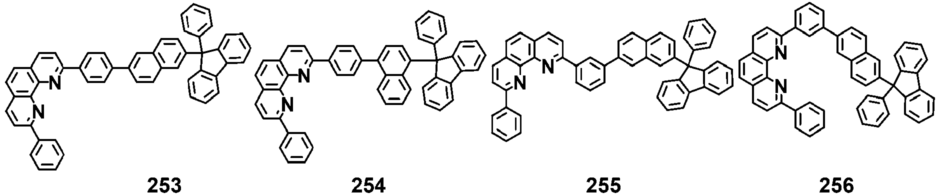

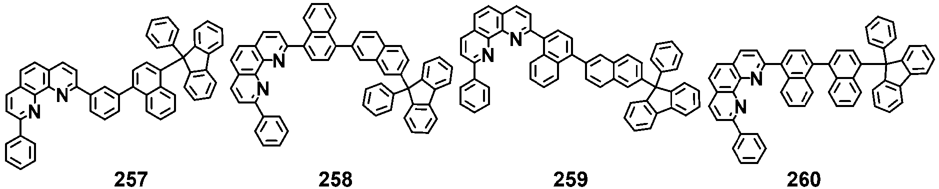

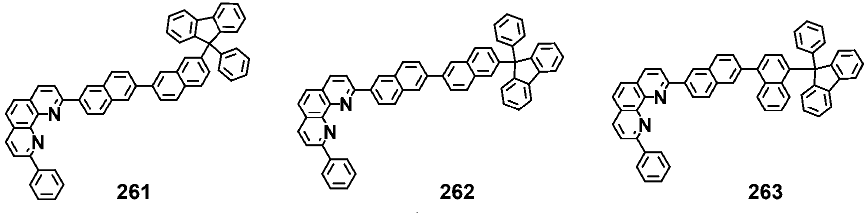

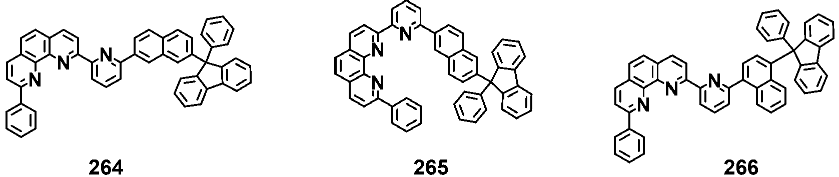

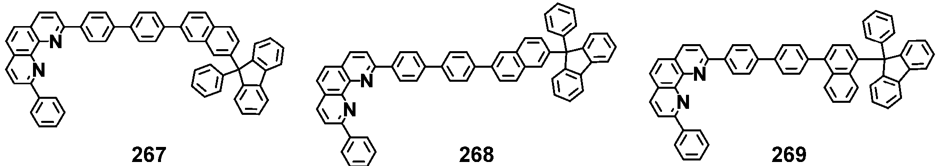

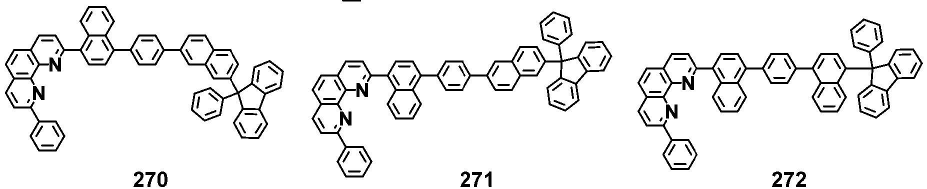

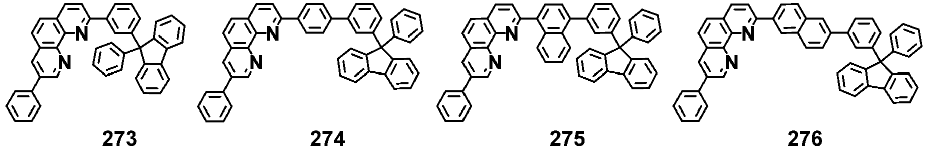

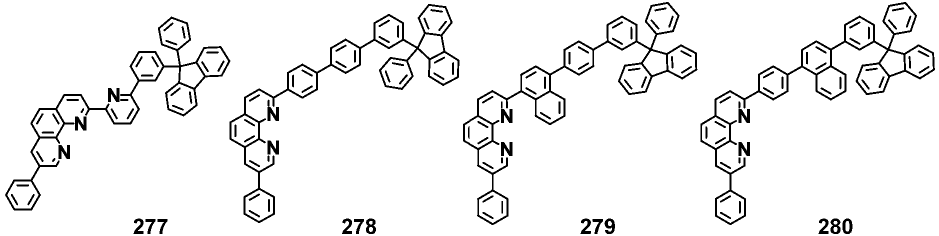

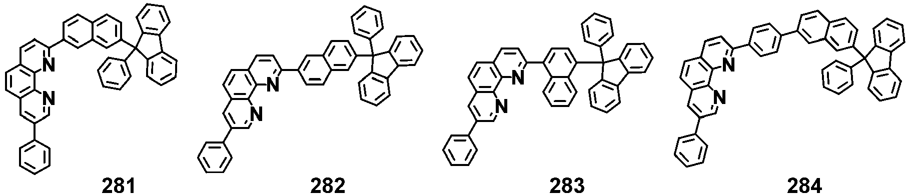

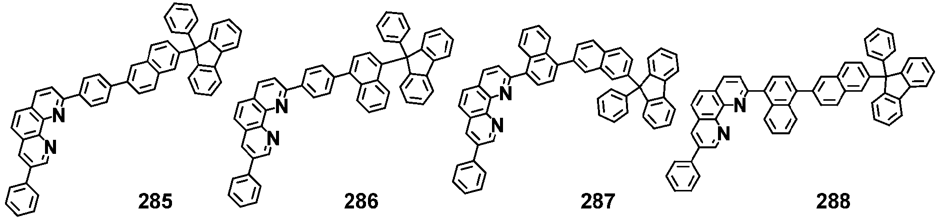

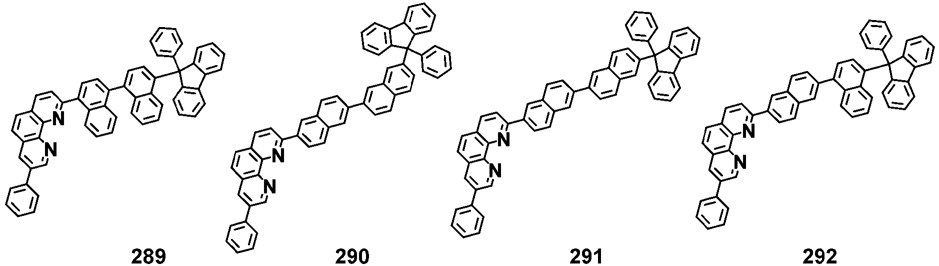

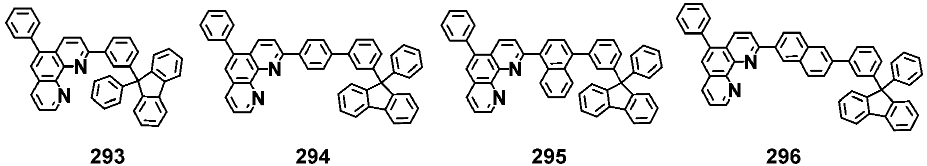

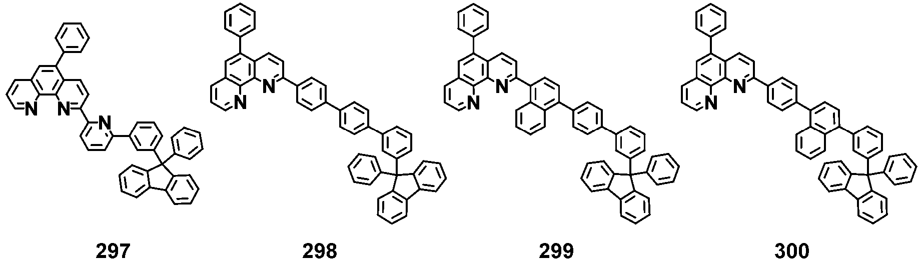

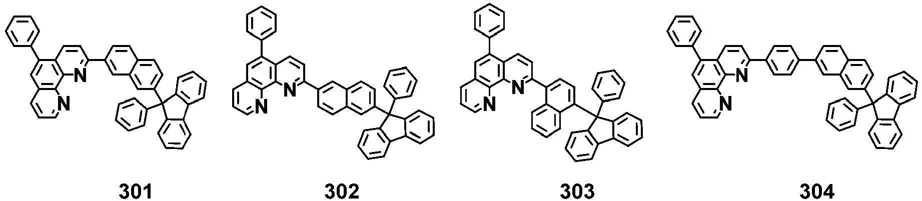

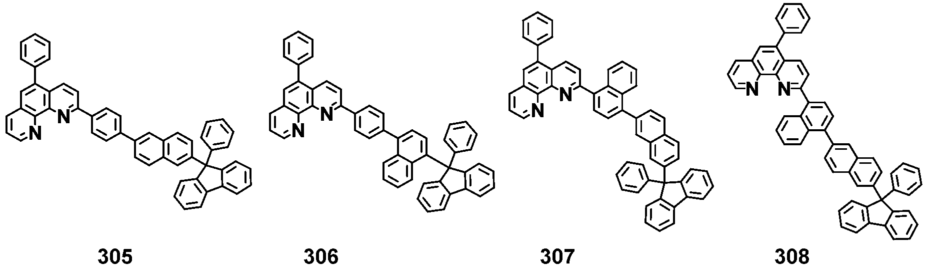

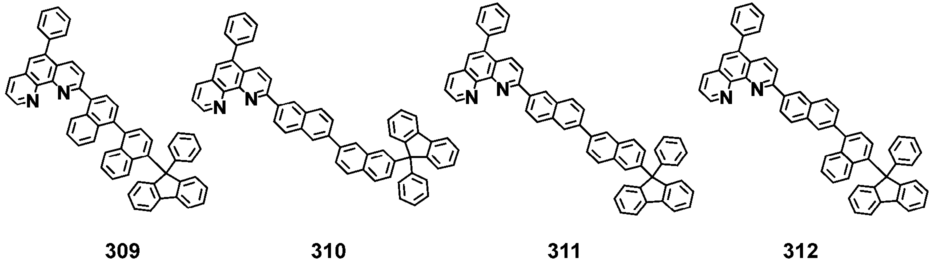

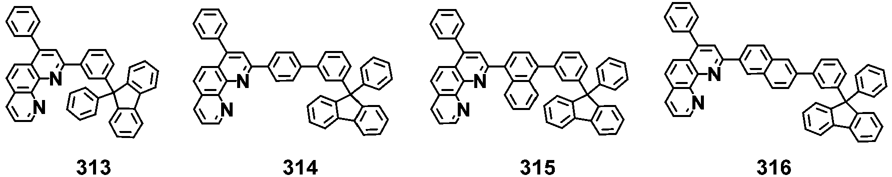

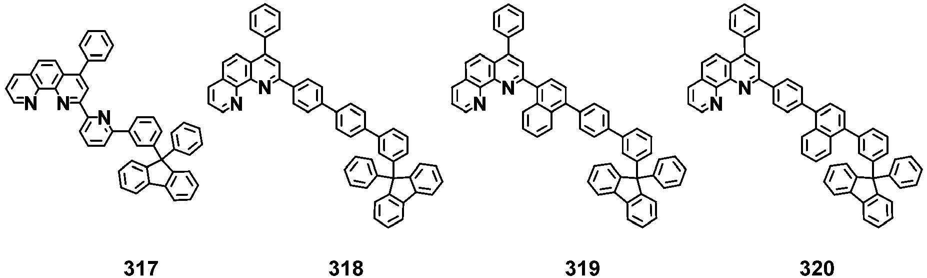

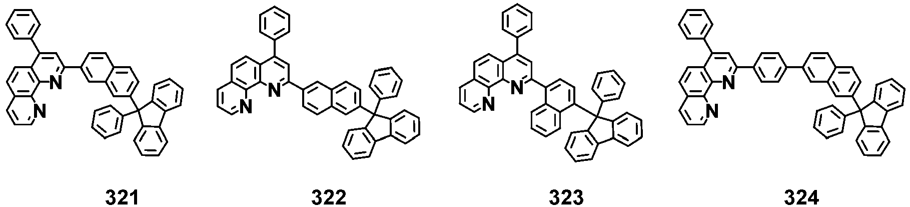

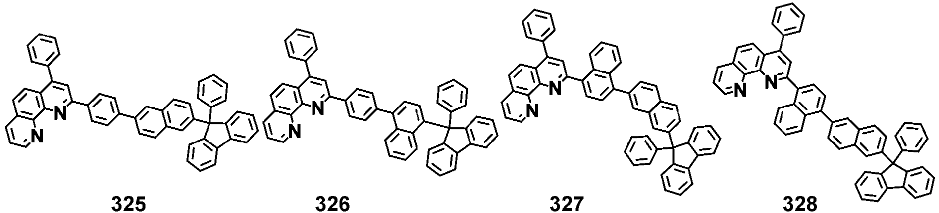

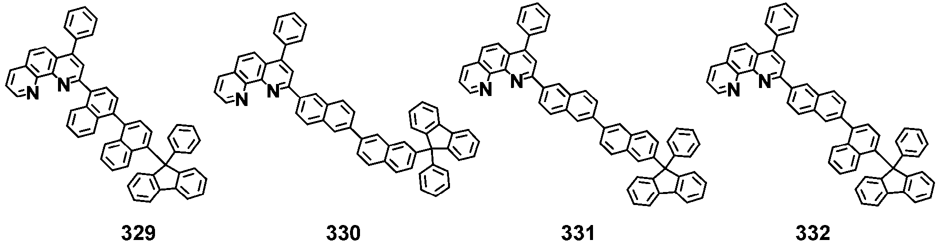

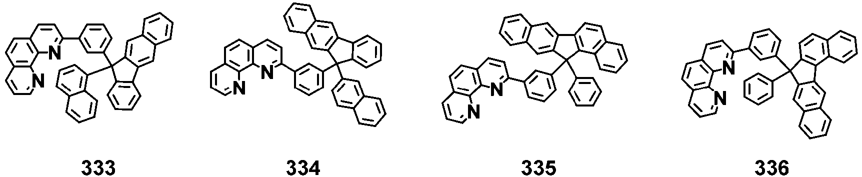

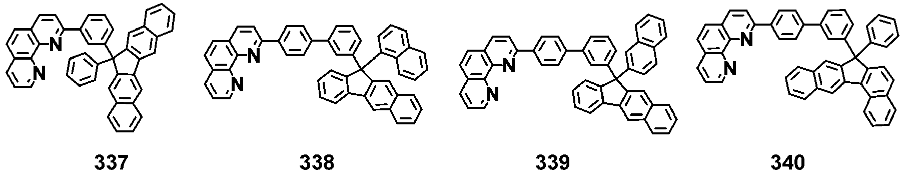

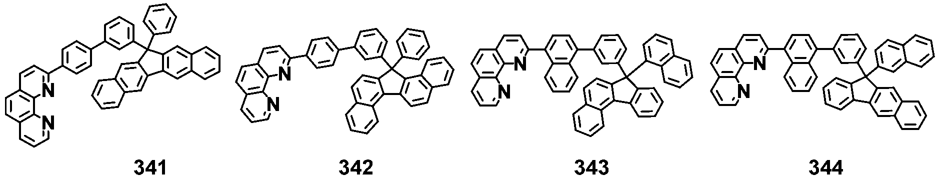

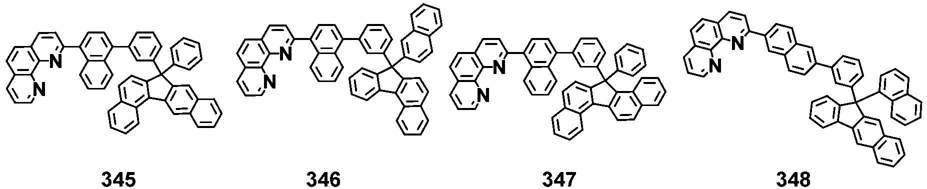

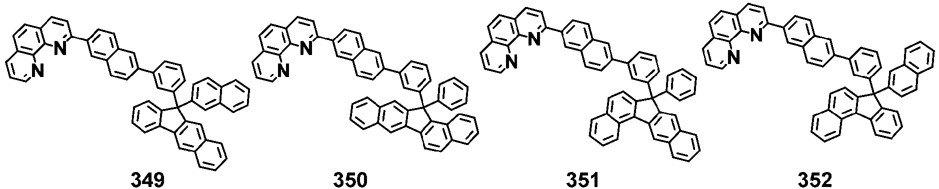

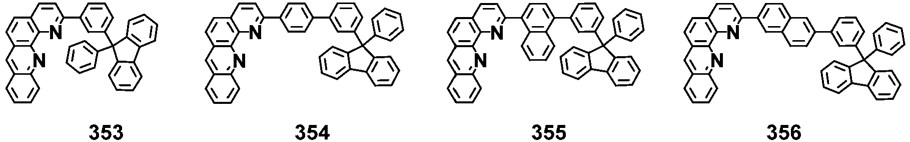

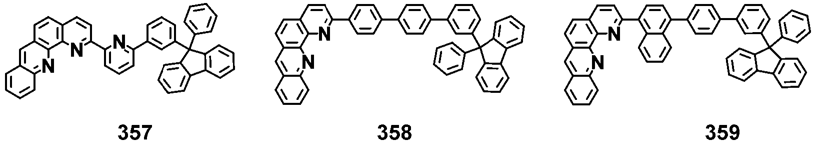

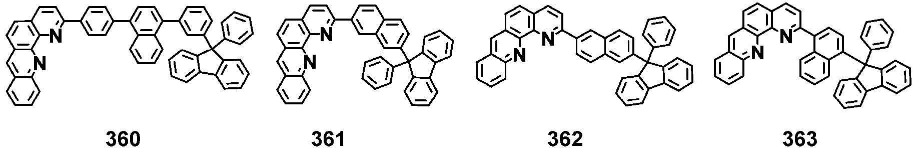

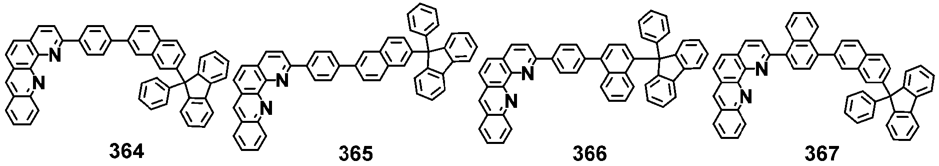

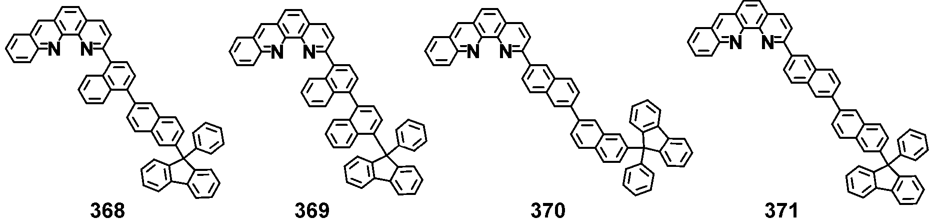

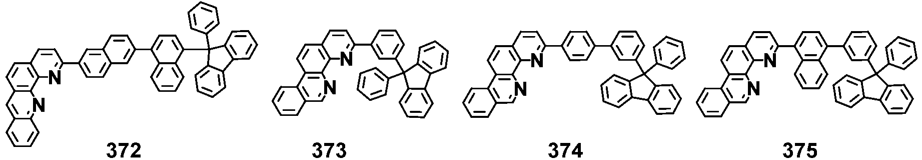

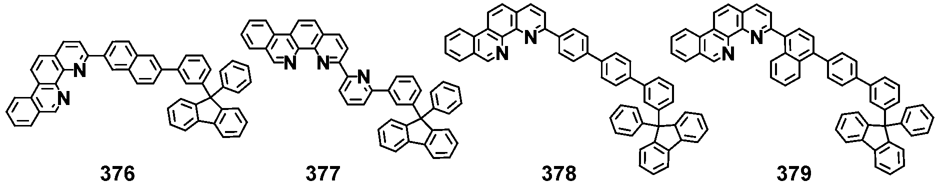

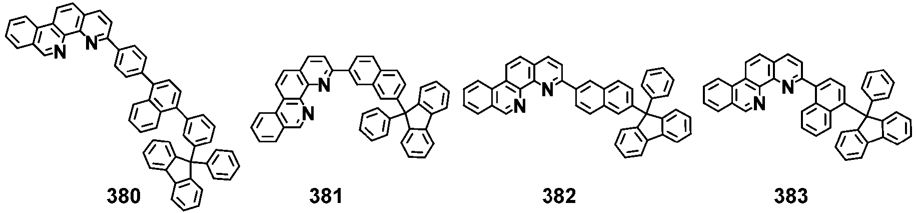

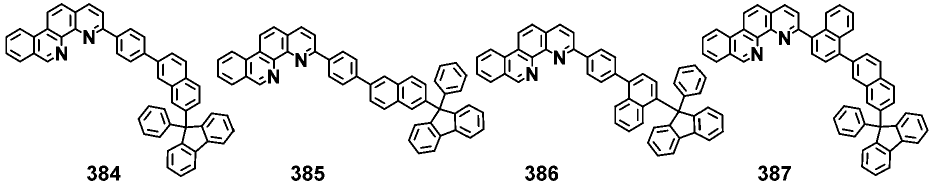

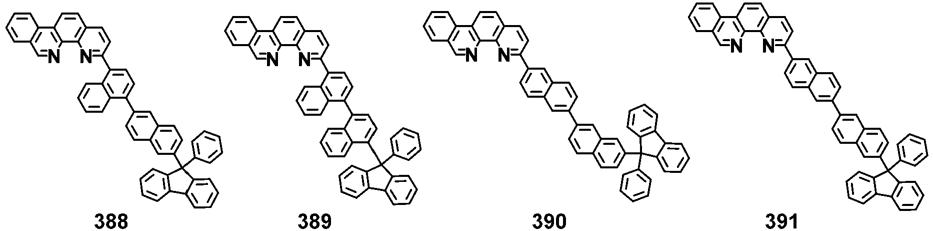

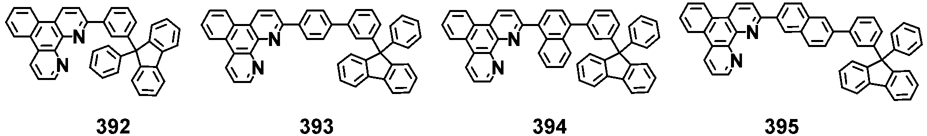

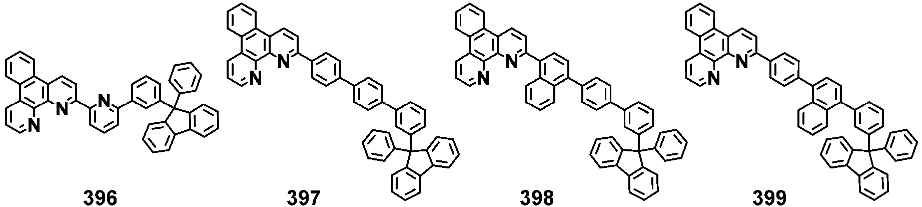

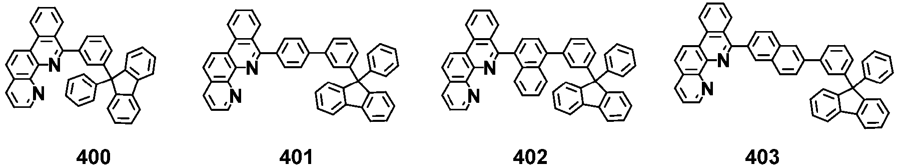

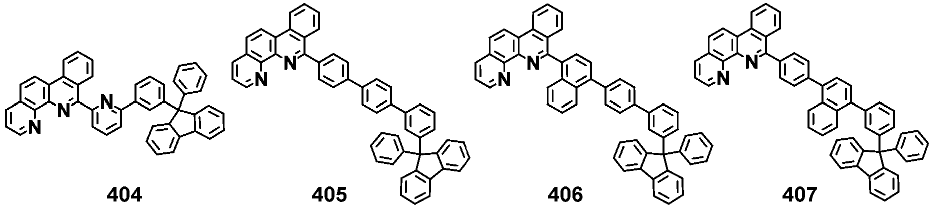

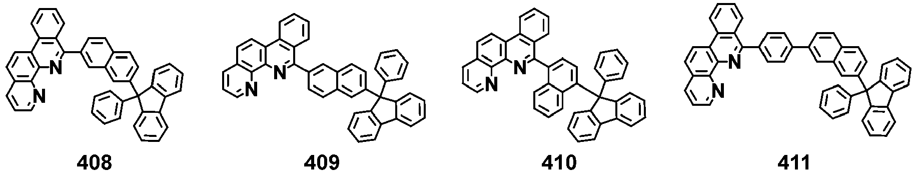

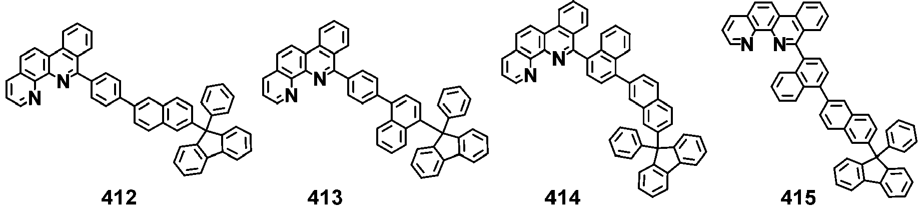

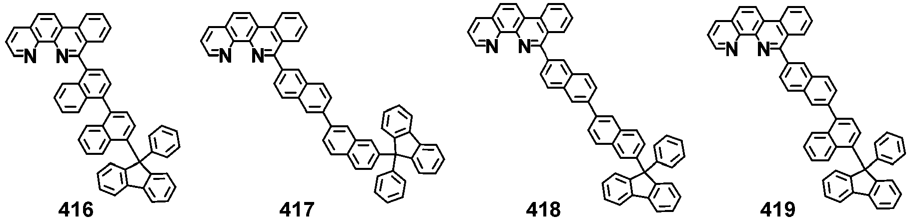

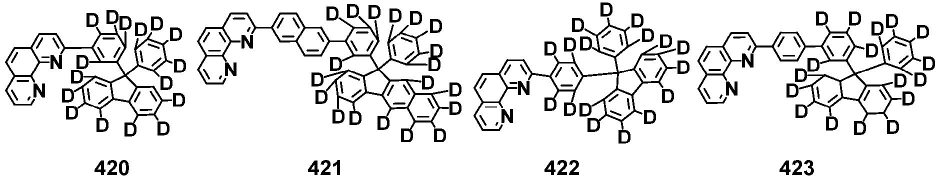

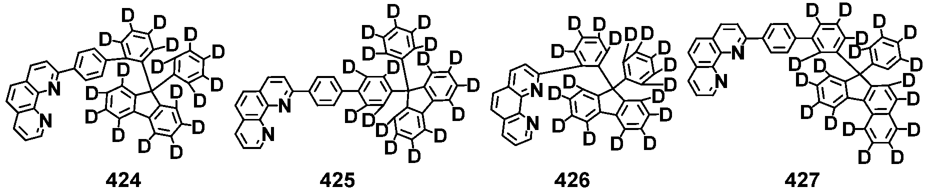

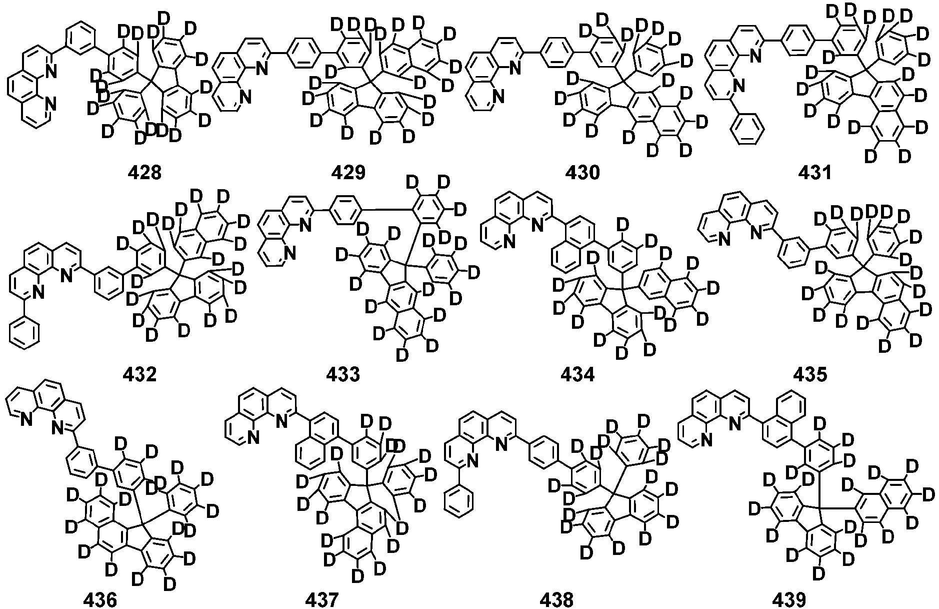

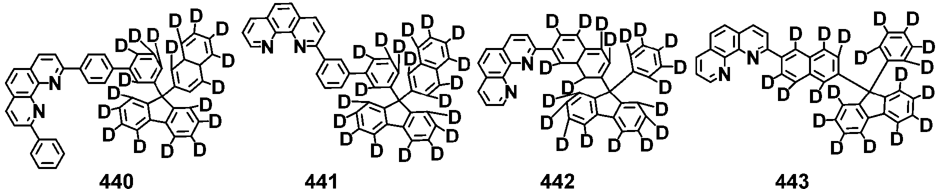

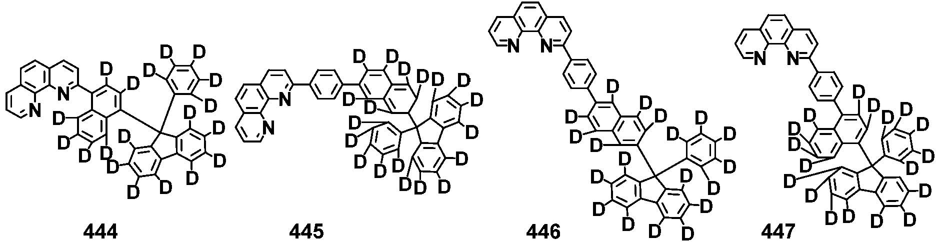

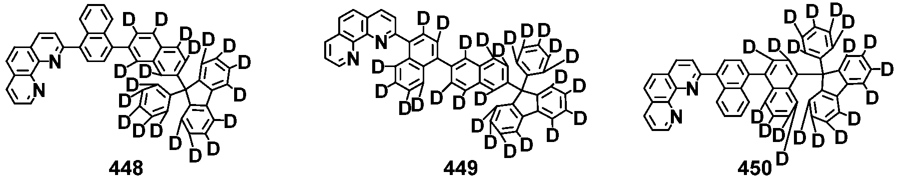

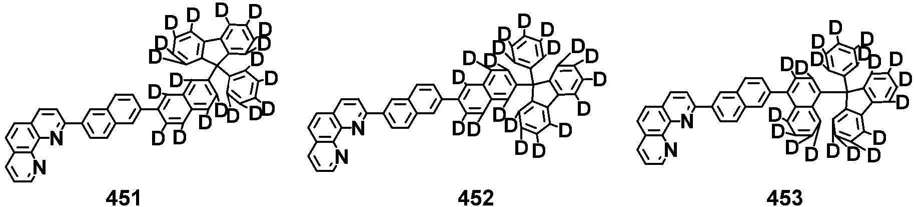

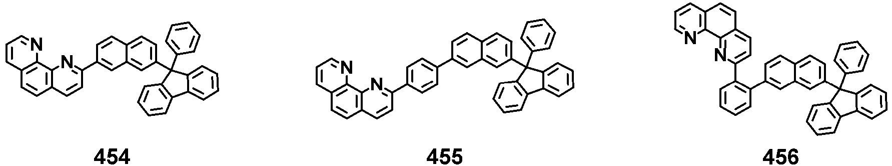

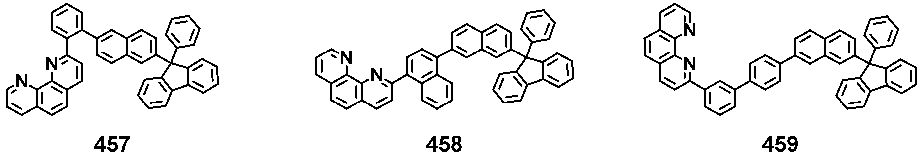

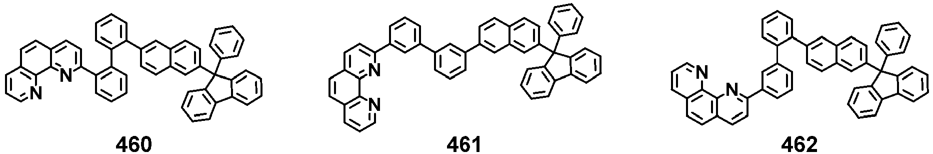

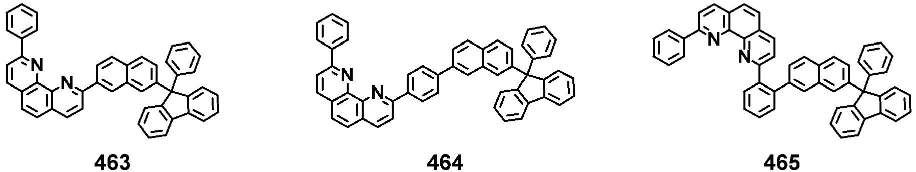

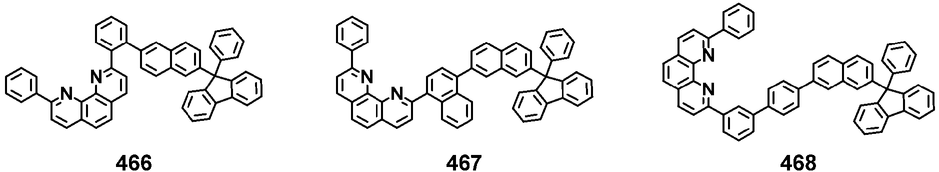

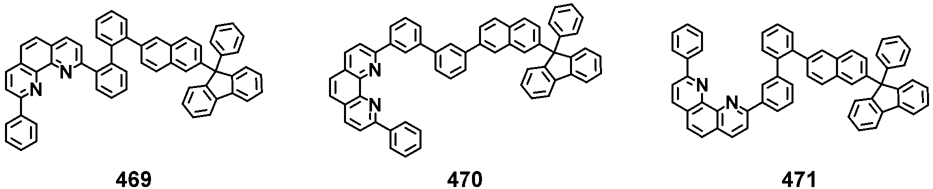

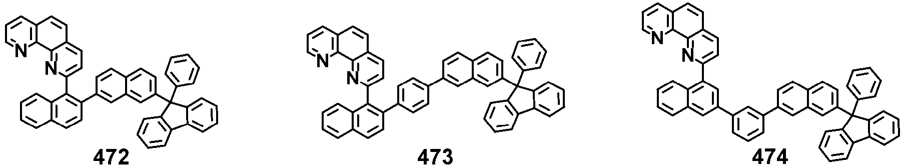

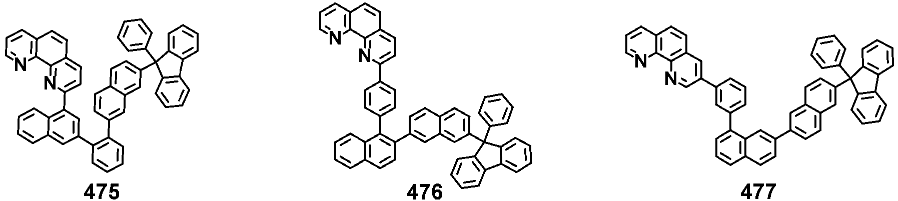

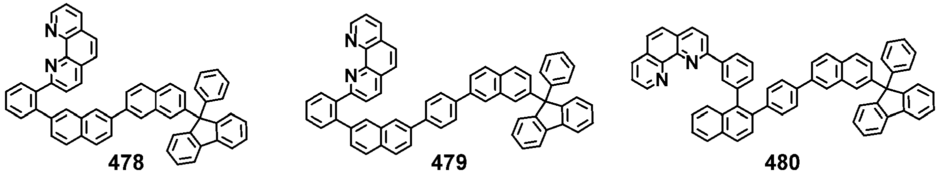

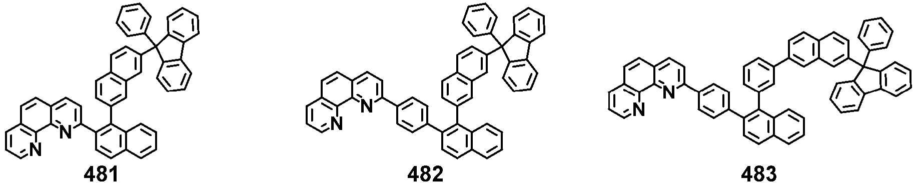

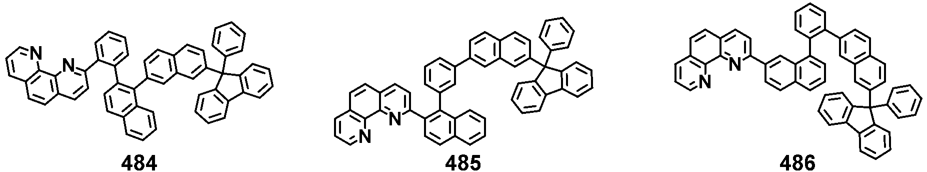

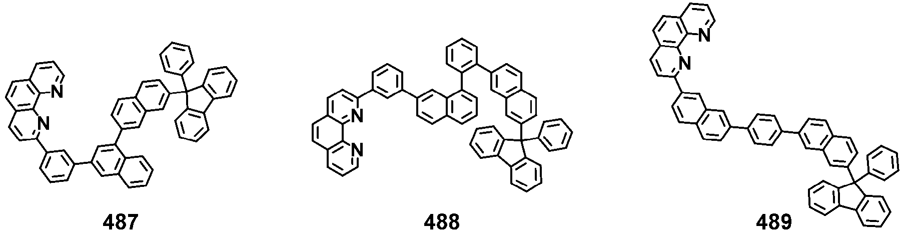

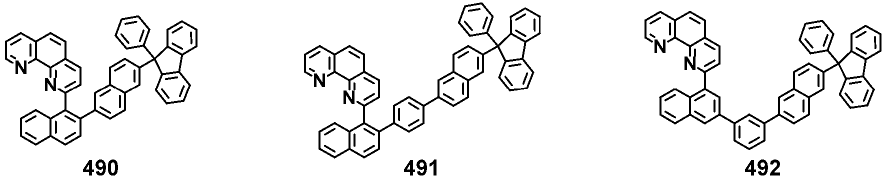

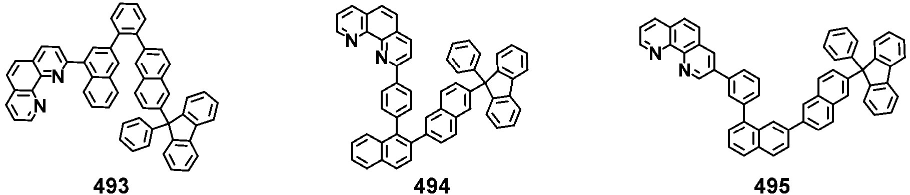

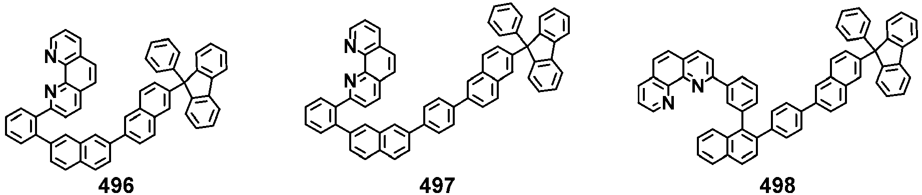

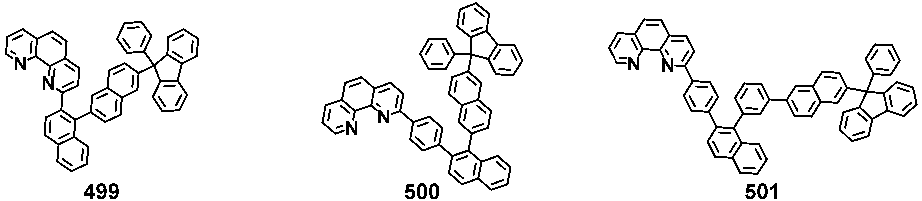

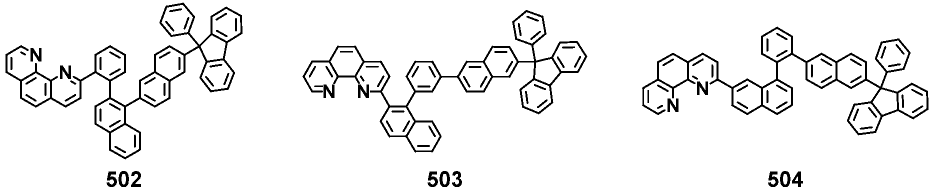

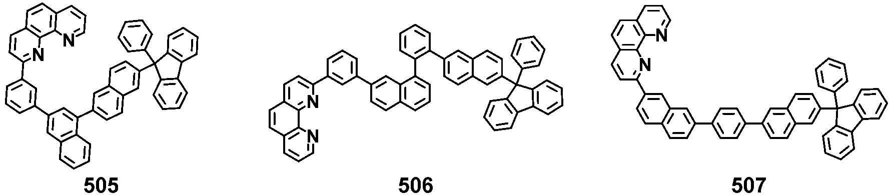

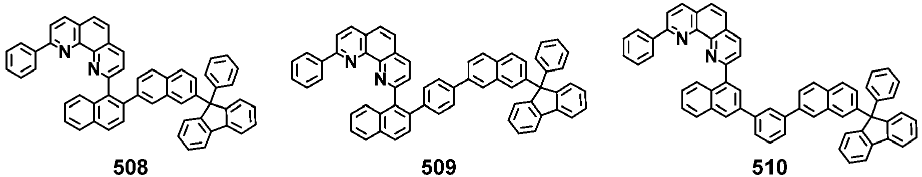

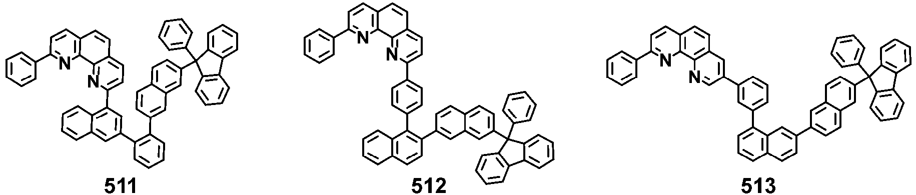

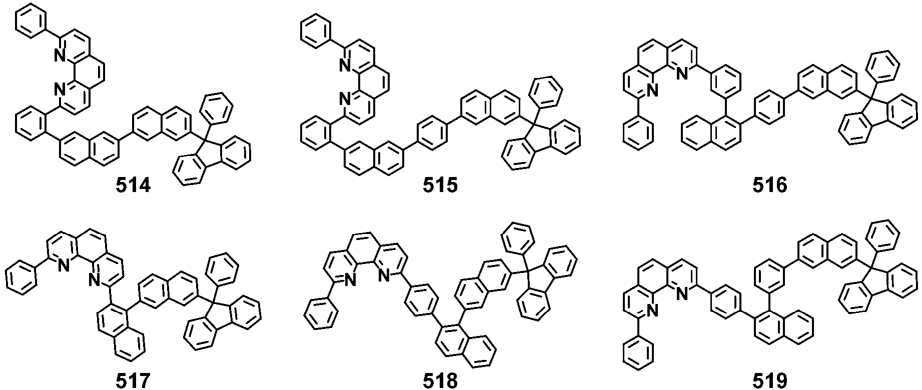

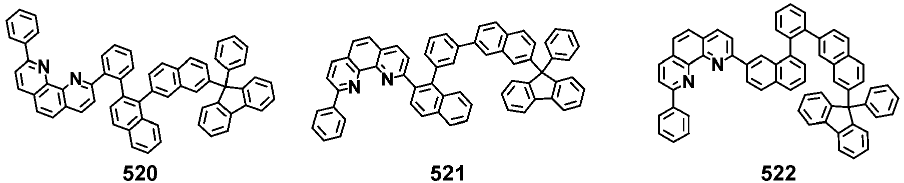

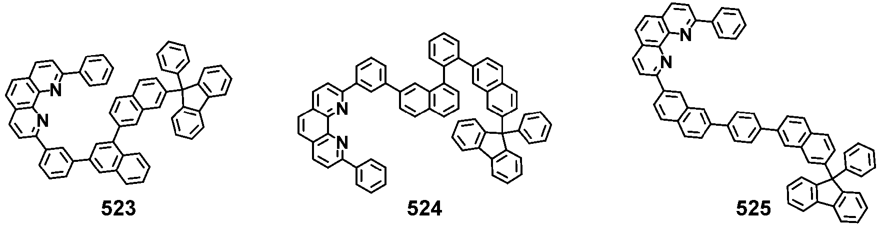

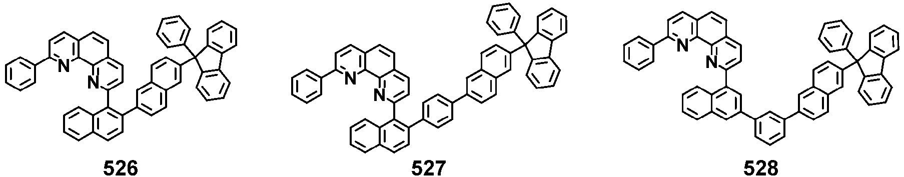

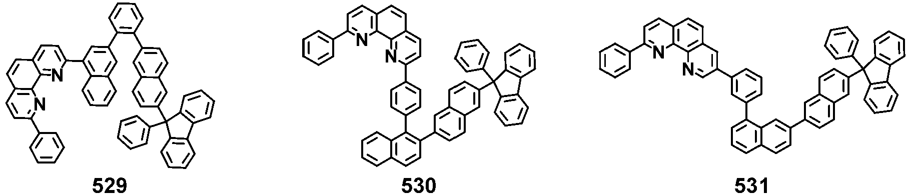

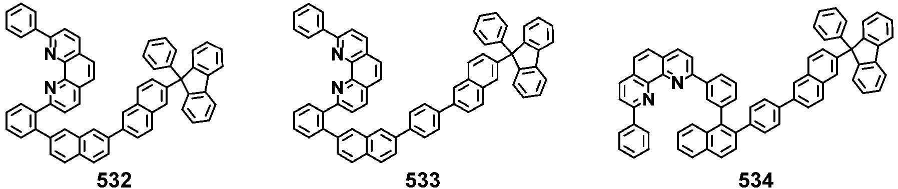

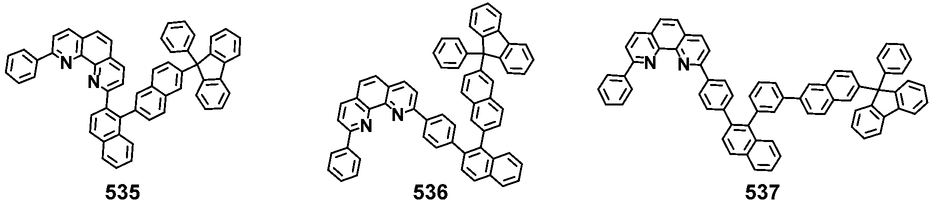

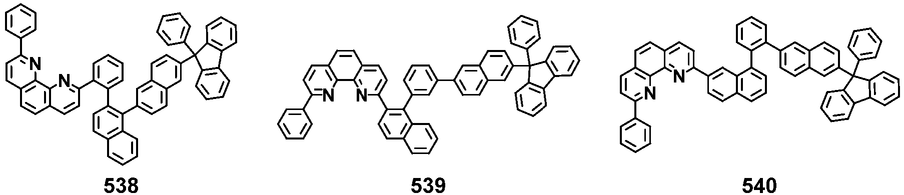

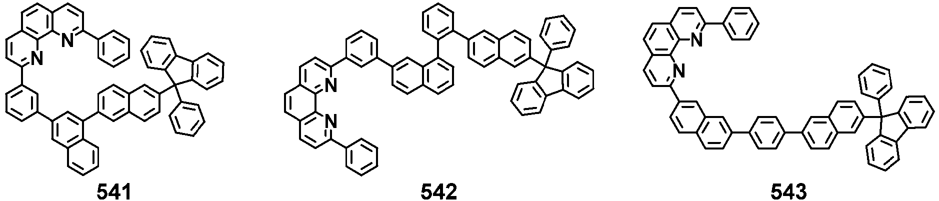

9. An organic light emitting device comprising:
a first electrode and a second electrode; and
at least one organic layer disposed between the first electrode and the second electrode,
wherein at least one of the organic layers comprises the compound according to any one of claims 1 to 8.

10. The organic light emitting device of claim 9, wherein the organic layer comprising the compound is at least one of an electron transport layer, a hole blocking layer, and a charge generation layer.

11. A tandem organic light emitting device comprising:
a first electrode and a second electrode;
a plurality of light-emitting members positioned between the first electrode and the second electrode; and
a charge generation layer respectively positioned at one or more locations between any two adjacent light-emitting members, wherein at least one of the charge generation layers comprises an N-type charge generation layer that includes a compound represented by the following Chemical Formula 1 and a dopant: 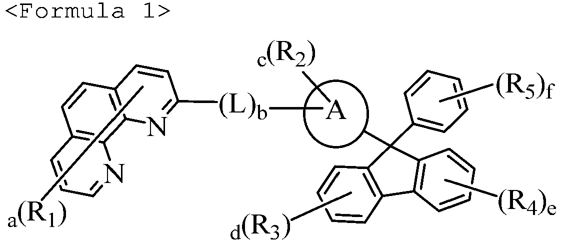
In the Chemical Formula 1,
ring A is phenyl or naphthyl,
R1 is each independently hydrogen, deuterium, a halogen group, a cyano group, a nitro group, a nitrile group, a hydroxyl group, a thiol group, a substituted or unsubstituted C0~C50 amine group, a substituted or unsubstituted C1~C50 alkyl group, a substituted or unsubstituted C1~C50 alkenyl group, a substituted or unsubstituted C1~C50 alkynyl group, a substituted or unsubstituted C1~C50 alkoxy group, a substituted or unsubstituted C1-C50 sulfide group, a substituted or unsubstituted C0~C50 silyl group, a substituted or unsubstituted C3~C50 cycloalkyl group, a substituted or unsubstituted C2~C50 heterocycloalkyl group, a substituted or unsubstituted C3~C50 cycloalkenyl group, a substituted or unsubstituted C1~C30 phosphine oxide group, a substituted or unsubstituted C2~C50 heterocycloalkenyl group, a substituted or unsubstituted C6~C50 aryl group, a substituted or unsubstituted C6~C50 aryloxy group, a substituted or unsubstituted C2~C50 heteroaryloxy group, or a substituted or unsubstituted C2~C50 heteroaryl group, wherein adjacent groups may or may not combine with each other to form a substituted or unsubstituted ring,
L is each independently a direct bond, a substituted or unsubstituted C6~C50 arylene group, or a substituted or unsubstituted C2~C50 heteroarylene group,
R2 to R5 are each independently hydrogen or deuterium, or may or may not be combined with each other to form a ring substituted or unsubstituted with one or more deuteriums,
a is an integer from 0 to 7,
b is an integer from 0 to 3,
c is an integer from 0 to 6,
d and e are each independently integers from 0 to 4, and
f is an integer from 0 to 5.

12. The tandem organic light emitting device of claim 11, wherein the dopant comprises at least one selected from lithium (Li), sodium (Na), potassium (K), rubidium (Rb), cesium (Cs), beryllium (Be), magnesium (Mg), calcium (Ca), strontium (Sr), barium (Ba), ytterbium (Yb), samarium (Sm), tin (Sn), copper (Cu), titanium (Ti), cadmium (Cd), mercury (Hg), lead (Pb), bismuth (Bi), zinc (Zn), iron (Fe), cobalt (Co), nickel (Ni), indium (In), gallium (Ga), thorium (Th), uranium (U), silver (Ag), aluminum (Al), gold (Au), molybdenum (Mo), niobium (Nb), palladium (Pd), platinum (Pt), and europium (Eu).
